(19) Europäisches Patentamt
European Patent Office
Office européen des brevets

(11) EP 4 026 907 A1

(12) **EUROPEAN PATENT APPLICATION**
published in accordance with Art. 153(4) EPC

(43) Date of publication:
**13.07.2022 Bulletin 2022/28**

(21) Application number: **21784935.5**

(22) Date of filing: **07.04.2021**

(51) International Patent Classification (IPC):
***C12N 15/12*** *(2006.01)* ***A61K 35/655*** *(2015.01)*
***A61K 38/17*** *(2006.01)* ***A61K 47/42*** *(2017.01)*
***A61K 47/50*** *(2017.01)* ***A61K 47/64*** *(2017.01)*
***C07K 14/435*** *(2006.01)* ***C07K 19/00*** *(2006.01)*
***C12N 1/15*** *(2006.01)* ***C12N 1/19*** *(2006.01)*
***C12N 1/21*** *(2006.01)* ***C12N 5/10*** *(2006.01)*
***C12N 15/63*** *(2006.01)* ***C12P 21/02*** *(2006.01)*

(52) Cooperative Patent Classification (CPC):
**A61K 35/655; A61K 38/17; A61K 47/42; A61K 47/50; A61K 47/64; A61P 5/00; A61P 31/00; A61P 35/00; A61P 37/02; C07K 14/435; C07K 19/00; C12N 5/10; C12N 15/63; C12N 15/74; C12N 15/80;** (Cont.)

(86) International application number:
**PCT/JP2021/015369**

(87) International publication number:
**WO 2021/206183 (14.10.2021 Gazette 2021/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.04.2020 JP 2020069328**

(71) Applicant: **National University Corporation Hokkaido University**
**Sapporo-shi, Hokkaido 060-0808 (JP)**

(72) Inventors:
- **SAKAI, Ryuichi**
  **Sapporo-shi, Hokkaido 060-0808 (JP)**
- **NAKANO, Koji**
  **Sapporo-shi, Hokkaido 060-0808 (JP)**

(74) Representative: **HGF**
**HGF Limited**
**1 City Walk**
**Leeds LS11 9DX (GB)**

# (54) FUNCTIONAL POLYPEPTIDES AND USE THEREOF

(57) A polypeptide that comprises the amino acid sequence of SEQ ID NO: 1 and has a function of delivering a substance into cells, a polypeptide that comprises the amino acid sequence of SEQ ID NO: 2 and has a function of degrading a nucleic acid molecule, a polypeptide that comprises the amino acid sequence of SEQ ID NO: 3 and has a function of degrading a nucleic acid molecule and a function of intranuclearly migrating the same, polynucleotides encoding the respective polypeptides, and transformed cells containing the respective polynucleotides.



Processed by Luminess, 75001 PARIS (FR)

(Cont. next page)

(52) Cooperative Patent Classification (CPC): (Cont.)
**C12N 15/81; C12P 21/02**

SEQUENCE LISTING

<110> National University Corporation Hokkaido University

<120> Functional polypeptide and use thereof

<130> G2477WO

<150> JP 2020-069328
<151> 2020-04-07

<160> 60

<170> PatentIn version 3.5

<210> 1
<211> 637
<212> PRT
<213> Spongosorites sp.

<400> 1

```
Asn Tyr Gly Ala Ile Glu Thr Thr Asp Asn Asn Gly Asn Thr Lys Ser
1               5                   10                  15

Asn Phe Val Glu Phe Thr Asn Pro Pro Gln Cys Thr Ala Pro Asp Thr
            20                  25                  30

Val Tyr Ala Lys Gly Ala Asp Phe Val Pro Asn Arg Thr Val Asp Ile
        35                  40                  45

Tyr Ile Thr Lys Gln Pro Asp Asn Gln Asn Phe Ile Pro Leu Pro Gln
    50                  55                  60

Pro Gln Asn Pro Thr Asn Pro Gly Gln Phe Lys Gln Val Gly Glu Ser
65                  70                  75                  80

Tyr Gln Leu Thr Asp Val Arg Thr Thr Gly Lys Ser Thr His Asn Ile
                85                  90                  95

Asp Ala Asn Gly Asn Ile Ala Asn Thr Ala Val Trp Val Gln Pro Thr
            100                 105                 110

Pro Asn Gln Gln Tyr Asn Leu Ile Val Asp Val Asp Arg Asp Gly Tyr
        115                 120                 125

Phe Thr Asn Ser Ile Asp Ile Val Asp Ala Phe Asn Thr Gln Gly Phe
    130                 135                 140

Asp Val Asp Pro Cys Leu Arg Lys Arg Leu Ala Ala Ser Leu Ser Asp
145                 150                 155                 160

Gly Ile Pro Ala Gly Asp Gly Thr Arg Asp Phe Val Lys Pro Pro Lys
```

```
                165                 170                 175

Ile Phe Ile Ser Gly Asp Trp Phe Pro Gln Gly Ser Ala Arg Ile Tyr
            180                 185                 190

Ile Ile Ile Tyr Asp Lys Asn Phe Asp Tyr Asn Gly Asn Asp Ser Lys
        195                 200                 205

Val Leu Thr Asp Val Arg Gly Thr Tyr Asp Thr Ile Thr Val Gly Glu
    210                 215                 220

Asn His Lys Phe Thr Gly Leu Glu Trp Ser Ser Pro His Val Gly Asn
225                 230                 235                 240

Tyr Asn Ile Ile Phe Asp Ala Asn Ser Asp Gly Val Tyr Lys Lys Ser
                245                 250                 255

Asp Gly Asp Ile Val Asp His Ile Asn Glu Ile Gly Phe Ala Met Leu
            260                 265                 270

Glu Ser Tyr Pro His His Ser His Ile Val Asp Leu Asp Asp Asn Ala
        275                 280                 285

Asn Gln Arg Glu Asp Phe Asn Ser Gly Ile Ala Lys Asn Val Tyr Val
    290                 295                 300

Leu Ala Thr Asn Leu Pro Asn Pro Ser Thr Ile Val Asp Val Tyr Val
305                 310                 315                 320

Ile Ser Glu Lys Leu Leu Lys Leu Asn Asn Pro Gly Trp Thr Thr Trp
                325                 330                 335

Gln Gln Ser Ser Asn Val Pro Leu Lys Glu Ser Ala Ala Pro Thr Asn
            340                 345                 350

Asn Gly Asp Arg Leu His Pro Thr Trp Val Thr Pro Gln Lys Thr Val
        355                 360                 365

Phe Gly Ser Val Trp His Met Pro Gly Asp Thr Phe Asp Pro Pro Tyr
    370                 375                 380

Ile Asn Tyr Tyr Gly Lys Lys Phe Thr Ile Val Ile Asp Val Asn Arg
385                 390                 395                 400

Asp Gly Lys Tyr Asp Pro Ser Ile Asp Met Val Asp Thr His Asp Ile
                405                 410                 415
```

```
Gly Asp Met Thr Asp Trp Phe Ile Thr Gln Gly His His Asp Leu Ser
            420                 425                 430

Pro Thr Ser Gly Asn Gly Gln Pro Ala Val Ser Glu Tyr Lys Glu Tyr
        435                 440                 445

Leu Asn Ser Lys Leu Asp Leu Glu Asn Pro Leu Asp Asn Thr Asn Asn
    450                 455                 460

Tyr Asp Ala Ala Thr Glu Ala Ala Ser Ala Gln Tyr Leu Cys Arg Thr
465                 470                 475                 480

Asn Leu Pro Ile Asn Leu Phe Glu Asn Val Ile Glu Leu Gly Ala Gln
                485                 490                 495

Thr Gly Phe Ile Met Phe Asn Pro Asp Glu Tyr Tyr Ala Gly Arg Asp
            500                 505                 510

Leu Asn Ser Gly Arg His Ile Tyr Asp Ala Val Asp Phe Asp Gly Leu
        515                 520                 525

Glu Ile Asn Asp Gly Tyr Thr Ser Val Ile Ser Ala Lys Glu Met Thr
    530                 535                 540

Leu Asn Arg Leu Gly Val Gly Gln Asn Ser Asn Leu Leu Thr Cys Gly
545                 550                 555                 560

Ala Asp Thr Val Thr Ile Asp Gly Met Ser Thr Gln Lys Asp Ser Thr
                565                 570                 575

Thr Asn Ile Val Ala Lys Thr Ile Ser His Lys Ser Asn Ile Ser Leu
            580                 585                 590

Glu Ala Asp Thr Cys Thr His Tyr Thr His Asn Leu Tyr Phe Glu Ser
        595                 600                 605

Asp Thr Asn Ile Leu Val Lys Ala Ile Glu Ser Pro Phe Ala Trp Ile
    610                 615                 620

Ile Asp Val Gly Asp Arg Ile Leu Pro Gly Thr Gly Cys
625                 630                 635
```

<210> 2
<211> 305
<212> PRT
<213> Spongosorites sp.

<400> 2

```
Arg Gln Ile Asp Ile Ala Ser Trp Asn Thr Phe Asp Phe Gly Gly Lys
1               5                   10                  15

Phe Gly Gly Val Cys Lys Ala Asn Asp Pro Thr Ile Leu Gln Ala Met
            20                  25                  30

Thr Asp Val Ile Ser Gln His Asp Val Ile Leu Val Gln Glu Ile Ala
        35                  40                  45

Asn Val Thr Asn Pro Cys Asn Pro Ala Asn Ile Phe Ser Gly Phe Asn
    50                  55                  60

Asn Leu Cys Ser Asn Asn Tyr Leu Gly Gly Leu Gly Tyr Leu Cys Gln
65                  70                  75                  80

Asp Thr Gly Ile Val Gly Ser Gly Glu Gly Tyr Gly Ile Ile Tyr Lys
                85                  90                  95

Asn Asn Thr Gly Ile Gly Asn Val Val Ile Glu Arg Thr Asp Asn Pro
            100                 105                 110

Asn Thr Val Pro Tyr Ile Pro Ala Gly Gly Gly Asn Asn Gln Met Lys
        115                 120                 125

Arg Pro Pro Met Lys Ala Thr Leu Asp Phe Asn Ala Gly Ala Phe Asn
    130                 135                 140

Val Val Val Tyr Asn Ala His Thr Ser Pro Ser Gly Gly Val Pro Lys
145                 150                 155                 160

Glu Ile Lys Leu Leu Ser Asp Arg Ile Ser Pro Ala His Asn Pro Leu
                165                 170                 175

Asp Lys Asn Ala Val Ile Thr Leu Gly Asp Leu Asn Ala Asp Gly Lys
            180                 185                 190

Arg Arg Leu Pro Asn Gly Gln Leu Asp His Gly Thr Asp Tyr Tyr Arg
        195                 200                 205

Gly Gly Phe Ala Ala His Pro Asn Asp Phe Pro Val Ser Gly Trp Gln
    210                 215                 220

Gln Thr Val Thr Asp Thr Asp Val Thr Asn Phe Ala Gln Thr Leu Arg
225                 230                 235                 240

Ala Tyr Asp Arg Met Ile Leu Ser Asn Ala Thr His Asn Ser Tyr Asp
                245                 250                 255
```

```
Glu Tyr Gly Ile Ile Gly Gly Ile Pro Asn Pro Leu Pro Tyr Pro Gly
            260                 265                 270

Gly Val Tyr Pro Ala Gly Thr Leu Phe Lys Asn Val His Thr Gly Gly
        275                 280                 285

Gly Asn Thr Gly Lys Ala Leu Ser Asp His Arg Leu Ile Trp Ser Lys
    290                 295                 300

Leu
305
```

<210> 3
<211> 947
<212> PRT
<213> Spongosorites sp.

<400> 3

```
Lys Leu Gly Asp Gln Arg Gln Ile Asp Ile Ala Ser Trp Asn Thr Phe
1               5                   10                  15

Asp Phe Gly Gly Lys Phe Gly Gly Val Cys Lys Ala Asn Asp Pro Thr
            20                  25                  30

Ile Leu Gln Ala Met Thr Asp Val Ile Ser Gln His Asp Val Ile Leu
        35                  40                  45

Val Gln Glu Ile Ala Asn Val Thr Asn Pro Cys Asn Pro Ala Asn Ile
    50                  55                  60

Phe Ser Gly Phe Asn Asn Leu Cys Ser Asn Asn Tyr Leu Gly Gly Leu
65                  70                  75                  80

Gly Tyr Leu Cys Gln Asp Thr Gly Ile Val Gly Ser Gly Glu Gly Tyr
                85                  90                  95

Gly Ile Ile Tyr Lys Asn Asn Thr Gly Ile Gly Asn Val Val Ile Glu
            100                 105                 110

Arg Thr Asp Asn Pro Asn Thr Val Pro Tyr Ile Pro Ala Gly Gly Gly
        115                 120                 125

Asn Asn Gln Met Lys Arg Pro Pro Met Lys Ala Thr Leu Asp Phe Asn
    130                 135                 140

Ala Gly Ala Phe Asn Val Val Val Tyr Asn Ala His Thr Ser Pro Ser
145                 150                 155                 160
```

```
Gly Gly Val Pro Lys Glu Ile Lys Leu Leu Ser Asp Arg Ile Ser Pro
                165                 170                 175

Ala His Asn Pro Leu Asp Lys Asn Ala Val Ile Thr Leu Gly Asp Leu
            180                 185                 190

Asn Ala Asp Gly Lys Arg Arg Leu Pro Asn Gly Gln Leu Asp His Gly
        195                 200                 205

Thr Asp Tyr Tyr Arg Gly Gly Phe Ala Ala His Pro Asn Asp Phe Pro
    210                 215                 220

Val Ser Gly Trp Gln Gln Thr Val Thr Asp Thr Asp Val Thr Asn Phe
225                 230                 235                 240

Ala Gln Thr Leu Arg Ala Tyr Asp Arg Met Ile Leu Ser Asn Ala Thr
                245                 250                 255

His Asn Ser Tyr Asp Glu Tyr Gly Ile Ile Gly Gly Ile Pro Asn Pro
            260                 265                 270

Leu Pro Tyr Pro Gly Gly Val Tyr Pro Ala Gly Thr Leu Phe Lys Asn
        275                 280                 285

Val His Thr Gly Gly Gly Asn Thr Gly Lys Ala Leu Ser Asp His Arg
    290                 295                 300

Leu Ile Trp Ser Lys Leu Asn Tyr Gly Ala Ile Glu Thr Thr Asp Asn
305                 310                 315                 320

Asn Gly Asn Thr Lys Ser Asn Phe Val Glu Phe Thr Asn Pro Pro Gln
                325                 330                 335

Cys Thr Ala Pro Asp Thr Val Tyr Ala Lys Gly Ala Asp Phe Val Pro
            340                 345                 350

Asn Arg Thr Val Asp Ile Tyr Ile Thr Lys Gln Pro Asp Asn Gln Asn
        355                 360                 365

Phe Ile Pro Leu Pro Gln Pro Gln Asn Pro Thr Asn Pro Gly Gln Phe
    370                 375                 380

Lys Gln Val Gly Glu Ser Tyr Gln Leu Thr Asp Val Arg Thr Thr Gly
385                 390                 395                 400

Lys Ser Thr His Asn Ile Asp Ala Asn Gly Asn Ile Ala Asn Thr Ala
                405                 410                 415
```

```
Val Trp Val Gln Pro Thr Pro Asn Gln Gln Tyr Asn Leu Ile Val Asp
            420                 425                 430

Val Asp Arg Asp Gly Tyr Phe Thr Asn Ser Ile Asp Ile Val Asp Ala
        435                 440                 445

Phe Asn Thr Gln Gly Phe Asp Val Asp Pro Cys Leu Arg Lys Arg Leu
    450                 455                 460

Ala Ala Ser Leu Ser Asp Gly Ile Pro Ala Gly Asp Gly Thr Arg Asp
465                 470                 475                 480

Phe Val Lys Pro Pro Lys Ile Phe Ile Ser Gly Asp Trp Phe Pro Gln
                485                 490                 495

Gly Ser Ala Arg Ile Tyr Ile Ile Ile Tyr Asp Lys Asn Phe Asp Tyr
            500                 505                 510

Asn Gly Asn Asp Ser Lys Val Leu Thr Asp Val Arg Gly Thr Tyr Asp
        515                 520                 525

Thr Ile Thr Val Gly Glu Asn His Lys Phe Thr Gly Leu Glu Trp Ser
    530                 535                 540

Ser Pro His Val Gly Asn Tyr Asn Ile Ile Phe Asp Ala Asn Ser Asp
545                 550                 555                 560

Gly Val Tyr Lys Lys Ser Asp Gly Asp Ile Val Asp His Ile Asn Glu
                565                 570                 575

Ile Gly Phe Ala Met Leu Glu Ser Tyr Pro His His Ser His Ile Val
            580                 585                 590

Asp Leu Asp Asp Asn Ala Asn Gln Arg Glu Asp Phe Asn Ser Gly Ile
        595                 600                 605

Ala Lys Asn Val Tyr Val Leu Ala Thr Asn Leu Pro Asn Pro Ser Thr
    610                 615                 620

Ile Val Asp Val Tyr Val Ile Ser Glu Lys Leu Leu Lys Leu Asn Asn
625                 630                 635                 640

Pro Gly Trp Thr Thr Trp Gln Gln Ser Ser Asn Val Pro Leu Lys Glu
                645                 650                 655

Ser Ala Ala Pro Thr Asn Asn Gly Asp Arg Leu His Pro Thr Trp Val
```

660 665 670

Thr Pro Gln Lys Thr Val Phe Gly Ser Val Trp His Met Pro Gly Asp
675 680 685

Thr Phe Asp Pro Pro Tyr Ile Asn Tyr Tyr Gly Lys Lys Phe Thr Ile
690 695 700

Val Ile Asp Val Asn Arg Asp Gly Lys Tyr Asp Pro Ser Ile Asp Met
705 710 715 720

Val Asp Thr His Asp Ile Gly Asp Met Thr Asp Trp Phe Ile Thr Gln
725 730 735

Gly His His Asp Leu Ser Pro Thr Ser Gly Asn Gly Gln Pro Ala Val
740 745 750

Ser Glu Tyr Lys Glu Tyr Leu Asn Ser Lys Leu Asp Leu Glu Asn Pro
755 760 765

Leu Asp Asn Thr Asn Asn Tyr Asp Ala Ala Thr Glu Ala Ala Ser Ala
770 775 780

Gln Tyr Leu Cys Arg Thr Asn Leu Pro Ile Asn Leu Phe Glu Asn Val
785 790 795 800

Ile Glu Leu Gly Ala Gln Thr Gly Phe Ile Met Phe Asn Pro Asp Glu
805 810 815

Tyr Tyr Ala Gly Arg Asp Leu Asn Ser Gly Arg His Ile Tyr Asp Ala
820 825 830

Val Asp Phe Asp Gly Leu Glu Ile Asn Asp Gly Tyr Thr Ser Val Ile
835 840 845

Ser Ala Lys Glu Met Thr Leu Asn Arg Leu Gly Val Gly Gln Asn Ser
850 855 860

Asn Leu Leu Thr Cys Gly Ala Asp Thr Val Thr Ile Asp Gly Met Ser
865 870 875 880

Thr Gln Lys Asp Ser Thr Thr Asn Ile Val Ala Lys Thr Ile Ser His
885 890 895

Lys Ser Asn Ile Ser Leu Glu Ala Asp Thr Cys Thr His Tyr Thr His
900 905 910

```
Asn Leu Tyr Phe Glu Ser Asp Thr Asn Ile Leu Val Lys Ala Ile Glu
        915                 920                 925

Ser Pro Phe Ala Trp Ile Ile Asp Val Gly Asp Arg Ile Leu Pro Gly
    930                 935                 940

Thr Gly Cys
945
```

<210> 4
<211> 1911
<212> DNA
<213> Spongosorites sp.

<400> 4

```
aactatggtg caatagagac taccgataat aacggcaata caaagtccaa ttttgttgaa      60
tttacaaacc caccacaatg tactgcacca gataccgtat atgcaaaggg tgcagacttt     120
gtaccaaata gaacagttga tatttacatc acaaagcagc cagacaatca aaactttatc     180
ccactgccac agccacaaaa cccaacaaac cctgggcagt tcaagcaagt tggcgaatca     240
taccagctaa cagatgtaag gactacaggc aaaagtacgc acaacattga tgcaaacggc     300
aacattgcaa atactgctgt atgggtacag ccaacaccaa accagcagta caacctcata     360
gttgatgtgg accgtgatgg atactttaca aacagcatag atatagttga tgcatttaac     420
acacaaggct ttgatgtaga cccatgcctt cgaaagagac tagcagcatc actttctgat     480
ggaataccag caggcgatgg cacacgtgac tttgtaaaac ctcccaaaat atttatttcc     540
ggtgactggt tcccacaggg ttctgcacgt atctacatta tcatatatga caagaacttt     600
gattataacg gcaatgactc caaagtatta actgacgtcc gtggaactta tgacacaatt     660
acagtgggtg agaaccacaa gtttacagga ctagaatggt cctcaccaca tgttggtaac     720
tataacataa tatttgatgc aaactctgat ggagtataca aaaaatccga tggtgacatt     780
gtagatcaca taaacgagat aggctttgca atgctcgaat cgtatccaca tcacagccat     840
attgtagatt tagatgacaa tgcaaaccag cgagaagact ttaacagtgg aattgcaaaa     900
aacgtctacg ttcttgcaac taatcttcca aacccaagca caatagttga cgtttatgta     960
attagtgaga aactcttaaa gctaaacaat cctggatgga ccacatggca gcaatcctct    1020
aatgtgccac taaaggagag tgcagcacca actaacaatg gagacaggct acaccctact    1080
tgggtaactc cacaaaagac agtatttggt tcagtatggc atatgcccgg tgatacattt    1140
gatccaccat acattaatta ttacggcaaa aaattcacta tagtaattga tgtgaatcgt    1200
gatggtaagt atgatcctag tatagatatg gtagatactc acgatatagg agacatgaca    1260
gactggttta tcacacaggg tcaccatgac ctaagcccaa ctagtggaaa cggacagcct    1320
gccgtatctg aatacaaaga gtatctcaac tcaaagctag acttggagaa cccgcttgat    1380
```

```
aacaccaaca actatgatgc tgcaactgag gcagcttctg cacaatatct atgccgtacc     1440

aatcttccaa tcaatctatt tgagaatgtc atagagctgg gagctcagac aggctttatc     1500

atgtttaacc ctgatgagta ttatgcaggc cgtgatctaa attctggacg ccacatttat     1560

gatgctgtag atttgatgg cttggagata aatgacggat acacatcagt aatctctgca      1620

aaagagatga ccctaaacag attaggtgtt ggccaaaact ccaatctgct tacctgcgga     1680

gctgacacag taacgataga tggcatgtca acacaaaaag attcaactac aaatattgtt     1740

gcaaagacca tatctcacaa aagtaacatc tcactagagg cagacacttg tacacattat     1800

actcacaatc tctactttga gtcagacacc aacatcttag ttaaggccat tgagagtcca     1860

tttgcatgga taattgacgt aggtgacaga atacttccag gtactggctg t              1911


<210>  5
<211>  1911
<212>  PRT
<213>  Artificial

<220>
<223>  Synthetic

<400>  5

Ala Ala Thr Thr Ala Cys Gly Gly Gly Gly Cys Cys Ala Thr Thr Gly
1               5                   10                  15

Ala Ala Ala Cys Thr Ala Cys Gly Gly Ala Cys Ala Ala Cys Ala Ala
            20                  25                  30

Cys Gly Gly Cys Ala Ala Cys Ala Cys Thr Ala Ala Ala Ala Gly Cys
        35                  40                  45

Ala Ala Thr Thr Thr Thr Gly Thr Cys Gly Ala Gly Thr Thr Cys Ala
    50                  55                  60

Cys Cys Ala Ala Cys Cys Cys Ala Cys Cys Thr Cys Ala Gly Thr Gly
65                  70                  75                  80

Cys Ala Cys Thr Gly Cys Ala Cys Cys Ala Gly Ala Cys Ala Cys Gly
                85                  90                  95

Gly Thr Thr Thr Ala Thr Gly Cys Thr Ala Ala Ala Gly Gly Cys Gly
            100                 105                 110

Cys Cys Gly Ala Cys Thr Thr Thr Gly Thr Gly Cys Cys Cys Ala Ala
        115                 120                 125

Thr Cys Gly Thr Ala Cys Ala Gly Thr Gly Gly Ala Thr Ala Thr Cys
```

```
    130                 135                 140

Thr Ala Thr Ala Thr Thr Ala Cys Thr Ala Ala Gly Cys Ala Gly Cys
145                 150                 155                 160

Cys Cys Gly Ala Cys Ala Ala Cys Cys Ala Ala Ala Ala Thr Thr Thr
                165                 170                 175

Cys Ala Thr Thr Cys Cys Cys Cys Thr Gly Cys Cys Gly Cys Ala Ala
            180                 185                 190

Cys Cys Cys Cys Ala Ala Ala Ala Thr Cys Cys Thr Ala Cys Gly Ala
        195                 200                 205

Ala Thr Cys Cys Thr Gly Gly Gly Cys Ala Ala Thr Thr Cys Ala Ala
    210                 215                 220

Ala Cys Ala Ala Gly Thr Thr Gly Gly Gly Gly Ala Ala Thr Cys Ala
225                 230                 235                 240

Thr Ala Cys Cys Ala Ala Thr Thr Gly Ala Cys Gly Gly Ala Cys Gly
                245                 250                 255

Thr Cys Cys Gly Thr Ala Cys Cys Ala Cys Cys Gly Gly Cys Ala Ala
            260                 265                 270

Gly Thr Cys Cys Ala Cys Cys Cys Ala Cys Ala Ala Thr Ala Thr Cys
        275                 280                 285

Gly Ala Cys Gly Cys Thr Ala Ala Cys Gly Gly Cys Ala Ala Cys Ala
    290                 295                 300

Thr Thr Gly Cys Thr Ala Ala Cys Ala Cys Gly Gly Cys Thr Gly Thr
305                 310                 315                 320

Ala Thr Gly Gly Gly Thr Gly Cys Ala Gly Cys Cys Ala Ala Cys Ala
                325                 330                 335

Cys Cys Ala Ala Ala Cys Cys Ala Ala Cys Ala Gly Thr Ala Cys Ala
            340                 345                 350

Ala Thr Cys Thr Gly Ala Thr Thr Gly Thr Gly Gly Ala Thr Gly Thr
        355                 360                 365

Gly Gly Ala Thr Cys Gly Cys Gly Ala Cys Gly Gly Cys Thr Ala Cys
    370                 375                 380
```

Thr Thr Cys Ala Cys Gly Ala Ala Cys Thr Cys Gly Ala Thr Cys Gly
385 390 395 400

Ala Cys Ala Thr Cys Gly Thr Ala Gly Ala Thr Gly Cys Ala Thr Thr
405 410 415

Cys Ala Ala Thr Ala Cys Gly Cys Ala Gly Gly Gly Ala Thr Thr Thr
420 425 430

Gly Ala Thr Gly Thr Cys Gly Ala Thr Cys Cys Ala Thr Gly Thr Thr
435 440 445

Thr Gly Cys Gly Cys Ala Ala Ala Cys Gly Thr Cys Thr Gly Gly Cys
450 455 460

Ala Gly Cys Gly Thr Cys Ala Cys Thr Gly Thr Cys Cys Gly Ala Cys
465 470 475 480

Gly Gly Cys Ala Thr Cys Cys Cys Cys Gly Cys Ala Gly Gly Cys Gly
485 490 495

Ala Thr Gly Gly Cys Ala Cys Gly Cys Gly Thr Gly Ala Cys Thr Thr
500 505 510

Thr Gly Thr Cys Ala Ala Ala Cys Cys Thr Cys Cys Ala Ala Ala Gly
515 520 525

Ala Thr Thr Thr Thr Cys Ala Thr Cys Thr Cys Ala Gly Gly Cys Gly
530 535 540

Ala Thr Thr Gly Gly Thr Thr Thr Cys Cys Ala Cys Ala Gly Gly Gly
545 550 555 560

Cys Ala Gly Thr Gly Cys Cys Cys Gly Cys Ala Thr Cys Thr Ala Cys
565 570 575

Ala Thr Cys Ala Thr Thr Ala Thr Cys Thr Ala Thr Gly Ala Cys Ala
580 585 590

Ala Gly Ala Ala Thr Thr Thr Cys Gly Ala Thr Thr Ala Thr Ala Ala
595 600 605

Cys Gly Gly Ala Ala Ala Cys Gly Ala Cys Ala Gly Thr Ala Ala Gly
610 615 620

Gly Thr Thr Cys Thr Gly Ala Cys Thr Gly Ala Thr Gly Thr Thr Cys
625 630 635 640

Gly Thr Gly Gly Cys Ala Cys Thr Thr Ala Thr Gly Ala Cys Ala Cys
645 650 655

Ala Ala Thr Cys Ala Cys Gly Gly Thr Ala Gly Gly Ala Gly Ala Ala
660 665 670

Ala Ala Cys Cys Ala Thr Ala Ala Gly Thr Thr Thr Ala Cys Cys Gly
675 680 685

Gly Cys Thr Thr Ala Gly Ala Ala Thr Gly Gly Ala Gly Thr Thr Cys
690 695 700

Thr Cys Cys Cys Cys Ala Cys Gly Thr Thr Gly Gly Cys Ala Ala Cys
705 710 715 720

Thr Ala Thr Ala Ala Thr Ala Thr Thr Ala Thr Cys Thr Thr Thr Gly
725 730 735

Ala Thr Gly Cys Thr Ala Ala Thr Ala Gly Cys Gly Ala Thr Gly Gly
740 745 750

Cys Gly Thr Thr Thr Ala Cys Ala Ala Gly Ala Ala Ala Thr Cys Ala
755 760 765

Gly Ala Thr Gly Gly Thr Gly Ala Thr Ala Thr Thr Gly Thr Ala Gly
770 775 780

Ala Cys Cys Ala Cys Ala Thr Thr Ala Ala Cys Gly Ala Gly Ala Thr
785 790 795 800

Cys Gly Gly Gly Thr Thr Cys Gly Cys Cys Ala Thr Gly Thr Thr Gly
805 810 815

Gly Ala Gly Thr Cys Thr Thr Ala Thr Cys Cys Ala Cys Ala Cys Cys
820 825 830

Ala Cys Thr Cys Gly Cys Ala Cys Ala Thr Thr Gly Thr Thr Gly Ala
835 840 845

Cys Thr Thr Gly Gly Ala Thr Gly Ala Cys Ala Ala Thr Gly Cys Gly
850 855 860

Ala Ala Thr Cys Ala Ala Cys Gly Thr Gly Ala Ala Gly Ala Thr Thr
865 870 875 880

Thr Cys Ala Ala Cys Ala Gly Cys Gly Gly Cys Ala Thr Thr Gly Cys
885 890 895

```
Thr Ala Ala Gly Ala Ala Cys Gly Thr Ala Thr Ala Cys Gly Thr Cys
            900                 905                 910

Thr Thr Ala Gly Cys Cys Ala Cys Gly Ala Ala Cys Thr Thr Ala Cys
        915                 920                 925

Cys Gly Ala Ala Cys Cys Cys Cys Thr Cys Cys Ala Cys Ala Ala Thr
    930                 935                 940

Cys Gly Thr Ala Gly Ala Cys Gly Thr Thr Thr Ala Cys Gly Thr Gly
945                 950                 955                 960

Ala Thr Thr Thr Cys Ala Gly Ala Ala Ala Ala Gly Thr Thr Gly Thr
                965                 970                 975

Thr Gly Ala Ala Ala Cys Thr Cys Ala Ala Thr Ala Ala Thr Cys Cys
            980                 985                 990

Thr Gly Gly Thr Thr Gly Gly Ala  Cys Cys Ala Cys Cys  Thr Gly Gly
        995                 1000                 1005

Cys Ala  Ala Cys Ala Ala Thr  Cys Cys Thr Cys Ala  Ala Ala Cys
    1010                 1015                 1020

Gly Thr  Gly Cys Cys Ala Cys  Thr Thr Ala Ala Gly  Gly Ala Gly
    1025                 1030                 1035

Ala Gly  Thr Gly Cys Gly Gly  Cys Ala Cys Cys Gly  Ala Cys Ala
    1040                 1045                 1050

Ala Ala  Cys Ala Ala Thr Gly  Gly Thr Gly Ala Cys  Cys Gly Thr
    1055                 1060                 1065

Cys Thr  Gly Cys Ala Thr Cys  Cys Ala Ala Cys Gly  Thr Gly Gly
    1070                 1075                 1080

Gly Thr  Ala Ala Cys Thr Cys  Cys Gly Cys Ala Gly  Ala Ala Gly
    1085                 1090                 1095

Ala Cys  Gly Gly Thr Ala Thr  Thr Thr Gly Gly Thr  Ala Gly Cys
    1100                 1105                 1110

Gly Thr  Cys Thr Gly Gly Cys  Ala Cys Ala Thr Gly  Cys Cys Gly
    1115                 1120                 1125

Gly Gly  Cys Gly Ala Thr Ala  Cys Ala Thr Thr Cys  Gly Ala Cys
```

1130 1135 1140

Cys Cys Gly Cys Cys Thr Thr Ala Cys Ala Thr Cys Ala Ala Thr
1145 1150 1155

Thr Ala Thr Thr Ala Thr Gly Gly Ala Ala Ala Gly Ala Ala Gly
1160 1165 1170

Thr Thr Thr Ala Cys Ala Ala Thr Cys Gly Thr Cys Ala Thr Thr
1175 1180 1185

Gly Ala Cys Gly Thr Thr Ala Ala Cys Cys Gly Cys Gly Ala Cys
1190 1195 1200

Gly Gly Cys Ala Ala Gly Thr Ala Cys Gly Ala Cys Cys Cys Ala
1205 1210 1215

Thr Cys Ala Ala Thr Thr Gly Ala Thr Ala Thr Gly Gly Thr Ala
1220 1225 1230

Gly Ala Thr Ala Cys Thr Cys Ala Thr Gly Ala Cys Ala Thr Thr
1235 1240 1245

Gly Gly Thr Gly Ala Cys Ala Thr Gly Ala Cys Thr Gly Ala Cys
1250 1255 1260

Thr Gly Gly Thr Thr Cys Ala Thr Thr Ala Cys Gly Cys Ala Ala
1265 1270 1275

Gly Gly Gly Cys Ala Cys Cys Ala Cys Gly Ala Thr Cys Thr Gly
1280 1285 1290

Thr Cys Ala Cys Cys Gly Ala Cys Thr Thr Cys Gly Gly Gly Cys
1295 1300 1305

Ala Ala Cys Gly Gly Thr Cys Ala Ala Cys Cys Gly Gly Cys Cys
1310 1315 1320

Gly Thr Gly Thr Cys Thr Gly Ala Ala Thr Ala Cys Ala Ala Ala
1325 1330 1335

Gly Ala Gly Thr Ala Cys Cys Thr Gly Ala Ala Thr Ala Gly Thr
1340 1345 1350

Ala Ala Ala Cys Thr Cys Gly Ala Cys Cys Thr Gly Gly Ala Ala
1355 1360 1365

```
Ala Ala  Thr Cys Cys Ala Thr  Thr Ala Gly Ala Thr  Ala Ala Cys
    1370                1375                1380

Ala Cys  Cys Ala Ala Thr Ala  Ala Thr Thr Ala Thr  Gly Ala Thr
    1385                1390                1395

Gly Cys  Ala Gly Cys Cys Ala  Cys Cys Gly Ala Ala  Gly Cys Ala
    1400                1405                1410

Gly Cys  Thr Thr Cys Thr Gly  Cys Cys Cys Ala Ala  Thr Ala Thr
    1415                1420                1425

Thr Thr  Ala Thr Gly Thr Cys  Gly Thr Ala Cys Cys  Ala Ala Cys
    1430                1435                1440

Cys Thr  Thr Cys Cys Gly Ala  Thr Cys Ala Ala Thr  Cys Thr Gly
    1445                1450                1455

Thr Thr  Cys Gly Ala Gly Ala  Ala Thr Gly Thr Ala  Ala Thr Thr
    1460                1465                1470

Gly Ala  Gly Cys Thr Thr Gly  Gly Cys Gly Cys Gly  Cys Ala Gly
    1475                1480                1485

Ala Cys  Cys Gly Gly Cys Thr  Thr Cys Ala Thr Thr  Ala Thr Gly
    1490                1495                1500

Thr Thr  Cys Ala Ala Cys Cys  Cys Cys Gly Ala Thr  Gly Ala Gly
    1505                1510                1515

Thr Ala  Thr Thr Ala Thr Gly  Cys Cys Gly Gly Gly  Cys Gly Thr
    1520                1525                1530

Gly Ala  Thr Cys Thr Thr Ala  Ala Thr Ala Gly Thr  Gly Gly Cys
    1535                1540                1545

Cys Gly  Cys Cys Ala Thr Ala  Thr Cys Thr Ala Thr  Gly Ala Thr
    1550                1555                1560

Gly Cys  Ala Gly Thr Gly Gly  Ala Cys Thr Thr Cys  Gly Ala Thr
    1565                1570                1575

Gly Gly  Ala Thr Thr Gly Gly  Ala Gly Ala Thr Thr  Ala Ala Thr
    1580                1585                1590

Gly Ala  Cys Gly Gly Ala Thr  Ala Thr Ala Cys Gly  Thr Cys Thr
    1595                1600                1605
```

Gly Thr Gly Ala Thr Cys Ala Gly Thr Gly Cys Ala Ala Ala Ala
1610 1615 1620

Gly Ala Ala Ala Thr Gly Ala Cys Gly Thr Thr Ala Ala Ala Cys
1625 1630 1635

Cys Gly Cys Cys Thr Cys Gly Gly Thr Gly Thr Thr Gly Gly Cys
1640 1645 1650

Cys Ala Ala Ala Ala Thr Ala Gly Thr Ala Ala Cys Cys Thr Thr
1655 1660 1665

Thr Thr Gly Ala Cys Thr Thr Gly Cys Gly Gly Cys Gly Cys Ala
1670 1675 1680

Gly Ala Cys Ala Cys Cys Gly Thr Gly Ala Cys Ala Ala Thr Cys
1685 1690 1695

Gly Ala Thr Gly Gly Ala Ala Thr Gly Thr Cys Thr Ala Cys Thr
1700 1705 1710

Cys Ala Ala Ala Ala Gly Gly Ala Thr Thr Cys Thr Ala Cys Gly
1715 1720 1725

Ala Cys Ala Ala Ala Cys Ala Thr Thr Gly Thr Ala Gly Cys Ala
1730 1735 1740

Ala Ala Gly Ala Cys Ala Ala Thr Cys Thr Cys Thr Cys Ala Cys
1745 1750 1755

Ala Ala Ala Ala Gly Thr Ala Ala Cys Ala Thr Thr Thr Cys Gly
1760 1765 1770

Cys Thr Gly Gly Ala Gly Gly Cys Gly Gly Ala Thr Ala Cys Gly
1775 1780 1785

Thr Gly Cys Ala Cys Cys Cys Ala Cys Thr Ala Thr Ala Cys Gly
1790 1795 1800

Cys Ala Thr Ala Ala Cys Thr Thr Ala Thr Ala Thr Thr Thr Thr
1805 1810 1815

Gly Ala Ala Thr Cys Cys Gly Ala Thr Ala Cys Gly Ala Ala Thr
1820 1825 1830

Ala Thr Cys Thr Thr Gly Gly Thr Thr Ala Ala Gly Gly Cys Ala
1835 1840 1845

```
Ala Thr Thr Gly Ala Gly Thr Cys Cys Cys Cys Ala Thr Thr Cys
    1850                1855                1860

Gly Cys Thr Thr Gly Gly Ala Thr Thr Ala Thr Thr Gly Ala Cys
    1865                1870                1875

Gly Thr Gly Gly Gly Thr Gly Ala Thr Cys Gly Thr Ala Thr Thr
    1880                1885                1890

Cys Thr Gly Cys Cys Cys Gly Gly Cys Ala Cys Thr Gly Gly Cys
    1895                1900                1905

Thr Gly Thr
    1910

<210>  6
<211>  930
<212>  DNA
<213>  Spongosorites sp.

<400>  6
aagcttggag atcagcgaca gatagatatt gcaagctgga acacatttga ttttggtggc       60

aagtttggcg gtgtatgtaa ggcaaatgat cctaccatac tgcaagcaat gactgatgta      120

atatctcagc atgatgtcat tttggttcaa gagattgcaa atgtcacaaa tccatgtaat      180

cctgcaaaca tcttttcagg ctttaataat ttatgttcaa ataactatct tggcggtctg      240

ggatatctct gccaagatac tggcattgta ggctctggtg agggctatgg tattatctac      300

aaaaacaaca ccggtattgg taatgtagta attgagcgta cagataaccc aaatacagtt      360

ccatacattc ctgcaggtgg tggcaacaac caaatgaaga ggccaccaat gaaggcaacc      420

cttgacttta atgcaggtgc attcaacgta gtagtatata atgcacacac aagcccaagt      480

ggcggtgttc ccaaagagat aaagctactc agtgatagaa tatcgcctgc tcacaatccg      540

ctagataaaa acgcagtaat tacactaggt gatcttaatg ccgacggcaa aagaagactg      600

cctaatggac agctagacca tggaacagac tattacagag gtggctttgc agctcaccca      660

aatgactttc ctgttagtgg atggcagcag acagttactg atactgatgt tacaaacttt      720

gcacagacac ttcgtgcata tgatagaatg atacttagca atgcaacaca taactcttat      780

gatgagtatg gaataatagg tggcattcca aacccacttc catatccggg aggagtttat      840

cctgctggaa ctttgtttaa aaatgtacat actggcggtg gaaatacagg aaaggcactc      900

tctgatcata gactgatatg gtcaaagctt                                       930

<210>  7
<211>  930
```

<212> DNA
<213> Artificial

<220>
<223> Synthetic

<400> 7
aagctcggcg atcaacgtca aatcgatatc gcgagctgga atacgtttga cttcggtggg 60

aaattcggcg gcgtgtgcaa agccaatgac ccgacgattc tccaagcgat gactgatgtg 120

atctcccagc acgatgtaat tctggtccag gagatcgcca acgtgacgaa cccgtgtaac 180

cccgctaata ttttcagtgg cttcaataac ttgtgtagta acaactactt aggtggttta 240

ggttatcttt gccaagacac tgggatcgtt ggtagcggtg agggctatgg gatcatctac 300

aagaacaata ccggtatcgg taacgtcgtc atcgagcgca ctgataaccc gaatactgtg 360

ccatatattc ccgccggagg cggcaacaac cagatgaagc gcccgccgat gaaggcaacg 420

cttgatttca atgcgggcgc cttcaatgta gtcgtgtata atgcccacac gagcccatcg 480

ggcggcgtac caaaggaaat caaattgctc tccgaccgta tcagcccagc acataatcct 540

cttgacaaga acgcagtaat tactttggga gacttgaatg cggatggaaa gcgtcgcctg 600

ccaaatggac agttagatca cggtacagat tattaccgcg gcggatttgc cgcacatccg 660

aacgactttc cggtaagtgg gtggcagcaa actgtgacgg atactgatgt aaccaacttc 720

gcacaaactc tccgtgcgta cgaccgcatg atcttatcaa acgccacgca caatagttac 780

gacgagtatg ggatcatcgg cggcatccct aaccctctcc cctacccggg tggcgtttac 840

cccgccggaa cgttgttcaa gaacgtgcat acaggcggtg gcaacacagg aaaggctctc 900

agtgatcacc gtctgatctg gtctaagtta 930

<210> 8
<211> 2844
<212> DNA
<213> Spongosorites sp.

<400> 8
aagcttggag atcagcgaca gatagatatt gcaagctgga acacatttga ttttggtggc 60

aagtttggcg gtgtatgtaa ggcaaatgat cctaccatac tgcaagcaat gactgatgta 120

atatctcagc atgatgtcat tttggttcaa gagattgcaa atgtcacaaa tccatgtaat 180

cctgcaaaca tcttttcagg ctttaataat ttatgttcaa ataactatct tggcggtctg 240

ggatatctct gccaagatac tggcattgta ggctctggtg agggctatgg tattatctac 300

aaaaacaaca ccggtattgg taatgtagta attgagcgta cagataaccc aaatacagtt 360

ccatacattc ctgcaggtgg tggcaacaac caaatgaaga ggccaccaat gaaggcaacc 420

cttgacttta atgcaggtgc attcaacgta gtagtatata atgcacacac aagcccaagt 480

ggcggtgttc ccaaagagat aaagctactc agtgatagaa tatcgcctgc tcacaatccg 540

ctagataaaa acgcagtaat tacactaggt gatcttaatg ccgacggcaa aagaagactg 600
cctaatggac agctagacca tggaacagac tattacagag gtggctttgc agctcaccca 660
aatgactttc ctgttagtgg atggcagcag acagttactg atactgatgt tacaaacttt 720
gcacagacac ttcgtgcata tgatagaatg atacttagca atgcaacaca taactcttat 780
gatgagtatg gaataatagg tggcattcca aacccacttc catatccggg aggagtttat 840
cctgctggaa ctttgtttaa aaatgtacat actggcggtg gaaatacagg aaaggcactc 900
tctgatcata gactgatatg gtcaaagctt aactatggtg caatagagac taccgataat 960
aacggcaata caaagtccaa ttttgttgaa tttacaaacc caccacaatg tactgcacca 1020
gataccgtat atgcaaaggg tgcagacttt gtaccaaata gaacagttga tatttacatc 1080
acaaagcagc cagacaatca aaactttatc ccactgccac agccacaaaa cccaacaaac 1140
cctgggcagt tcaagcaagt tggcgaatca taccagctaa cagatgtaag gactacaggc 1200
aaaagtacgc acaacattga tgcaaacggc aacattgcaa atactgctgt atgggtacag 1260
ccaacaccaa accagcagta caacctcata gttgatgtgg accgtgatgg atactttaca 1320
aacagcatag atatagttga tgcatttaac acacaaggct ttgatgtaga cccatgcctt 1380
cgaaagagac tagcagcatc actttctgat ggaataccag caggcgatgg cacacgtgac 1440
tttgtaaaac ctcccaaaat atttatttcc ggtgactggt tcccacaggg ttctgcacgt 1500
atctacatta tcatatatga caagaacttt gattataacg gcaatgactc caaagtatta 1560
actgacgtcc gtggaactta tgacacaatt acagtgggtg agaaccacaa gtttacagga 1620
ctagaatggt cctcaccaca tgttggtaac tataacataa tatttgatgc aaactctgat 1680
ggagtataca aaaaatccga tggtgacatt gtagatcaca taaacgagat aggctttgca 1740
atgctcgaat cgtatccaca tcacagccat attgtagatt tagatgacaa tgcaaaccag 1800
cgagaagact ttaacagtgg aattgcaaaa aacgtctacg ttcttgcaac taatcttcca 1860
aacccaagca caatagttga cgtttatgta attagtgaga aactcttaaa gctaaacaat 1920
cctggatgga ccacatggca gcaatcctct aatgtgccac taaaggagag tgcagcacca 1980
actaacaatg gagacaggct acaccctact tgggtaactc cacaaaagac agtatttggt 2040
tcagtatggc atatgcccgg tgatacattt gatccaccat acattaatta ttacggcaaa 2100
aaattcacta tagtaattga tgtgaatcgt gatggtaagt atgatcctag tatagatatg 2160
gtagatactc acgatatagg agacatgaca gactggttta tcacacaggg tcaccatgac 2220
ctaagcccaa ctagtggaaa cggacagcct gccgtatctg aatacaaaga gtatctcaac 2280
tcaaagctag acttggagaa cccgcttgat aacaccaaca actatgatgc tgcaactgag 2340
gcagcttctg cacaatatct atgccgtacc aatcttccaa tcaatctatt tgagaatgtc 2400

atagagctgg gagctcagac aggctttatc atgtttaacc ctgatgagta ttatgcaggc 2460

cgtgatctaa attctggacg ccacatttat gatgctgtag attttgatgg cttggagata 2520

aatgacggat acacatcagt aatctctgca aaagagatga ccctaaacag attaggtgtt 2580

ggccaaaact ccaatctgct tacctgcgga gctgacacag taacgataga tggcatgtca 2640

acacaaaaag attcaactac aaatattgtt gcaaagacca tatctcacaa aagtaacatc 2700

tcactagagg cagacacttg tacacattat actcacaatc tctactttga gtcagacacc 2760

aacatcttag ttaaggccat tgagagtcca tttgcatgga taattgacgt aggtgacaga 2820

atacttccag gtactggctg ttag 2844

<210> 9
<211> 2848
<212> DNA
<213> Artificial

<220>
<223> Synthetic

<400> 9
aagctcggcg atcaacgtca aatcgatatc gcgagctgga atacgtttga cttcggtggg 60

aaattcggcg gcgtgtgcaa agccaatgac ccgacgattc tccaagcgat gactgatgtg 120

atctcccagc acgatgtaat tctggtccag gagatcgcca acgtgacgaa cccgtgtaac 180

cccgctaata ttttcagtgg cttcaataac ttgtgtagta acaactactt aggtggttta 240

ggttatcttt gccaagacac tgggatcgtt ggtagcggtg agggctatgg gatcatctac 300

aagaacaata ccggtatcgg taacgtcgtc atcgagcgca ctgataaccc gaatactgtg 360

ccatatattc ccgccggagg cggcaacaac cagatgaagc gcccgccgat gaaggcaacg 420

cttgatttca atgcgggcgc cttcaatgta gtcgtgtata atgcccacac gagcccatcg 480

ggcggcgtac caaaggaaat caaattgctc tccgaccgta tcagcccagc acataatcct 540

cttgacaaga acgcagtaat tactttggga gacttgaatg cggatggaaa gcgtcgcctg 600

ccaaatggac agttagatca cggtacagat tattaccgcg gcggatttgc cgcacatccg 660

aacgactttc cggtaagtgg gtggcagcaa actgtgacgg atactgatgt aaccaacttc 720

gcacaaactc tccgtgcgta cgaccgcatg atcttatcaa acgccacgca caatagttac 780

gacgagtatg ggatcatcgg cggcatccct aaccctctcc cctacccggg tggcgtttac 840

cccgccggaa cgttgttcaa gaacgtgcat acaggcggtg gcaacacagg aaaggctctc 900

agtgatcacc gtctgatctg gtctaagtta aattacgggg ccattgaaac tacggacaac 960

aacggcaaca ctaaaagcaa ttttgtcgag ttcaccaacc cacctcagtg cactgcacca 1020

gacacggttt atgctaaagg cgccgacttt gtgcccaatc gtacagtgga tatctatatt 1080

actaagcagc ccgacaacca aaatttcatt cccctgccgc aaccccaaaa tcctacgaat 1140

```
cctgggcaat tcaaacaagt tggggaatca taccaattga cggacgtccg taccaccggc      1200
aagtccaccc acaatatcga cgctaacggc aacattgcta acacggctgt atgggtgcag      1260
ccaacaccaa accaacagta caatctgatt gtggatgtgg atcgcgacgg ctacttcacg      1320
aactcgatcg acatcgtaga tgcattcaat acgcagggat ttgatgtcga tccatgtttg      1380
cgcaaacgtc tggcagcgtc actgtccgac ggcatccccg caggcgatgg cacgcgtgac      1440
tttgtcaaac ctccaaagat tttcatctca ggcgattggt ttccacaggg cagtgcccgc      1500
atctacatca ttatctatga caagaatttc gattataacg gaaacgacag taaggttctg      1560
actgatgttc gtggcactta tgacacaatc acggtaggag aaaaccataa gtttaccggc      1620
ttagaatgga gttctcccca cgttggcaac tataatatta tctttgatgc taatagcgat      1680
ggcgtttaca agaaatcaga tggtgatatt gtagaccaca ttaacgagat cgggttcgcc      1740
atgttggagt cttatccaca ccactcgcac attgttgact tggatgacaa tgcgaatcaa      1800
cgtgaagatt tcaacagcgg cattgctaag aacgtatacg tcttagccac gaacttaccg      1860
aacccctcca caatcgtaga cgtttacgtg atttcagaaa agttgttgaa actcaataat      1920
cctggttgga ccacctggca acaatcctca aacgtgccac ttaaggagag tgcggcaccg      1980
acaaacaatg gtgaccgtct gcatccaacg tgggtaactc cgcagaagac ggtatttggt      2040
agcgtctggc acatgccggg cgatacattc gacccgcctt acatcaatta ttatggaaag      2100
aagtttacaa tcgtcattga cgttaaccgc gacggcaagt acgacccatc aattgatatg      2160
gtagatactc atgacattgg tgacatgact gactggttca ttacgcaagg gcaccacgat      2220
ctgtcaccga cttcgggcaa cggtcaaccg gccgtgtctg aatacaaaga gtacctgaat      2280
agtaaactcg acctggaaaa tccattagat aacaccaata attatgatgc agccaccgaa      2340
gcagcttctg cccaatattt atgtcgtacc aaccttccga tcaatctgtt cgagaatgta      2400
attgagcttg gcgcgcagac cggcttcatt atgttcaacc ccgatgagta ttatgccggg      2460
cgtgatctta atagtggccg ccatatctat gatgcagtgg acttcgatgg attggagatt      2520
aatgacggat atacgtctgt gatcagtgca aaagaaatga cgttaaaccg cctcggtgtt      2580
ggccaaaata gtaacctttt gacttgcggc gcagacaccg tgacaatcga tggaatgtct      2640
actcaaaagg attctacgac aaacattgta gcaaagacaa tctctcacaa aagtaacatt      2700
tcgctggagg cggatacgtg cacccactat acgcataact tatattttga atccgatacg      2760
aatatcttgg ttaaggcaat tgagtcccca ttcgcttgga ttattgacgt gggtgatcgt      2820
attctgcccg gcactggctg tctcgaga                                         2848
```

<210> 10
<211> 24
<212> PRT

<213> Spongosorites sp.

<220>
<221> misc_feature
<222> (21)..(21)
<223> Not determined

<400> 10

```
Lys Leu Gly Asp Gln Arg Gln Ile Asp Ile Ala Ser Trp Asn Thr Phe
1               5                   10                  15

Asp Gly Gly Val Xaa Lys Ala Asn
            20
```

<210> 11
<211> 3
<212> PRT
<213> Spongosorites sp.

<220>
<221> misc_feature
<222> (1)..(1)
<223> Not determined

<400> 11

```
Xaa Gly His
1
```

<210> 12
<211> 7
<212> PRT
<213> Spongosorites sp.

<220>
<221> misc_feature
<222> (4)..(6)
<223> Not determined

<400> 12

```
Thr Gly Ala Xaa Xaa Xaa Glu
1               5
```

<210> 13
<211> 5
<212> PRT
<213> Spongosorites sp.

<220>
<221> misc_feature
<222> (3)..(3)
<223> Not determined

<400> 13

Ser Gly Xaa Ser Thr
1               5

<210> 14
<211> 13
<212> PRT
<213> Spongosorites sp.

<220>
<221> misc_feature
<222> (1)..(1)
<223> Q or G

<220>
<221> misc_feature
<222> (8)..(8)
<223> Not determined

<220>
<221> misc_feature
<222> (10)..(10)
<223> Not determined

<220>
<221> misc_feature
<222> (12)..(12)
<223> Not determined

<400> 14

Xaa Ala Gly Phe Val Pro Asn Xaa Thr Xaa Asp Xaa Thr
1               5                   10

<210> 15
<211> 4
<212> PRT
<213> Spongosorites sp.

<400> 15

Asn Phe Asp Tyr
1

<210> 16
<211> 13
<212> PRT
<213> Spongosorites sp.

<220>
<221> misc_feature
<222> (12)..(12)
<223> Not determined

<400> 16

Leu Ala Leu Glu Val Pro Leu Arg Thr Val Asn Xaa Thr

1 5 10

<210> 17
<211> 14
<212> PRT
<213> Spongosorites sp.

<220>
<221> misc_feature
<222> (6)..(6)
<223> Not determined

<220>
<221> misc_feature
<222> (10)..(10)
<223> Not determined

<220>
<221> misc_feature
<222> (12)..(12)
<223> Not determined

<400> 17

Gly Asp Gly Glu Asn Xaa Asn Asp Asn Xaa Asp Xaa Tyr Asp
1 5 10

<210> 18
<211> 13
<212> PRT
<213> Spongosorites sp.

<220>
<221> misc_feature
<222> (9)..(9)
<223> Not determined

<220>
<221> misc_feature
<222> (11)..(12)
<223> Not determined

<400> 18

Ala Ser Thr Gly Ser Thr Ile Pro Xaa Gly Xaa Xaa Thr
1 5 10

<210> 19
<211> 9
<212> PRT
<213> Spongosorites sp.

<220>
<221> misc_feature
<222> (9)..(9)
<223> E or G

<400> 19

Glu Ser Ala Ala Glu Thr Glu Asn Xaa
1               5

<210> 20
<211> 19
<212> PRT
<213> Spongosorites sp.

<220>
<221> misc_feature
<222> (9)..(9)
<223> N or A

<220>
<221> misc_feature
<222> (10)..(10)
<223> Not determined

<220>
<221> misc_feature
<222> (17)..(17)
<223> Not determined

<220>
<221> misc_feature
<222> (19)..(19)
<223> E or G

<400> 20

Ala Ser Ala Ala Pro Thr Asn Asn Xaa Xaa Thr Ser Leu Ser Ser Gly
1               5                   10                  15

Xaa Asp Xaa

<210> 21
<211> 8
<212> PRT
<213> Spongosorites sp.

<220>
<221> misc_feature
<222> (1)..(1)
<223> Not determined

<220>
<221> misc_feature
<222> (4)..(4)
<223> D or E

<400> 21

Xaa Leu Asp Xaa Glu Thr Leu Glu
1               5

<210> 22
<211> 13
<212> PRT
<213> Spongosorites sp.

<220>
<221> misc_feature
<222> (9)..(9)
<223> D or E

<220>
<221> misc_feature
<222> (10)..(12)
<223> Not determined

<400> 22

Pro Leu Asp Val Arg Gly Thr Tyr Xaa Xaa Xaa Xaa Val
1 5 10

<210> 23
<211> 14
<212> PRT
<213> Spongosorites sp.

<220>
<221> misc_feature
<222> (2)..(2)
<223> Not determined

<220>
<221> misc_feature
<222> (10)..(10)
<223> Not determined

<220>
<221> misc_feature
<222> (13)..(13)
<223> Not determined

<400> 23

His Xaa Gln Asp Arg Ile Gln Pro Ala Xaa Pro Pro Xaa His
1 5 10

<210> 24
<211> 33
<212> PRT
<213> Spongosorites sp.

<220>
<221> misc_feature
<222> (25)..(25)
<223> A or G

<220>
<221> misc_feature

<222> (27)..(27)
<223> Not determined

<220>
<221> misc_feature
<222> (32)..(32)
<223> Not determined

<400> 24

Ala Asn Thr Gly Ile Gly Asn Val Val Ile Glu Arg Thr Asp Asn Pro
1 5 10 15

Asn Thr Val Pro Tyr Ile Pro Ala Xaa Gly Xaa Asn Asn Asn His Xaa
20 25 30

His

<210> 25
<211> 15
<212> PRT
<213> Spongosorites sp.

<220>
<221> misc_feature
<222> (14)..(14)
<223> Not determined

<400> 25

Phe Ala Val Ile Thr Leu Gly Asp Leu Asn Ala Asp Gly Xaa His
1 5 10 15

<210> 26
<211> 3
<212> PRT
<213> Spongosorites sp.

<400> 26

Leu Pro Gly
1

<210> 27
<211> 21
<212> DNA
<213> Artificial

<220>
<223> Synthetic

<220>
<221> misc_feature
<222> (6)..(6)
<223> n is a, c, g, or t

<220>
<221> misc_feature
<222> (21)..(21)
<223> n is a, c, g, or t

<400> 27
carmgncara thgayathgc n 21

<210> 28
<211> 20
<212> DNA
<213> Artificial

<220>
<223> Synthetic

<220>
<221> misc_feature
<222> (3)..(3)
<223> n is a, c, g, or t

<220>
<221> misc_feature
<222> (6)..(6)
<223> n is a, c, g, or t

<220>
<221> misc_feature
<222> (9)..(9)
<223> n is a, c, g, or t

<220>
<221> misc_feature
<222> (12)..(12)
<223> n is a, c, g, or t

<220>
<221> misc_feature
<222> (15)..(15)
<223> n is a, c, g, or t

<220>
<221> misc_feature
<222> (18)..(18)
<223> n is a, c, g, or t

<400> 28
atngynswnc cngtnswngc 20

<210> 29
<211> 20
<212> DNA
<213> Artificial

<220>
<223> Synthetic

<220>

<221> misc_feature
<222> (9)..(9)
<223> n is a, c, g, or t

<400> 29
tggaayacnt tygayttygg 20

<210> 30
<211> 20
<212> DNA
<213> Artificial

<220>
<223> Synthetic

<220>
<221> misc_feature
<222> (3)..(3)
<223> n is a, c, g, or t

<220>
<221> misc_feature
<222> (6)..(6)
<223> n is a, c, g, or t

<220>
<221> misc_feature
<222> (9)..(9)
<223> n is a, c, g, or t

<220>
<221> misc_feature
<222> (18)..(18)
<223> n is a, c, g, or t

<400> 30
ccngcngcng gdatrtangg 20

<210> 31
<211> 28
<212> DNA
<213> Artificial

<220>
<223> Synthetic

<400> 31
caaatgtcac aaatccatgt aatcctgc 28

<210> 32
<211> 22
<212> DNA
<213> Artificial

<220>
<223> Synthetic

<400> 32
atgatcctac catactgcaa gc 22

<210> 33
<211> 23
<212> DNA
<213> Artificial

<220>
<223> Synthetic

<400> 33
gatactggca ttgtaggctc tgg 23

<210> 34
<211> 22
<212> DNA
<213> Artificial

<220>
<223> Synthetic

<400> 34
ggcactctct gatcatagac tg 22

<210> 35
<211> 25
<212> DNA
<213> Artificial

<220>
<223> Synthetic

<400> 35
cagctgatga ggtatacgtc aaagg 25

<210> 36
<211> 28
<212> DNA
<213> Artificial

<220>
<223> Synthetic

<400> 36
aatctcttga accaaaatga catcatgc 28

<210> 37
<211> 23
<212> DNA
<213> Artificial

<220>
<223> Synthetic

<400> 37
cttacataca ccgccaaact tgc 23

<210> 38

<211> 28
<212> DNA
<213> Artificial

<220>
<223> Synthetic

<400> 38
gtgctggttt tagatgtgtg gcttgtgc 28

<210> 39
<211> 28
<212> DNA
<213> Artificial

<220>
<223> Synthetic

<400> 39
tctggacata tcaaggccct gcaggtgg 28

<210> 40
<211> 28
<212> DNA
<213> Artificial

<220>
<223> Synthetic

<400> 40
ccattaaact tgaaaacatg acagggcc 28

<210> 41
<211> 28
<212> DNA
<213> Artificial

<220>
<223> Synthetic

<400> 41
tgatcttaca gtacaaccag cagcactg 28

<210> 42
<211> 45
<212> DNA
<213> Artificial

<220>
<223> Synthetic

<400> 42
ctaatacgac tcactatagg gcaagcagtg gtatcaacgc agagt 45

<210> 43
<211> 22
<212> DNA
<213> Artificial

<220>
<223> Synthetic

<400> 43
ctaatacgac tcactatagg gc 22

<210> 44
<211> 23
<212> DNA
<213> Artificial

<220>
<223> Synthetic

<400> 44
aagcagtggt atcaacgcag agt 23

<210> 45
<211> 27
<212> DNA
<213> Artificial

<220>
<223> Synthetic

<400> 45
gtcgacaagc ttggagatca gcgacag 27

<210> 46
<211> 27
<212> DNA
<213> Artificial

<220>
<223> Synthetic

<400> 46
gaattcctaa cagccagtac ctggaag 27

<210> 47
<211> 344
<212> DNA
<213> Artificial

<220>
<223> Synthetic

<400> 47
tggaatactt ttgactttgg tggcaagttt ggcggtgtat gtaaggcaaa tgatcctacc 60

atactgcaag caatgactga tgtaatatct cagcatgatg tcattttggt tcaagagatt 120

gcaaatgtca caaatccatg taatcctgca aacatctttt caggctttaa taatttatgt 180

tcaaataact atcttggcgg tctgggatat ctctgccaag atactggcat tgtaggctct 240

ggtgagggct atggtattat ctacaaaaac aacaccggta ttggtaatgt agtaattgag 300

cgtacagata acccaaatac agttccatat ataccagcag cagg 344

<210> 48
<211> 115
<212> PRT
<213> Artificial

<220>
<223> Synthetic

<400> 48

Trp Asn Thr Phe Asp Phe Gly Gly Lys Phe Gly Gly Val Cys Lys Ala
1 5 10 15

Asn Asp Pro Thr Ile Leu Gln Ala Met Thr Asp Val Ile Ser Gln His
20 25 30

Asp Val Ile Leu Val Gln Glu Ile Ala Asn Val Thr Asn Pro Cys Asn
35 40 45

Pro Ala Asn Ile Phe Ser Gly Phe Asn Asn Leu Cys Ser Asn Asn Tyr
50 55 60

Leu Gly Gly Leu Gly Tyr Leu Cys Gln Asp Thr Gly Ile Val Gly Ser
65 70 75 80

Gly Glu Gly Tyr Gly Ile Ile Tyr Lys Asn Asn Thr Gly Ile Gly Asn
85 90 95

Val Val Ile Glu Arg Thr Asp Asn Pro Asn Thr Val Pro Tyr Ile Pro
100 105 110

Ala Ala Gly
115

<210> 49
<211> 864
<212> DNA
<213> Artificial

<220>
<223> Synthetic

<400> 49
gatactggca ttgtaggctc tggtgagggc tatggtatta tctacaaaaa caacaccggt 60

attggtaatg tagtaattga gcgtacagat aacccaaata cagttccata cattcctgca 120

ggtggtggca acaaccaaat gaagaggcca ccaatgaagg caacccttga ctttaatgca 180

ggtgcattca acgtagtagt atataatgca cacacaagcc caagtggcgg tgttcccaaa 240

gagataaagc tactcagtga tagaatatcg cctgctcaca atccgctaga taaaaacgca 300

```
gtaattacac taggtgatct taatgccgac ggcaaaagaa gactgcctaa tggacagcta      360

gaccatggaa cagactatta cagaggtggc tttgcagctc acccaaatga ctttcctgtt      420

agtggatggc agcagacagt tactgatact gatgttacaa actttgcaca gacacttcgt      480

gcatatgata gaatgatact tagcaatgca acacataact cttatgatga gtatggaata      540

ataggtggca ttccaaaccc acttccatat ccgggaggag tttatcctgc tggaactttg      600

tttaaaaatg tacatactgg cggtggaaat acaggaaagg cactctctga tcatagactg      660

atatggtcaa agcttaacta tggtgcaata gagactaccg ataataacgg caatacaaag      720

tccaattttg ttgaatttac aaacccacca caatgtactg caccagatac cgtatatgca      780

aagggtgcag actttgtacc aaatagaaca gttgatattt acatcacaaa gcagccagac      840

aatcaaaact ttatcccact gcca                                             864


<210>  50
<211>  295
<212>  PRT
<213>  Artificial

<220>
<223>  Synthetic

<400>  50

Asp Thr Gly Ile Val Gly Ser Gly Glu Gly Tyr Gly Ile Ile Tyr Lys
1               5                   10                  15


Asn Asn Thr Gly Ile Gly Asn Val Val Ile Glu Arg Thr Asp Asn Pro
            20                  25                  30


Asn Thr Val Pro Tyr Ile Pro Ala Gly Gly Gly Asn Asn Gln Met Lys
        35                  40                  45


Arg Pro Pro Met Lys Ala Thr Leu Asp Phe Asn Ala Gly Ala Phe Asn
    50                  55                  60


Val Val Val Tyr Asn Ala His Thr Ser Pro Ser Gly Gly Val Pro Lys
65                  70                  75                  80


Glu Ile Lys Leu Leu Ser Asp Arg Ile Ser Pro Ala His Asn Pro Leu
                85                  90                  95


Asp Lys Asn Ala Val Ile Thr Leu Gly Asp Leu Asn Ala Asp Gly Lys
            100                 105                 110


Arg Arg Leu Pro Asn Gly Gln Leu Asp His Gly Thr Asp Tyr Tyr Arg
        115                 120                 125
```

Gly Gly Phe Ala Ala His Pro Asn Asp Phe Pro Val Ser Gly Trp Gln
130 135 140

Gln Thr Val Thr Asp Thr Asp Val Thr Asn Phe Ala Gln Thr Leu Arg
145 150 155 160

Ala Tyr Asp Arg Met Ile Leu Ser Asn Ala Thr His Asn Ser Tyr Asp
165 170 175

Glu Tyr Gly Ile Ile Gly Gly Ile Pro Asn Pro Leu Pro Tyr Pro Gly
180 185 190

Gly Val Tyr Pro Ala Gly Thr Leu Phe Lys Asn Val His Thr Gly Gly
195 200 205

Gly Asn Thr Gly Lys Ala Leu Ser Asp His Arg Leu Ile Trp Ser Lys
210 215 220

Leu Asn Tyr Gly Ala Ile Glu Thr Thr Asp Asn Asn Gly Asn Thr Lys
225 230 235 240

Ser Asn Phe Val Glu Phe Thr Asn Pro Pro Gln Cys Thr Ala Pro Asp
245 250 255

Thr Val Tyr Ala Lys Gly Ala Asp Phe Val Pro Asn Arg Thr Val Asp
260 265 270

Ile Tyr Ile Thr Lys Gln Pro Asp Asn Gln Asn Phe Ile Pro Leu Pro
275 280 285

Glu Pro Thr Met Pro Val Ser
290 295

<210> 51
<211> 2511
<212> DNA
<213> Artificial

<220>
<223> Synthetic

<400> 51
ggcactctct gatcatagac tgatatggtc aaagcttaac tatggtgcaa tagagactac 60

cgataataac ggcaatacaa agtccaattt tgttgaattt acaaacccac cacaatgtac 120

tgcaccagat accgtatatg caaagggtgc agactttgta ccaaatagaa cagttgatat 180

ttacatcaca aagcagccag acaatcaaaa ctttatccca ctgccacagc cacaaaaccc 240

aacaaaccct gggcagttca agcaagttgg cgaatcatac cagctaacag atgtaaggac 300

tacaggcaaa agtacgcaca acattgatgc aaacggcaac attgcaaata ctgctgtatg 360
ggtacagcca acaccaaacc agcagtacta cctcatagtt gatgtggacc gtgatggata 420
ctttacaaac agcatagata tagttgatgc atttaacaca caaggctttg atgtagaccc 480
atgccttcga aagagactag cagcatcact ttctgatgga ataccagcag gcgatggcac 540
acgtgacttt gtaaaacctc ccaaaatatt tatttccggt gactggttcc cacagggttc 600
tgcacgtatc tacattatca tatatgacaa gaactttgat tataacggca atgactccaa 660
agtattaact gacgtccgtg gaacttatga cacaattaca gtgggtgaga accacaagtt 720
tacaggacta gaatggtcct caccacatgt tggtaactat aacataatat ttgatgcaaa 780
ctctgatgga gtatacaaaa aatccgatgg tgacattgta gatcacataa acgagatagg 840
ctttgcaatg ctcgaatcgt atccacatca cagccatatt gtagatttag atgacaatgc 900
aaaccagcga gaagacttta acagtggaat tgcaaaaaac gtctacgttc ttgcaactaa 960
tcttccaaac ccaagcacaa tagttgacgt ttatgtaatt agtgagaaac tcttaaagct 1020
aaacaatcct ggatggacca catggcagca atcctctaat gtgctactaa aggagagtgc 1080
agcaccaact aacaatggag acaggctaca ccctacttgg gtaactccac aaaagacagt 1140
atttggttca gtatggcata tgcccggtga tacatttgat ccaccataca ttaattatta 1200
cggcaaaaaa ttcactatag taattgatgt gaatcgtgat ggtaagtatg atcctagtat 1260
agatatggta gatactcacg atataggaga catgacagac tggtttatca cacagggtca 1320
ccatgaccta agcccaacta gtggaaacgg acagcctgcc gtatctgaat acaaagagta 1380
tctcaactca aagctagact tggagaaccc gcttgataac accaacaact atgatgctgc 1440
aactgaggca gcttctgcac aatatctatg ccgtaccaat cttccaatca atctatttga 1500
gaatgtcata gagctgggag ctcagacagg ctttatcatg tttaaccctg atgagtatta 1560
tgcaggccgt gatctaaatt ctggacgcca catttatgat gctgtagatt ttgatggctt 1620
ggagataaat gacggataca catcagtaat ctctgcaaaa gagatgaccc taaacagatt 1680
aggtgttggc caaaactcca atctgcttac ctgcggagct gacacagtaa cgatagatgg 1740
catgtcaaca caaaaagatt caactacaaa tattgttgca aagaccatat ctcacaaaag 1800
taacatctca ctagaggcag acacttgtac acattatact cacaatctct actttgagtc 1860
agacaccaac atcttagtta aggccattga gagtccattt gcatggataa ttgacgtagg 1920
tgacagaata cttccaggta ctggctgtta gatgaatcgt gtgtgtgggt tcttctaata 1980
tgattgcaca agccacacat ctaaaaccag cacaatgcac acacaccttc tgctgtttta 2040
taactttaat acattatgac attttactct aaattgcaat aatagttaat cagtgtatat 2100
actcaaaaac aaaaaactat ccctcatacc attgagtgct atgatattat ttttgggaat 2160

```
aataataata ataatggggg ggggcatctt cgcagtcctt ttctcaaact gatatataga     2220

taaactaaat gacaattcac aagatctggg taatctatag attacttaac atgatgttat     2280

catgctatcc tttctatggg ggccacctgc agggccttga tatgtccaga taaaaaacag     2340

gtcaaattac gtcccacctt ttgtacagat ctcctactat cgaatatcta aaataacggc     2400

taaaatagcc aaacgctagg aaggttatct aatgagctta aaaaaaaaac aagaaacaaa     2460

aaaaaaaaaa aaaaaaaaaa aaaaaagtac tctgcgttga taccactgct t              2511


<210>  52
<211>  649
<212>  PRT
<213>  Artificial

<220>
<223>  Synthetic

<400>  52

Ala Leu Ser Asp His Arg Leu Ile Trp Ser Lys Leu Asn Tyr Gly Ala
1               5                   10                  15

Ile Glu Thr Thr Asp Asn Asn Gly Asn Thr Lys Ser Asn Phe Val Glu
            20                  25                  30

Phe Thr Asn Pro Pro Gln Cys Thr Ala Pro Asp Thr Val Tyr Ala Lys
        35                  40                  45

Gly Ala Asp Phe Val Pro Asn Arg Thr Val Asp Ile Tyr Ile Thr Lys
    50                  55                  60

Gln Pro Asp Asn Gln Asn Phe Ile Pro Leu Pro Gln Pro Gln Asn Pro
65                  70                  75                  80

Thr Asn Pro Gly Gln Phe Lys Gln Val Gly Glu Ser Tyr Gln Leu Thr
                85                  90                  95

Asp Val Arg Thr Thr Gly Lys Ser Thr His Asn Ile Asp Ala Asn Gly
            100                 105                 110

Asn Ile Ala Asn Thr Ala Val Trp Val Gln Pro Thr Pro Asn Gln Gln
        115                 120                 125

Tyr Tyr Leu Ile Val Asp Val Asp Arg Asp Gly Tyr Phe Thr Asn Ser
    130                 135                 140

Ile Asp Ile Val Asp Ala Phe Asn Thr Gln Gly Phe Asp Val Asp Pro
145                 150                 155                 160
```

```
Cys Leu Arg Lys Arg Leu Ala Ala Ser Leu Ser Asp Gly Ile Pro Ala
                165                 170                 175

Gly Asp Gly Thr Arg Asp Phe Val Lys Pro Pro Lys Ile Phe Ile Ser
            180                 185                 190

Gly Asp Trp Phe Pro Gln Gly Ser Ala Arg Ile Tyr Ile Ile Ile Tyr
        195                 200                 205

Asp Lys Asn Phe Asp Tyr Asn Gly Asn Asp Ser Lys Val Leu Thr Asp
    210                 215                 220

Val Arg Gly Thr Tyr Asp Thr Ile Thr Val Gly Glu Asn His Lys Phe
225                 230                 235                 240

Thr Gly Leu Glu Trp Ser Ser Pro His Val Gly Asn Tyr Asn Ile Ile
                245                 250                 255

Phe Asp Ala Asn Ser Asp Gly Val Tyr Lys Lys Ser Asp Gly Asp Ile
            260                 265                 270

Val Asp His Ile Asn Glu Ile Gly Phe Ala Met Leu Glu Ser Tyr Pro
        275                 280                 285

His His Ser His Ile Val Asp Leu Asp Asp Asn Ala Asn Gln Arg Glu
    290                 295                 300

Asp Phe Asn Ser Gly Ile Ala Lys Asn Val Tyr Val Leu Ala Thr Asn
305                 310                 315                 320

Leu Pro Asn Pro Ser Thr Ile Val Asp Val Tyr Val Ile Ser Glu Lys
                325                 330                 335

Leu Leu Lys Leu Asn Asn Pro Gly Trp Thr Thr Trp Gln Gln Ser Ser
            340                 345                 350

Asn Val Leu Leu Lys Glu Ser Ala Ala Pro Thr Asn Asn Gly Asp Arg
        355                 360                 365

Leu His Pro Thr Trp Val Thr Pro Gln Lys Thr Val Phe Gly Ser Val
    370                 375                 380

Trp His Met Pro Gly Asp Thr Phe Asp Pro Pro Tyr Ile Asn Tyr Tyr
385                 390                 395                 400

Gly Lys Lys Phe Thr Ile Val Ile Asp Val Asn Arg Asp Gly Lys Tyr
                405                 410                 415
```

Asp Pro Ser Ile Asp Met Val Asp Thr His Asp Ile Gly Asp Met Thr
420 425 430

Asp Trp Phe Ile Thr Gln Gly His His Asp Leu Ser Pro Thr Ser Gly
435 440 445

Asn Gly Gln Pro Ala Val Ser Glu Tyr Lys Glu Tyr Leu Asn Ser Lys
450 455 460

Leu Asp Leu Glu Asn Pro Leu Asp Asn Thr Asn Asn Tyr Asp Ala Ala
465 470 475 480

Thr Glu Ala Ala Ser Ala Gln Tyr Leu Cys Arg Thr Asn Leu Pro Ile
485 490 495

Asn Leu Phe Glu Asn Val Ile Glu Leu Gly Ala Gln Thr Gly Phe Ile
500 505 510

Met Phe Asn Pro Asp Glu Tyr Tyr Ala Gly Arg Asp Leu Asn Ser Gly
515 520 525

Arg His Ile Tyr Asp Ala Val Asp Phe Asp Gly Leu Glu Ile Asn Asp
530 535 540

Gly Tyr Thr Ser Val Ile Ser Ala Lys Glu Met Thr Leu Asn Arg Leu
545 550 555 560

Gly Val Gly Gln Asn Ser Asn Leu Leu Thr Cys Gly Ala Asp Thr Val
565 570 575

Thr Ile Asp Gly Met Ser Thr Gln Lys Asp Ser Thr Thr Asn Ile Val
580 585 590

Ala Lys Thr Ile Ser His Lys Ser Asn Ile Ser Leu Glu Ala Asp Thr
595 600 605

Cys Thr His Tyr Thr His Asn Leu Tyr Phe Glu Ser Asp Thr Asn Ile
610 615 620

Leu Val Lys Ala Ile Glu Ser Pro Phe Ala Trp Ile Ile Asp Val Gly
625 630 635 640

Asp Arg Ile Leu Pro Gly Thr Gly Cys
645

<210> 53
<211> 1607

<212> DNA
<213> Artificial

<220>
<223> Synthetic

<400> 53
gatactggca ttgtaggctc tggtgagggc tatggtatta tctacaaaaa caacaccggt 60

attggtaatg tagtaattga gcgtacagat aacccaaata cagttccata caggcaatga 120

ctatagatag tacaggcaag gtaggaattg gaactactag tccaaatgca aaattacatg 180

tacaaggatc atttagagtc caaggctata acatcttaac tgaccctgat gaacaataca 240

acagtcattt tcctcattca agtaataatg tctacatctc cggtgataaa acatatctta 300

gaagtggcaa accaaacggt tactccacta caatgactgt tgataacatc aatggtaatg 360

taggcattgg actaaaagag cccaaagtaa aacttcatat gtttggtgat gcctttttgc 420

aaacaggctc ttctggcgat tcaagaaatt tgcaattaag cagtttctat acttcacaac 480

caaaagctat caatcttgag agtcgttact ttgatacgat agagggattc aaacttggag 540

caagtggaga tagttttgtc catgacaagt tctacataaa tagatacact gcccatgtaa 600

atgacaatcc cggaattaca actaacaacg ttgattcaac cagactcgtt acagttacag 660

caactgggaa tgtaggaatt ggaactacta gtccaagtaa gacacttgat gtaaatggcg 720

atgccattgc aaataactgg cttagacatt ccagccagca atggaaaaca gacatagcac 780

cactagataa ctccctaagc aagctagagc agctacaagg cataagctac aagtggaaag 840

cagatgaatt cgcagacatg ggatttcaca ataaaacaac tcttgggttt attgccgaag 900

atgtagagac tatacttcca cagctaatcc atactgacca aaatggtgaa aaatcaatgg 960

actatggtaa actaacacca attctagttg aagcaatcaa agagcagcaa aacaccatag 1020

aacagctaac cactgcaata tgtaaagaca gcccaaaaca tgacgtgtgc agcagctaaa 1080

aaacactttt ttttaatttt ttgatcttac agtacaacca gcagcactga aacatttttg 1140

gcatcgaatc taaaaagaaa aaaaaatcat actatcacat aatgatgata tgctgtattg 1200

tgagaaactc aaagacactg aaaaacattt ttggcatcga atctaaaaag aaaaaaaaat 1260

catactatca cataatgatg atgatgatgc tgtattgtga gaaactcaaa gacactgaaa 1320

cattttatac aatccattaa acttgaaaac atgacagggc caacaaatct caaaatactt 1380

cctatatacc ttacacgcca taacacttct cattgaaacg acaagatagt agtattacac 1440

atcagactgt gtcaattttt tgcattacca tcttgctttt atcagttagt gcatactttc 1500

ctaattctat tccagaggcg caagctaagc ttggagatca gcgacagata gatattgcaa 1560

gctggaacac atttgatttt ggtggcaagt ttggcggtgt atgtaag 1607

<210> 54

<211> 37
<212> PRT
<213> Artificial

<220>
<223> Synthetic

<400> 54

Asp Thr Gly Ile Val Gly Ser Gly Glu Gly Tyr Gly Ile Ile Tyr Lys
1 5 10 15

Asn Asn Thr Gly Ile Gly Asn Val Val Ile Glu Arg Thr Asp Asn Pro
20 25 30

Asn Thr Val Pro Tyr
35

<210> 55
<211> 68
<212> PRT
<213> Artificial

<220>
<223> Synthetic

<400> 55

His Phe Ser Leu Lys Arg Gln Asp Ser Ser Ile Thr His Gln Thr Val
1 5 10 15

Ser Ile Phe Cys Ile Thr Ile Leu Leu Leu Ser Val Ser Ala Tyr Phe
20 25 30

Pro Asn Ser Ile Pro Glu Ala Gln Ala Lys Leu Gly Asp Gln Arg Gln
35 40 45

Ile Asp Ile Ala Ser Trp Asn Thr Phe Asp Phe Gly Gly Lys Phe Gly
50 55 60

Gly Val Cys Lys
65

<210> 56
<211> 3099
<212> DNA
<213> Artificial

<220>
<223> Synthetic

<400> 56
ccattaaact tgaaaacatg acagggccaa caaatctcaa aatacttcct atatacctta 60

cacgccataa cacttctcat tgaaacgaca agatagtagt attacacatc agactgtgtc 120

aatttttgc attaccatct tgcttttatc agttagtgca tactttccta attctattcc 180

agaggcgcaa gctaagcttg gagatcagcg acagatagat attgcaagct ggaacacatt 240

tgattttggt ggcaagtttg gcggtgtatg taaggcaaat gatcctacca tactgcaagc 300

aatgactgat gtaatatctc agcatgatgt cattttggtt caagagattg caaatgtcac 360

aaatccatgt aatcctgcaa acatcttttc aggctttaat aatttatgtt caaataacta 420

tcttggcggt ctgggatatc tctgccaaga tactggcatt gtaggctctg gtgagggcta 480

tggtattatc tacaaaaaca acaccggtat tggtaatgta gtaattgagc gtacagataa 540

cccaaataca gttccataca ttcctgcagg tggtggcaac aaccaaatga agaggccacc 600

aatgaaggca acccttgact ttaatgcagg tgcattcaac gtagtagtat ataatgcaca 660

cacaagccca agtggcggtg ttcccaaaga gataaagcta ctcagtgata gaatatcgcc 720

tgctcacaat ccgctagata aaaacgcagt aattacacta ggtgatctta atgccgacgg 780

caaaagaaga ctgcctaatg gacagctaga ccatggaaca gactattaca gaggtggctt 840

tgcagctcac ccaaatgact ttcctgttag tggatggcag cagacagtta ctgatactga 900

tgttacaaac tttgcacaga cacttcgtgc atatgataga atgatactta gcaatgcaac 960

acataactct tatgatgagt atggaataat aggtggcatt ccaaacccac ttccatatcc 1020

gggaggagtt tatcctgctg gaactttgtt taaaaatgta catactggcg gtggaaatac 1080

aggaaaggca ctctctgatc atagactgat atggtcaaag cttaactatg gtgcaataga 1140

gactaccgat aataacggca atacaaagtc caattttgtt gaatttacaa acccaccaca 1200

atgtactgca ccagataccg tatatgcaaa gggtgcagac tttgtaccaa atagaacagt 1260

tgatatttac atcacaaagc agccagacaa tcaaaacttt atcccactgc cacagccaca 1320

aaacccaaca aaccctgggc agttcaagca agttggcgaa tcataccagc taacagatgt 1380

aaggactaca ggcaaaagta cgcacaacat tgatgcaaac ggcaacattg caaatactgc 1440

tgtatgggta cagccaacac caaaccagca gtacaacctc atagttgatg tggaccgtga 1500

tggatacttt acaaacagca tagatatagt tgatgcattt aacacacaag gctttgatgt 1560

agacccatgc cttcgaaaga gactagcagc atcactttct gatggaatac cagcaggcga 1620

tggcacacgt gactttgtaa aacctcccaa aatatttatt tccggtgact ggttcccaca 1680

gggttctgca cgtatctaca ttatcatata tgacaagaac tttgattata acggcaatga 1740

ctccaaagta ttaactgacg tccgtggaac ttatgacaca attacagtgg gtgagaacca 1800

caagtttaca ggactagaat ggtcctcacc acatgttggt aactataaca taatatttga 1860

tgcaaactct gatggagtat acaaaaaatc cgatggtgac attgtagatc acataaacga 1920

gataggcttt gcaatgctcg aatcgtatcc acatcacagc catattgtag atttagatga 1980

caatgcaaac cagcgagaag actttaacag tggaattgca aaaaacgtct acgttcttgc 2040

aactaatctt ccaaacccaa gcacaatagt tgacgtttat gtaattagtg agaaactctt 2100

aaagctaaac aatcctggat ggaccacatg gcagcaatcc tctaatgtgc cactaaagga 2160

gagtgcagca ccaactaaca atggagacag gctacaccct acttgggtaa ctccacaaaa 2220

gacagtattt ggttcagtat ggcatatgcc cggtgataca tttgatccac catacattaa 2280

ttattacggc aaaaaattca ctatagtaat tgatgtgaat cgtgatggta agtatgatcc 2340

tagtatagat atggtagata ctcacgatat aggagacatg acagactggt ttatcacaca 2400

gggtcaccat gacctaagcc caactagtgg aaacggacag cctgccgtat ctgaatacaa 2460

agagtatctc aactcaaagc tagacttgga gaacccgctt gataacacca acaactatga 2520

tgctgcaact gaggcagctt ctgcacaata tctatgccgt accaatcttc caatcaatct 2580

atttgagaat gtcatagagc tgggagctca gacaggcttt atcatgttta accctgatga 2640

gtattatgca ggccgtgatc taaattctgg acgccacatt tatgatgctg tagattttga 2700

tggcttggag ataaatgacg gatacacatc agtaatctct gcaaaagaga tgaccctaaa 2760

cagattaggt gttggccaaa actccaatct gcttacctgc ggagctgaca cagtaacgat 2820

agatggcatg tcaacacaaa aagattcaac tacaaatatt gttgcaaaga ccatatctca 2880

caaaagtaac atctcactag aggcagacac ttgtacacat tatactcaca atctctactt 2940

tgagtcagac accaacatct tagttaaggc cattgagagt ccatttgcat ggataattga 3000

cgtaggtgac agaatacttc caggtactgg ctgttagatg aatcgtgtgt gtgggttctt 3060

ctaatatgat tgcacaagcc acacatctaa aaccagcac 3099

<210> 57
<211> 988
<212> PRT
<213> Artificial

<220>
<223> Synthetic

<400> 57

His Phe Ser Leu Lys Arg Gln Asp Ser Ser Ile Thr His Gln Thr Val
1 5 10 15

Ser Ile Phe Cys Ile Thr Ile Leu Leu Leu Ser Val Ser Ala Tyr Phe
20 25 30

Pro Asn Ser Ile Pro Glu Ala Gln Ala Lys Leu Gly Asp Gln Arg Gln
35 40 45

Ile Asp Ile Ala Ser Trp Asn Thr Phe Asp Phe Gly Gly Lys Phe Gly
50 55 60

```
Gly Val Cys Lys Ala Asn Asp Pro Thr Ile Leu Gln Ala Met Thr Asp
65                  70                  75                  80

Val Ile Ser Gln His Asp Val Ile Leu Val Gln Glu Ile Ala Asn Val
                85                  90                  95

Thr Asn Pro Cys Asn Pro Ala Asn Ile Phe Ser Gly Phe Asn Asn Leu
            100                 105                 110

Cys Ser Asn Asn Tyr Leu Gly Gly Leu Gly Tyr Leu Cys Gln Asp Thr
        115                 120                 125

Gly Ile Val Gly Ser Gly Glu Gly Tyr Gly Ile Ile Tyr Lys Asn Asn
    130                 135                 140

Thr Gly Ile Gly Asn Val Val Ile Glu Arg Thr Asp Asn Pro Asn Thr
145                 150                 155                 160

Val Pro Tyr Ile Pro Ala Gly Gly Gly Asn Asn Gln Met Lys Arg Pro
                165                 170                 175

Pro Met Lys Ala Thr Leu Asp Phe Asn Ala Gly Ala Phe Asn Val Val
            180                 185                 190

Val Tyr Asn Ala His Thr Ser Pro Ser Gly Gly Val Pro Lys Glu Ile
        195                 200                 205

Lys Leu Leu Ser Asp Arg Ile Ser Pro Ala His Asn Pro Leu Asp Lys
    210                 215                 220

Asn Ala Val Ile Thr Leu Gly Asp Leu Asn Ala Asp Gly Lys Arg Arg
225                 230                 235                 240

Leu Pro Asn Gly Gln Leu Asp His Gly Thr Asp Tyr Tyr Arg Gly Gly
                245                 250                 255

Phe Ala Ala His Pro Asn Asp Phe Pro Val Ser Gly Trp Gln Gln Thr
            260                 265                 270

Val Thr Asp Thr Asp Val Thr Asn Phe Ala Gln Thr Leu Arg Ala Tyr
        275                 280                 285

Asp Arg Met Ile Leu Ser Asn Ala Thr His Asn Ser Tyr Asp Glu Tyr
    290                 295                 300

Gly Ile Ile Gly Gly Ile Pro Asn Pro Leu Pro Tyr Pro Gly Gly Val
```

305 310 315 320

Tyr Pro Ala Gly Thr Leu Phe Lys Asn Val His Thr Gly Gly Gly Asn
325 330 335

Thr Gly Lys Ala Leu Ser Asp His Arg Leu Ile Trp Ser Lys Leu Asn
340 345 350

Tyr Gly Ala Ile Glu Thr Thr Asp Asn Asn Gly Asn Thr Lys Ser Asn
355 360 365

Phe Val Glu Phe Thr Asn Pro Pro Gln Cys Thr Ala Pro Asp Thr Val
370 375 380

Tyr Ala Lys Gly Ala Asp Phe Val Pro Asn Arg Thr Val Asp Ile Tyr
385 390 395 400

Ile Thr Lys Gln Pro Asp Asn Gln Asn Phe Ile Pro Leu Pro Gln Pro
405 410 415

Gln Asn Pro Thr Asn Pro Gly Gln Phe Lys Gln Val Gly Glu Ser Tyr
420 425 430

Gln Leu Thr Asp Val Arg Thr Thr Gly Lys Ser Thr His Asn Ile Asp
435 440 445

Ala Asn Gly Asn Ile Ala Asn Thr Ala Val Trp Val Gln Pro Thr Pro
450 455 460

Asn Gln Gln Tyr Asn Leu Ile Val Asp Val Asp Arg Asp Gly Tyr Phe
465 470 475 480

Thr Asn Ser Ile Asp Ile Val Asp Ala Phe Asn Thr Gln Gly Phe Asp
485 490 495

Val Asp Pro Cys Leu Arg Lys Arg Leu Ala Ala Ser Leu Ser Asp Gly
500 505 510

Ile Pro Ala Gly Asp Gly Thr Arg Asp Phe Val Lys Pro Pro Lys Ile
515 520 525

Phe Ile Ser Gly Asp Trp Phe Pro Gln Gly Ser Ala Arg Ile Tyr Ile
530 535 540

Ile Ile Tyr Asp Lys Asn Phe Asp Tyr Asn Gly Asn Asp Ser Lys Val
545 550 555 560

Leu Thr Asp Val Arg Gly Thr Tyr Asp Thr Ile Thr Val Gly Glu Asn
565 570 575

His Lys Phe Thr Gly Leu Glu Trp Ser Ser Pro His Val Gly Asn Tyr
580 585 590

Asn Ile Ile Phe Asp Ala Asn Ser Asp Gly Val Tyr Lys Lys Ser Asp
595 600 605

Gly Asp Ile Val Asp His Ile Asn Glu Ile Gly Phe Ala Met Leu Glu
610 615 620

Ser Tyr Pro His His Ser His Ile Val Asp Leu Asp Asp Asn Ala Asn
625 630 635 640

Gln Arg Glu Asp Phe Asn Ser Gly Ile Ala Lys Asn Val Tyr Val Leu
645 650 655

Ala Thr Asn Leu Pro Asn Pro Ser Thr Ile Val Asp Val Tyr Val Ile
660 665 670

Ser Glu Lys Leu Leu Lys Leu Asn Asn Pro Gly Trp Thr Thr Trp Gln
675 680 685

Gln Ser Ser Asn Val Pro Leu Lys Glu Ser Ala Ala Pro Thr Asn Asn
690 695 700

Gly Asp Arg Leu His Pro Thr Trp Val Thr Pro Gln Lys Thr Val Phe
705 710 715 720

Gly Ser Val Trp His Met Pro Gly Asp Thr Phe Asp Pro Pro Tyr Ile
725 730 735

Asn Tyr Tyr Gly Lys Lys Phe Thr Ile Val Ile Asp Val Asn Arg Asp
740 745 750

Gly Lys Tyr Asp Pro Ser Ile Asp Met Val Asp Thr His Asp Ile Gly
755 760 765

Asp Met Thr Asp Trp Phe Ile Thr Gln Gly His His Asp Leu Ser Pro
770 775 780

Thr Ser Gly Asn Gly Gln Pro Ala Val Ser Glu Tyr Lys Glu Tyr Leu
785 790 795 800

Asn Ser Lys Leu Asp Leu Glu Asn Pro Leu Asp Asn Thr Asn Asn Tyr
805 810 815

Asp Ala Ala Thr Glu Ala Ala Ser Ala Gln Tyr Leu Cys Arg Thr Asn
820 825 830

Leu Pro Ile Asn Leu Phe Glu Asn Val Ile Glu Leu Gly Ala Gln Thr
835 840 845

Gly Phe Ile Met Phe Asn Pro Asp Glu Tyr Tyr Ala Gly Arg Asp Leu
850 855 860

Asn Ser Gly Arg His Ile Tyr Asp Ala Val Asp Phe Asp Gly Leu Glu
865 870 875 880

Ile Asn Asp Gly Tyr Thr Ser Val Ile Ser Ala Lys Glu Met Thr Leu
885 890 895

Asn Arg Leu Gly Val Gly Gln Asn Ser Asn Leu Leu Thr Cys Gly Ala
900 905 910

Asp Thr Val Thr Ile Asp Gly Met Ser Thr Gln Lys Asp Ser Thr Thr
915 920 925

Asn Ile Val Ala Lys Thr Ile Ser His Lys Ser Asn Ile Ser Leu Glu
930 935 940

Ala Asp Thr Cys Thr His Tyr Thr His Asn Leu Tyr Phe Glu Ser Asp
945 950 955 960

Thr Asn Ile Leu Val Lys Ala Ile Glu Ser Pro Phe Ala Trp Ile Ile
965 970 975

Asp Val Gly Asp Arg Ile Leu Pro Gly Thr Gly Cys
980 985

<210> 58
<211> 930
<212> DNA
<213> Artificial

<220>
<223> Synthetic

<400> 58
aaactgggag atcagcggca aattgacatc gcttcatgga acacctttga ctttggcggg 60

aaattcggtg gtgtctgtaa agccaatgat ccgacgattc tccaggccat gactgatgtg 120

atttcccagc atgacgtcat cctggtgcag gagattgcca atgtgaccaa cccgtgtaat 180

cccgccaaca ttttctctgg gttcaataac ctgtgctcca acaactacct gggcggtctt 240

ggctatttgt gccaagacac gggcatcgtt ggtagtggcg aaggctacgg gattatctac 300

aagaataaca ccggaatcgg aaatgtggtc attgaacgta ccgataatcc gaatacggtt 360

ccgtatattc ccgctggtgg cggtaacaac cagatgaaac gcccaccgat gaaagcaacc 420

ctggacttca acgcaggcgc gtttaacgta gtggtctata acgcgcatac aagcccatca 480

ggtggtgtac cgaaagaaat caaactgctg tctgaccgca ttagccctgc ccataatccg 540

ttagacaaga acgcggtgat tactctgggc gatctgaatg cggatgggaa acgtcgctta 600

ccaaatggcc aactcgatca cgggacggat tactaccgtg gtggttttgc cgcgcatccg 660

aacgattttc cggtttcggg ctggcaacag acagtgacag atacggatgt tacgaacttt 720

gcgcagacct tacgtgcgta tgatcgcatg atccttagca atgcaaccca caactcgtac 780

gatgagtatg gcattatcgg cggaatccca aacccgttgc cttatccggg tggcgtttat 840

cctgctggga ccctgttcaa gaacgtacac actggcggtg gcaataccgg taaagcactg 900

agtgaccatc gcctgatttg gagcaaattg 930

<210> 59
<211> 948
<212> DNA
<213> Artificial

<220>
<223> Synthetic

<400> 59
gtcgacaaac tgggagatca gcggcaaatt gacatcgctt catggaacac ctttgacttt 60

ggcgggaaat tcggtggtgt ctgtaaagcc aatgatccga cgattctcca ggccatgact 120

gatgtgattt cccagcatga cgtcatcctg gtgcaggaga ttgccaatgt gaccaacccg 180

tgtaatcccg ccaacatttt ctctgggttc aataacctgt gctccaacaa ctacctgggc 240

ggtcttggct atttgtgcca agacacgggc atcgttggta gtggcgaagg ctacgggatt 300

atctacaaga ataacaccgg aatcggaaat gtggtcattg aacgtaccga taatccgaat 360

acggttccgt atattcccgc tggtggcggt aacaaccaga tgaaacgccc accgatgaaa 420

gcaaccctgg acttcaacgc aggcgcgttt aacgtagtgg tctataacgc gcatacaagc 480

ccatcaggtg gtgtaccgaa agaaatcaaa ctgctgtctg accgcattag ccctgcccat 540

aatccgttag acaagaacgc ggtgattact ctgggcgatc tgaatgcgga tgggaaacgt 600

cgcttaccaa atggccaact cgatcacggg acggattact accgtggtgg ttttgccgcg 660

catccgaacg attttccggt ttcgggctgg caacagacag tgacagatac ggatgttacg 720

aactttgcgc agaccttacg tgcgtatgat cgcatgatcc ttagcaatgc aacccacaac 780

tcgtacgatg agtatggcat tatcggcgga atcccaaacc cgttgcctta tccgggtggc 840

gtttatcctg ctgggaccct gttcaagaac gtacacactg gcggtggcaa taccggtaaa 900

gcactgagtg accatcgcct gatttggagc aaattgaatt aaaagctt 948

<210> 60
<211> 2966
<212> DNA
<213> Artificial

<220>
<223> Synthetic

<400> 60
atcagatcgg aagagcgtcg ttaagggaaa gagtgttccg tactcagaga gctattacaa 60

ttcactggcc gtcgttttac aacgtcgtga ctgggaaaac ccaggcgtta cccaacttaa 120

tcgccttgca gcacatcctc cgtttgcgag ttggcggaat agcgaagaag cgcgtacaga 180

tcgtccgtca cagcagttgc gctctctgta acgtaccgca gttcaaagtc tacacctaca 240

aacgcgaaag tcgctatcgc ctgtttgtgg atgtgcaaag cgacatcatc gatactcctg 300

gtcgtcgcac ggtgattccg ttggcttctg cacggttact gagcgacaaa gtttcccgtg 360

aactctatcc ggttgtccac attggggatg aaagctggcg tatgatgacc acggatatgg 420

cgtcagtacc agtgtcggta attggcgaag aggttgcgga tctgtcgcat cgcgagaatg 480

acatcaagaa cgccattaac ctgatgtttt ggggcattta attaagccag ccccgacacc 540

cgccaacacc cgctgacgcg ccctgacggg cttgtctgct cccggcatcc gcttacagac 600

aagctgtgac cgtctccggg agctgcatgt gtcagaggtt ttcaccgtca tcaccgaaac 660

gcgcgagacg aaagggcctc gtgatacgcc tatttttata ggttaatgtc atgataataa 720

tggtttctta gacgtcaggt ggcacttttc ggggaaatgt gcgcggaacc cctatttgtt 780

tatttttcta aatacattca aatatgtatc cgctcatgag acaataaccc tgataaatgc 840

ttcaataata ttgaaaaagg aagagtatga gtattcaaca tttccgtgtc gcccttattc 900

cctttttttgc ggcattttgc cttcctgttt ttgctcaccc agaaacgctg gtgaaagtaa 960

aagatgctga agatcagttg ggtgcacgag tgggttacat cgaactggat ctcaacagcg 1020

gtaagatcct tgagagtttt cgccccgaag aacgttttcc aatgatgagc acttttaaag 1080

ttctgctatg tggcgcggta ttatcccgta ttgacgccgg gcaagagcaa ctcggtcgcc 1140

gcatacacta ttctcagaat gacttggttg agtactcacc agtcacagaa aagcatctta 1200

cggatggcat gacagtaaga gaattatgca gtgctgccat aaccatgagt gataacactg 1260

cggccaactt acttctgaca acgatcggag gaccgaagga gctaaccgct tttttgcaca 1320

acatggggga tcatgtaact cgccttgatc gttgggaacc ggagctgaat gaagccatac 1380

caaacgacga gcgtgacacc acgatgcctg tagcaatggc aacaacgttg cgcaaactat 1440

taactggcga actacttact ctagcttccc ggcaacaatt aatagactgg atggaggcgg 1500

ataaagttgc aggaccactt ctgcgctcgg cccttccggc tggctggttt attgctgata 1560

```
aatctggagc cggtgagcgt gggtctcgcg gtatcattgc agcactgggg ccagatggta     1620
agccctcccg tatcgtagtt atctacacga cggggagtca ggcaactatg gatgaacgaa     1680
atagacagat cgctgagata ggtgcctcac tgattaagca ttggtaactg tcagaccaag     1740
tttactcata tatactttag attgatttaa aacttcattt ttaatttaaa aggatctagg     1800
tgaagatcct ttttgataat ctcatgacca aaatccctta acgtgagttt tcgttccact     1860
gagcgtcaga ccccgtagaa aagatcaaag gatcttcttg agatcctttt tttctgcgcg     1920
taatctgctg cttgcaaaca aaaaaaccac cgctaccagc ggtggtttgt ttgccggatc     1980
aagagctacc aactcttttt ccgaaggtaa ctggcttcag cagagcgcag ataccaaata     2040
ctgttcttct agtgtagccg tagttaggcc accacttcaa gaactctgta gcaccgccta     2100
catacctcgc tctgctaatc ctgttaccag tggctgctgc cagtggcgat aagtcgtgtc     2160
ttaccgggtt ggactcaaga cgatagttac cggataaggc gcagcggtcg ggctgaacgg     2220
ggggttcgtg cacacagccc agcttggagc gaacgaccta caccgaactg agatacctac     2280
agcgtgagct atgagaaagc gccacgcttc ccgaagggag aaaggcggac aggtatccgg     2340
taagcggcag ggtcggaaca ggagagcgca cgagggagct tccaggggga aacgcctggt     2400
atctttatag tcctgtcggg tttcgccacc tctgacttga gcgtcgattt ttgtgatgct     2460
cgtcaggggg gcggagccta tggaaaaacg ccagcaacgc ggccttttta cggttcctgg     2520
ccttttgctg gccttttgct cacatgttct ttcctgcgtt atcccctgat tctgtggata     2580
accgtattac cgcctttgag tgagctgata ccgctcgccg cagccgaacg accgagcgca     2640
gcgagtcagt gagcgaggaa gcggaagagc gcccaatacg caaaccgcct ctccccgcgc     2700
gttggccgat tcattaatgc agctggcacg acaggtttcc cgactggaaa gcgggcagtg     2760
agcgcaacgc aattaatgtg agttagctca ctcattaggc accccaggct ttacacttta     2820
tgcttccggc tcgtatgttg tgtggaattg tgagcggata acaatttcac acaggaaaca     2880
gctatgacca tgaccatgat tacgccaagc tatctgggcg ataccattga agactggagt     2940
tcagacgtgt gctcttccga tctgat                                         2966
```

**Description**

TECHNICAL FIELD

The present disclosure relates to a polypeptide having a function of delivering a substance into a cell, a polypeptide having a function of degrading a nucleic acid molecule, a polynucleotide encoding any of these polypeptides, etc.

BACKGROUND ART

Marine organisms are regarded as a great source of physiologically active substances, and many secondary metabolites that exhibits various physiological activities such as antiviral activity, antiproliferative activity, antioxidative activity, and anticoagulating activity have been found therefrom (Non-Patent Literature 1). In the meantime, it has been reported that sponges of the genus *Spongosorites* contain proteins having strong toxicity and having a function of translocating into the cell nucleus (Non-Patent Literatures 2 to 4).

CITATION LIST

Non-Patent Literature

Non-Patent Literature 1: Gogineni and Hamann, Biochim Biophys Acta Gen Subj. 2018;1862(1):81-196
Non-Patent Literature 2: Yuusuke Ida et al., "Mechanism of action of novel proteinous toxin derived from sponge of the genus Spongosorites from Iriomote Island", Abstracts for the annual meeting of the Japanese Society of Fisheries Science, 2018 Spring, 107
Non-Patent Literature 3: http://asp2017.org/wp-content/uploads/2016/12/ASP20201720Annual20Meeting_web.pdf, p. 28, S-12
Non-Patent Literature 4: http://dokuso-symposium.bio.tokushima-u.ac.jp/program/62th-abstract/62th-o.html, O-11

DISCLOSURE OF THE INVENTION

The exploitation of a useful physiologically active substance derived from a marine organism is expected.
Some aspects of the present disclosure provide the following.

[1] A polypeptide having a function of delivering a substance into a cell, the polypeptide being selected from

(a) a polypeptide comprising the amino acid sequence of SEQ ID NO: 1,
(b) a polypeptide that comprises an amino acid sequence containing a mutation of one or more amino acids in the amino acid sequence of SEQ ID NO: 1, and has the function of delivering a substance into a cell,
(c) a polypeptide that comprises an amino acid sequence having 80% or higher sequence identity to the amino acid sequence of SEQ ID NO: 1, and has the function of delivering a substance into a cell, and
(d) a polypeptide that comprises an amino acid sequence encoded by a polynucleotide hybridizing under highly stringent conditions to a polynucleotide encoding the amino acid sequence of SEQ ID NO: 1, and has the function of delivering a substance into a cell.

[2] The polypeptide according to [1], wherein the polypeptide has a function of delivering a substance into the nucleus.
[3] The polypeptide according to [1] or [2], wherein the cell is a mammalian cell.
[4] A complex of the polypeptide according to any one of [1] to [3] and a substance that functions intracellularly.
[5] The complex according to [4], wherein the polypeptide according to any one of [1] to [3] and the substance that functions intracellularly are bound through a covalent bond and/or a non-covalent bond.
[6] The complex according to [4] or [5], wherein the substance that functions intracellularly is selected from a toxin, an enzyme, a cell growth-inhibiting substance, an inhibitory nucleic acid molecule, a genome editing molecule, an antibody or an antigen binding fragment thereof, a signal transduction-regulating substance, a transcriptional factor, a gene and a label.
[7] A fusion polypeptide comprising, in a single molecule, the polypeptide according to any one of [1] to [3] and a polypeptide that functions intracellularly.
[8] A composition for use in delivering a substance into a cell, comprising the polypeptide according to any one of [1] to [3].

[9] A composition for use in treating a disease that is improved by the delivery of a substance that functions intracellularly into a cell, comprising the complex according to any one of [4 ]to [6] or the polypeptide according to [7].
[10] A method of delivering a substance that functions intracellularly into a cell, comprising the step of contacting a complex of the substance and the polypeptide according to any one of [1] to [3] with the cell.
[11] A polypeptide having a function of degrading a nucleic acid molecule, the polypeptide being selected from

(e) a polypeptide comprising the amino acid sequence of SEQ ID NO: 2,
(f) a polypeptide that comprises an amino acid sequence containing a mutation of one or more amino acids in the amino acid sequence of SEQ ID NO: 2, and has the function of degrading a nucleic acid molecule,
(g) a polypeptide that comprises an amino acid sequence having 80% or higher sequence identity to the amino acid sequence of SEQ ID NO: 2, and has the function of degrading a nucleic acid molecule, and
(h) a polypeptide that comprises an amino acid sequence encoded by a polynucleotide hybridizing under highly stringent conditions to a polynucleotide encoding the amino acid sequence of SEQ ID NO: 2, and has the function of degrading a nucleic acid molecule.

[12] A complex of the polypeptide according to [11] and a substance that promotes the translocation of the polypeptide into a cell.
[13] The complex according to [12], wherein the polypeptide according to [11] and the substance that promotes the translocation of the polypeptide into a cell are bound through a covalent bond and/or a non-covalent bond.
[14] The complex according to [12] or [13], wherein the substance that promotes the translocation of the polypeptide into a cell is selected from the polypeptide according to any one of [1] to [3], a cell-penetrating peptide, a cell binding domain of a proteinous toxin, a virus vector and a non-viral vector.
[15] A fusion polypeptide comprising, in a single molecule, the polypeptide according to [11] and a polypeptide that promotes the translocation of the polypeptide according to [11] into a cell.
[16] A composition for use in treating a disease that is improved by the degradation of a nucleic acid molecule, comprising the polypeptide according to [11] or [15] or the complex according to any one of [12] to [14].
[17] A method of degrading a nucleic acid molecule, comprising the step of allowing the polypeptide according to [11] or [15] or the complex according to any one of [12] to [14] to act on the nucleic acid molecule.
[18] A polypeptide having a function of degrading a nucleic acid molecule and a function of translocating into the nucleus, the polypeptide being selected from

(i) a polypeptide comprising the amino acid sequence of SEQ ID NO: 3,
(j) a polypeptide that comprises an amino acid sequence containing a mutation of one or more amino acids in the amino acid sequence of SEQ ID NO: 3, and has the function of degrading a nucleic acid molecule and the function of translocating into the nucleus,
(k) a polypeptide that comprises an amino acid sequence having 80% or higher sequence identity to the amino acid sequence of SEQ ID NO: 3, and has the function of degrading a nucleic acid molecule and the function of translocating into the nucleus, and
(l) a polypeptide that comprises an amino acid sequence encoded by a polynucleotide hybridizing under highly stringent conditions to a polynucleotide encoding the amino acid sequence of SEQ ID NO: 3, and has the function of degrading a nucleic acid molecule and the function of translocating into the nucleus.

[19] A complex of the polypeptide according to [18] and a targeting molecule.
[20] A composition for use in damaging a cell, comprising the polypeptide according to [18] or the complex according to [19].
[21] A method of damaging a cell, comprising the step of contacting the polypeptide according to [18] or the complex according to [19] with the cell.
[22] A polynucleotide encoding the polypeptide according to any one of [1] to [3], [7], [11], [15] and [18].
[23] A vector comprising the polynucleotide according to [22].
[24] A transformed cell comprising the polynucleotide according to [23].
[25] A method of producing the polypeptide according to any one of [1] to [3], [7], [11], [15] and [18], comprising the step of culturing the transformed cell according to [24] under conditions suitable for the expression of the polypeptide according to any one of [1] to [3], [7], [11], [15] and [18].

The intracellular delivery polypeptide according to the present disclosure provides a new option for transporting a substance from the outside of a cell into the cytoplasm and further into the nucleus. The nucleic acid-degrading polypeptide according to the present disclosure provides a new option for suppressing cell growth or treating a disease related to cell growth.

BRIEF DESCRIPTION OF DRAWINGS

Figure 1 is a graph in which the influence of a temperature on the toxicity of sponge extracts was evaluated. The ordinate depicts a survival rate (%), and the abscissa depicts incubation time (h). "Control" depicts untreated control, and "No heat" depicts crude extracts without heat treatment.

Figure 2 is a graph in which the toxicity of a captured fraction (Retentate) and a flow-through fraction (Filtrate) was evaluated when crude extracts were subjected to ultrafiltration. The ordinate depicts survival rate (%), and the abscissa depicts incubation time (h). "Control" depicts untreated control.

Figure 3 is a diagram showing difference in the color of extracts between the presence and absence of kojic acid added. 1 depicts extracts obtained with a buffer without kojic acid, and 2 depicts extracts obtained with a buffer supplemented with 0.5% w/v kojic acid.

Figure 4 is a diagram showing difference in the toxicity of extracts to brine shrimp between the presence and absence of kojic acid added.

Figure 5 is a graph in which the influence of pH on the toxicity of sponge extracts was evaluated. The ordinate depicts death rate (%).

Figure 6 is a chromatogram of sponge extracts treated by hydrophobic interaction chromatography. The green region depicts a fraction found to have toxicity.

Figure 7 is a chromatogram of sponge extracts treated by hydrophobic interaction chromatography and further treated by another hydrophobic interaction chromatography. The green region depicts a fraction found to have toxicity.

Figure 8 is a chromatogram of sponge extracts treated by two stages of hydrophobic interaction chromatography and further treated by ion-exchange chromatography. The green region depicts a fraction found to have toxicity.

Figure 9 is a chromatogram of sponge extracts treated by two stages of hydrophobic interaction chromatography and ion-exchange chromatography and further treated by gel filtration. The green region depicts a fraction found to have toxicity.

Figure 10 is a graph in which Kav of fraction A was plotted on a calibration curve prepared by plotting the molecular weight of each standard substance against Kav calculated by gel filtration chromatography. The filled circles depict each standard substance, and * depicts fraction A.

Figure 11 is an SDS-PAGE image of fraction A. Left lane depicts a molecular weight marker, and right lane depicts fraction A.

Figure 12 is an SDS-PAGE image of a sample at each purification stage. Lane M was loaded with a molecular weight marker, lane 1 was loaded with a redissolved solution, lane 2 was loaded with a sample after hydrophobic interaction chromatography treatment, lane 3 was loaded with a sample after ion-exchange chromatography treatment, and lane 4 was loaded with a toxic component finally isolated by HPLC.

Figure 13 is a diagram showing results of purification by HPLC. A depicts chromatogram of the first run of HPLC, B depicts SDS-PAGE image of a toxic fraction in the first run of HPLC, C depicts chromatogram of the second run of HPLC, and D depicts SDS-PAGE image of a toxic fraction in the second run of HPLC.

Figure 14 is a chart showing results of analysis of SOR by MALDI-TOF.

Figure 15 is a two-dimensional polyacrylamide gel electrophoresis image of SOR. Mr depicts molecular weight, and pI depicts isoelectric point.

Figure 16 is a graph in which the cytotoxicity of SOR was evaluated. The ordinate depicts relative cell growth rate with the cell growth of an untreated control defined as 100%, and the abscissa depicts concentration of SOR.

Figure 17 is a graph in which the toxicity of SOR to brine shrimp was evaluated. The ordinate depicts number of live individuals, and the abscissa depicts concentration of SOR. The filled circles depict number of live individuals after exposure for 24 hours, the filled squares depict number of live individuals after exposure for 36 hours, and the filled triangles depict number of live individuals after exposure for 48 hours.

Figure 18 is a diagram showing the influence of SOR on the development of *Aplysia kurodai* eggs. The arrow depicts a bubble-like projection on cell surface. The scale bar represents 100 $\mu$m.

Figure 19 is a diagram showing the state of a mouse 14 hours and 16 hours after SOR administration.

Figure 20 is an illustrative diagram summarizing a gene sequencing approach of SOR.

Figure 21 is a diagram showing the nucleotide sequence of a PCR product obtained by 1st strand PCR, and a predicted amino acid sequence thereof. An amino acid residue consistent with sequence information obtained by Edman degradation is indicated in red.

Figure 22 is a diagram showing the nucleotide sequence of a PCR product obtained by miss priming 3' RACE, and a predicted amino acid sequence thereof. An amino acid residue consistent with sequence information obtained by Edman degradation is indicated in red. The nucleic acid sequence of C-terminal 8 amino acid residues was complementary by accident with SOR4Fw2 primer.

Figure 23 is a diagram showing the nucleotide sequence of a PCR product obtained by 3'-RACE, and a predicted amino acid sequence thereof. Amino acid residues consistent with sequence information obtained by Edman degradation are indicated in red. Four clones were obtained in 3'-RACE, and one of them is shown. In the moiety surrounded by the cyan blanket (positions 2397 to 2511 of SEQ ID NO: 51) in 3' UTR, difference in the length and sequence of the poly-A moiety was found among the clones.

Figure 24 is a schematic diagram summarizing inverse PCR.

Figure 25 is a diagram showing the nucleotide sequence of a PCR product obtained by inverse PCR, and a predicted amino acid sequence thereof. Amino acid residues consistent with sequence information obtained by Edman degradation are indicated in red.

Figure 26 is a diagram showing the nucleotide sequence of a PCR product obtained by full length PCR, and a predicted amino acid sequence thereof. Amino acid residues consistent with sequence information obtained by Edman degradation are indicated in red, and bases and amino acid residues inconsistent with a nucleotide sequence obtained by 3'-RACE are indicated in blue.

Figure 27 is an SDS-PAGE image of liquid cell extracts and cell homogenates *of E. coli* transformed with a plasmid containing SOR. Lane M was loaded with a molecular weight marker, lane 1 was loaded with cell homogenates of control cells transformed with an empty vector, lane 2 was loaded with cell homogenates of cells harboring SOR, lane 3 was loaded with liquid cell extracts of control cells transformed with an empty vector, and lane 4 was loaded with liquid cell extracts of cells harboring SOR.

Figure 28 is an electrophoresis image in which the cleavage of DNA by SOR was evaluated. Lane 1 depicts molecular weight marker, lane 2 depicts plasmid alone, lane 3 depicts plasmid + SOR, and lane 4 depicts plasmid + SOR + EDTA.

Figure 29 is an electrophoresis image in which the cleavage of DNA by the N-terminal moiety of SOR was evaluated. Lane M depicts molecular weight marker, lane NC (negative control) depicts plasmid (pBSK+) alone, lane PC (positive control) depicts 2.5 μL of plasmid + bovine pancreatic DNaseI (1 mg/mL, Worthington Industries, Inc.), lane 1 depicts plasmid + recombinant SOR N-terminal moiety, and lane 2 depicts plasmid + recombinant SOR N-terminal moiety + EDTA.

Figure 30 is confocal laser microscope images taken after 18 μg/mL FITC-labeled SOR or FITC-labeled BSA was allowed to act on Ba/F3 cells. The left side shows fluorescence images, and the right side shows bright field images.

Figure 31 is confocal laser microscope images taken after 0.6 μg/mL FITC-labeled SOR was allowed to act on Ba/F3 cells. The left side shows fluorescence image, the center shows bright field image, and the right side shows merged image.

Figure 32 is a confocal laser microscope image taken 12 hours after administration of FITC-labeled BSA to HeLa cells.

Figure 33 is confocal laser microscope images taken 0.5 hours (left) or 1 hour (right) after administration of FITC-labeled SOR to HeLa cells.

Figure 34 is confocal laser microscope images taken 12 hours (left) or 16 hours (right) after administration of FITC-labeled SOR to HeLa cells.

DESCRIPTION OF EMBODIMENTS

All technical terms and scientific terms used herein have the same meanings as those usually understood by those skilled in the art, unless otherwise specified herein. All patents, applications and other publications (including online information) cited herein are incorporated herein by reference in their entirety. The present application claims the priority benefit of the Japanese application filed on April 7, 2020 (Japanese Application No. 2020-069328), the contents described in the specification and drawings of which are incorporated herein.

1. Intracellular delivery

In one aspect, the present disclosure provides a polypeptide having a function of delivering a substance into a cell, the polypeptide being selected from

(a) a polypeptide comprising the amino acid sequence of SEQ ID NO: 1,

(b) a polypeptide that comprises an amino acid sequence containing a mutation of one or more amino acids in the amino acid sequence of SEQ ID NO: 1, and has the function of delivering a substance into a cell,

(c) a polypeptide that comprises an amino acid sequence having 80% or higher sequence identity to the amino acid sequence of SEQ ID NO: 1, and has the function of delivering a substance into a cell, and

(d) a polypeptide that comprises an amino acid sequence encoded by a polynucleotide hybridizing under highly stringent conditions to a polynucleotide encoding the amino acid sequence of SEQ ID NO: 1, and has the function of delivering a substance into a cell

(hereinafter, also referred to as the "intracellular delivery polypeptide of the present disclosure").

In the polypeptide (b), the mutation of amino acid(s) includes deletion, a substitution and/or an addition of amino acid(s). The polypeptide (b) can contain a mutation of, for example, but is not limited to, 1 to 150, 1 to 145, 1 to 140, 1 to 135, 1 to 130, 1 to 125, 1 to 120, 1 to 115, 1 to 110, 1 to 105, 1 to 100, 1 to 95, 1 to 90, 1 to 85, 1 to 80, 1 to 75, 1 to 70, 1 to 65, 1 to 65, 1 to 63, 1 to 60, 1 to 58, 1 to 55, 1 to 53, 1 to 50, 1 to 48, 1 to 45, 1 to 43, 1 to 40, 1 to 38, 1 to 35, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9 (1 to several), 1 to 8, 1 to 7, 1 to 6, 1 to 5, 1 to 4, 1 to 3, 1 to 2, or 1 amino acid(s) in the amino acid sequence of SEQ ID NO: 1. In general, a fewer number of mutations is more preferred.

The polypeptide (c) can have 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity to the amino acid sequence of SEQ ID NO: 1. In general, higher sequence identity is more preferred.

In the present disclosure, the "polynucleotide hybridizing under highly stringent conditions" refers to, for example, a polynucleotide that is obtained by using colony hybridization, plaque hybridization or Southern hybridization, etc., with the whole or a portion of a polynucleotide consisting of a nucleotide sequence complementary to a reference nucleotide sequence as a probe. A method described in, for example, Sambrook and Russell, Molecular Cloning: A Laboratory Manual Vol. 4, Cold Spring Harbor, Laboratory Press 2012, Ausubel, Current Protocols in Molecular Biology, John Wiley & Sons 1987-1997 can be used as a hybridization method.

In the present disclosure, the "highly stringent conditions" are, for example, but are not limited to, conditions involving 5 × SSC, 5 × Denhardt's solution, 0.5% SDS, 50% formamide, and 50°C, or 0.2 × SSC, 0.1% SDS, and 60°C, or 0.2 × SSC, 0.1% SDS, and 62°C, or 0.2 × SSC, 0.1% SDS, and 65°C. Under these conditions, the elevation of the temperature can be expected to efficiently produce DNA having higher sequence identity. However, possible factors that influence the stringency of hybridization are a plurality of factors such as a temperature, a probe concentration, a probe length, ionic strength, a time, and a salt concentration. Those skilled in the art are capable of achieving similar stringency by appropriately selecting these factors.

In the case of using a commercially available kit in hybridization, for example, Alkphos Direct Labelling and Detection System (GE Healthcare) can be used. In this case, hybridized DNA can be detected after overnight incubation with a labeled probe followed by washing of membrane with a primary washing buffer containing 0.1% (w/v) SDS under a condition of 55 to 60°C according to a protocol attached to the kit. Alternatively, when a probe is prepared on the basis of the whole or a portion of a nucleotide sequence complementary to the nucleotide sequence of SEQ ID NO: 1, or a nucleotide sequence complementary to a nucleotide sequence encoding the amino acid sequence of SEQ ID NO: 3 or 4, the probe may be labeled with digoxigenin (DIG) using a commercially available reagent (e.g., PCR Labeling Mix (Roche Diagnostics GmbH)). In this case, hybridization can be detected using DIG Nucleic Acid Detection Kit (Roche Diagnostics GmbH).

Other examples of the polynucleotide capable of hybridizing can include a polynucleotide having 60% or higher, 61% or higher, 62% or higher, 63% or higher, 64% or higher, 65% or higher, 66% or higher, 67% or higher, 68% or higher, 69% or higher, 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity to the nucleotide sequence of SEQ ID NO: 1 by calculation with BLAST homology search software using default parameters.

The sequence identity of an amino acid sequence or a nucleotide sequence can be determined using algorithm BLAST by Karlin and Altschul (Basic Local Alignment Search Tool) (Proc Natl Acad Sci USA. 1990;87(6):2264-8,1990, Proc Natl Acad Sci USA. 1993;90(12):5873-7). In the case of using BLAST, default parameters of each program are used.

In the present disclosure, the "function of delivering a substance into a cell" means a function by which a polypeptide transfers the substance attached thereto into a cell. The delivery into a cell includes delivery from the outside of the cell into the cytoplasm, delivery from the inside of the cytoplasm into the nucleus, and delivery from the outside of the cell via the inside of the cytoplasm into the nucleus. Whether or not a polypeptide has the function of delivering a substance into a cell can be determined, for example, by binding a detectable label (e.g., a fluorescent label such as FITC) to the polypeptide, contacting the labeled polypeptide with the cell, and observing the localization of the label after a lapse of a predetermined time (e.g., 1 to 24 hours). In case the label is localized in the cell (in the cytoplasm or in the nucleus) after a lapse of a predetermined time, the polypeptide has the function of delivering a substance into a cell.

The cell into which the intracellular delivery polypeptide of the present disclosure delivers the substance is not particularly limited and includes prokaryotic or eukaryotic cells, for example, microbial (e.g., bacterial, fungal, and yeast) cells, animal cells, and plant cells. Examples of animal cells include mammalian, avian, reptilian, amphibian, and fish cells. Examples of the mammalian cells include human, nonhuman primate, rodent (e.g., mouse, rat, and guinea pig), dog, cat, bovine, horse, pig, goat, sheep, and rabbit cells.

The substance to be delivered by the intracellular delivery polypeptide of the present disclosure is not particularly limited and includes atoms, molecules, compounds, proteins, nucleic acids, and the like. In one embodiment, the substance to be delivered by the intracellular delivery polypeptide of the present disclosure is a substance that functions intracellularly. Examples of the substance that functions intracellularly include, but are not limited to, toxins, enzymes, enzyme inhibitors, receptor inhibitors, dominant negative mutants, cell growth-inhibiting substances, inhibitory nucleic acid molecules, genome editing molecules, transcriptional factors, antibodies and antigen binding fragments thereof, signal transduction-regulating substances, genes and labels.

The inhibitory nucleic acid molecule means a nucleic acid molecule that inhibits the expression of a target molecule and includes RNAi molecule, microRNA, microRNA mimic, piRNA (Piwi-interacting RNA), ribozyme (see, e.g., Jimenez et al., Trends Biochem Sci. 2015;40(11):648-661), antisense nucleic acids, and Bonac nucleic acids, for example.

The RNAi (RNA interference) molecule means any molecule having RNAi activity and encompasses, for example, but is not limited to, siRNA (small interfering RNA), shRNA (short hairpin RNA), and the like.

The siRNA refers to a small nucleic acid that has an antisense strand having complementarity with a target sequence and a sense strand having complementarity with the antisense strand, both the strands of which form a duplex at least partially.

The shRNA is a single-stranded RNA molecule containing an antisense region and a sense region having complementarity with each other, and a loop region interposed therebetween, and a duplex region is formed by the pairing of the antisense region and the sense region to assume a hairpin-like three-dimensional structure.

The microRNA mimic is a nucleic acid molecule that mimics the function of endogenous microRNA, and is well known in the art (e.g., van Rooij and Kauppinen, EMBO Mol Med. 2014;6(7):851-64, and Chorn et al., RNA. 2012;18(10): 1796-804).

The genome editing molecule is a molecule or a molecule set capable of modifying a genome sequence in a site-specific manner. Examples thereof include, but are not limited to, molecules based on CRISPR/Cas system, TALEN, ZFN, or meganuclease (MN). The genome editing molecule based on CRISPR/Cas system functions as a set of guide RNA and Cas (CRISPR-associated protein). Examples of Cas contained in CRISPR/Cas system include, but are not limited to, Cas9, Cas12a(Cpf1), Cas12b, Cas13, xCas9, VQR, VRER, spCas9-NG, spCas9-HF1, Cas nickase (e.g., Cas9 nickase), eSpCas9, evoCas9, HypaCas9, CjCas9, Split-Cas (e.g., Split-Cas9), dCas-BE (e.g., dCas9-BE), and the like (Broeders et al., iScience. 2019;23(1):100789). CRISPR/Cas13 can edit RNA and as such, can be used in gene silencing similar to that of RNAi.

The antibody is not particularly limited and includes IgA, IgD, IgE, IgG and IgM, including IgA1, IgA2, IgG1, IgG2a, IgG2b, IgG3 and IgG4 as subclasses. The antibody also includes IgNAR and heavy chain antibodies. The antigen binding fragment of the antibody includes antibody fragments containing the complementarity-determining regions (CDRs) of the antibody, or combinations thereof, and encompasses, for example, but is not limited to, Fab, Fab', $F(ab')_2$, Fd, Fcab, vNAR, VHH (nanobody), scFv, minibody, scFv-Fc, $scFv_2$ (diabody), $scFv_3$ (triabody), $scFv_4$ (tetrabody), Fv-clasp (Arimori et al., Structure. 2017;25(10):1611-1622), BIf (bispecific scFv immunofusion, Kuo et al., Protein Eng Des Sel. 2012;25(10):561-9), and the like.

Examples of the label include, but are not limited to, fluorescent labels, luminescent labels, radioactive labels, metal atoms detectable by MRI, etc., and compounds (e.g., complexes) containing a metal atom.

The intracellular delivery polypeptide of the present disclosure may form a complex with the substance to be delivered. Thus, the present disclosure provides a complex of the intracellular delivery polypeptide of the present disclosure and a substance to be delivered (hereinafter, also referred to as the "intracellular delivery polypeptide complex of the present disclosure"). Examples of binding mode between the intracellular delivery polypeptide of the present disclosure and the substance to be delivered in the intracellular delivery polypeptide complex of the present disclosure include, but are not particularly limited to, covalent bond and non-covalent bond (hydrogen bond, ionic bond, hydrophobic bond, disulfide bond, etc.). The intracellular delivery polypeptide of the present disclosure and the substance to be delivered may be bound directly or may be bound via an intervening element such as a linker. The linker may be degradable or nondegradable. When delivery into cytoplasm is desired, a linker that is cleaved by an intracytoplasmic enzyme or the like can be used. When delivery into the nucleus is desired, a linker that is cleaved in the nucleus without being cleaved in the cytoplasm can be used.

When the substance to be delivered is a polypeptide (e.g., a polypeptide that functions intracellularly), the intracellular delivery polypeptide complex of the present disclosure can be configured as a fusion polypeptide. Thus, the present disclosure provides a fusion polypeptide comprising the intracellular delivery polypeptide of the present disclosure and a polypeptide to be delivered in a single molecule (hereinafter, also referred to as the "intracellular delivery fusion polypeptide of the present disclosure"). The location of the polypeptide to be delivered in the fusion polypeptide is not particularly limited, and the polypeptide to be delivered is preferably located on the N-terminal side of the intracellular delivery polypeptide of the present disclosure. The fusion polypeptide can be configured such that a linker is interposed between the intracellular delivery polypeptide of the present disclosure and the polypeptide to be delivered. For the linker in the fusion polypeptide, see, for example, Chen et al., Adv Drug Deliv Rev. 2013; 65 (10): 1357-69. Hereinafter, the intracellular delivery polypeptide complex of the present disclosure encompasses the intracellular delivery fusion polypeptide of the present disclosure, unless otherwise specified.

The present disclosure also provides a composition comprising the intracellular delivery polypeptide and/or the intracellular delivery polypeptide complex of the present disclosure (hereinafter, also referred to as the "intracellular delivery polypeptide composition of the present disclosure"). This composition may comprise an additive such as a buffer, an excipient, a stabilizer, a tonicity agent, or a preservative, and a substance to be delivered, in addition to the intracellular delivery polypeptide of the present disclosure. The additive may be pharmaceutically acceptable, and the composition may be a pharmaceutical composition.

The intracellular delivery polypeptide, the intracellular delivery polypeptide complex and the intracellular delivery polypeptide composition of the present disclosure may be for delivering the substance into a cell (e.g., into the cytoplasm or into the nucleus).

The intracellular delivery polypeptide complex and the intracellular delivery polypeptide composition of the present disclosure comprising a substance that functions intracellularly may be for use in treating a disease that is improved by the delivery of the substance that functions intracellularly into a cell.

The disease that is improved by the delivery of the substance that functions intracellularly into a cell may differ depending on the substance that functions intracellularly and encompasses, for example, but is not limited to, cell proliferative disease such as cancer, infection, autoimmune disease, endocrine disease such as diabetes mellitus, neurological disease such as neurodegenerative disease, mental disease, fibrous disease such as fibrosis, cardiovascular disease, gastrointestinal disease, motor disorder, urological disease, and ophthalmologic disease.

The present disclosure also provides a method of delivering a substance into a cell, the method comprising the step of contacting the intracellular delivery polypeptide complex of the present disclosure with the cell (hereinafter, also referred to as the "intracellular delivery method of the present disclosure "). This method can be performed *in vitro, ex vivo* or *in vivo.* The contact of the intracellular delivery polypeptide complex of the present disclosure with the cell can be achieved *in vitro,* for example, by adding the intracellular delivery polypeptide complex of the present disclosure to a medium containing the target cell, and *in vivo,* for example, by administering the intracellular delivery polypeptide complex of the present disclosure to a living body containing the target cell.

The present disclosure also provides a method of treating a disease that is improved by the delivery of a substance that functions intracellularly into a cell, the method comprising the step of administering the intracellular delivery polypeptide complex or the intracellular delivery polypeptide composition of the present disclosure comprising the substance that functions intracellularly to a subject in need thereof (hereinafter, also referred to as the "treatment method A of the present disclosure").

In the present disclosure, the "treatment" encompasses every type of medically acceptable prophylactic and/or therapeutic intervention aimed at cure, transient remission or prevention, etc., of a disease. The "treatment" encompasses medically acceptable intervention for various purposes including, for example, the delay or arrest of progression of a disease, the regression or disappearance of a lesion, the prevention of onset of the disease or the prevention of recurrence of the disease.

In the present disclosure, the "subject" means any individual organism, preferably animal, more preferably mammalian, further preferably human individual. In another embodiment, the subject may be a nonhuman animal such as a companion animal or a farm animal, particularly, a dog, a cat, a horse, a donkey, a bovine, a goat, or a sheep. The subject may be healthy (e.g., having no particular disease or not having any disease) or may be affected by some disease. When the treatment, etc., of a disease is intended, the subject typically means a subject affected by the disease or having a risk of being affected by the disease.

In this method, the intracellular delivery polypeptide complex or the intracellular delivery polypeptide composition of the present disclosure can be administered in a therapeutically effective amount. In this context, the therapeutically effective amount is an amount that prevents the onset and recurrence of the target disease, or cures the target disease.

The specific dosage of the intracellular delivery polypeptide complex or the intracellular delivery polypeptide composition of the present disclosure to be administered may be determined in consideration of the type of the substance that functions intracellularly and various conditions as to the subject in need of the administration, for example, a therapeutic purpose, a therapeutic regimen, the type of disease, the severity of symptoms, the general state of health, age, and body weight of the subject, the sex of the subject, diets, the timing and frequency of administration, a concurrent drug, responsiveness to therapy, and compliance with therapy. The total daily dose of the intracellular delivery polypeptide complex or the intracellular delivery polypeptide composition of the present disclosure is not limited and may be, for example, approximately 1 μg/kg body weight to approximately 1000 mg/kg body weight, approximately 10 μg/kg body

weight to approximately 100 mg/kg body weight, or approximately 100 μg/kg body weight to approximately 10 mg/kg body weight, in terms of the amount of the intracellular delivery polypeptide. Alternatively, the dose may be calculated on the basis of the body surface area of a patient.

The administration in this method can be performed through any of various routes including both oral and parenteral routes, for example, but are not limited to, oral, intravenous, intramuscular, subcutaneous, local, intrapulmonary, intratracheal, endotracheal], intrabronchial, transnasal, transgastric, enteral, intrarectal, intraarterial, intraportal, intraventricular, intramedullary, intra-lymph node, intra-lymphatic, intracerebral, intrathecal, intracerebroventricular, transmucosal, transdermal, intranasal, intraperitoneal and intrauterine routes. The frequency of administration differs depending on the properties of the preparation or the composition used or the conditions of the subject as described above and may be, for example, a plurality of times per day (i.e., 2, 3, 4 or 5 or more times per day), once a day, every few days (i.e., every 2, 3, 4, 5, 6, or 7 days, etc.), several times a week (e.g., 2, 3, or 4 times a week), every week, or every few weeks (i.e., every 2, 3, or 4 weeks, etc.).

2. Nucleic acid-degrading polypeptide

In another aspect, the present disclosure provides a polypeptide having a function of degrading a nucleic acid molecule, the polypeptide being selected from

(e) a polypeptide comprising the amino acid sequence of SEQ ID NO: 2,
(f) a polypeptide that comprises an amino acid sequence containing a mutation of one or more amino acids in the amino acid sequence of SEQ ID NO: 2, and has the function of degrading a nucleic acid molecule,
(g) a polypeptide that comprises an amino acid sequence having 80% or higher sequence identity to the amino acid sequence of SEQ ID NO: 2, and has the function of degrading a nucleic acid molecule, and
(h) a polypeptide that comprises an amino acid sequence encoded by a polynucleotide hybridizing under highly stringent conditions to a polynucleotide encoding the amino acid sequence of SEQ ID NO: 2, and has the function of degrading a nucleic acid molecule

(hereinafter, also referred to as the "nucleic acid-degrading polypeptide of the present disclosure").

In the polypeptide (f), the mutation of amino acid(s) includes deletion, substitution and/or addition of the amino acid(s). The polypeptide (f) can contain a mutation of, for example, but is not limited to, 1 to 62, 1 to 61, 1 to 60, 1 to 59, 1 to 58, 1 to 57, 1 to 56, 1 to 55, 1 to 54, 1 to 53, 1 to 52, 1 to 51, 1 to 50, 1 to 49, 1 to 48, 1 to 47, 1 to 46, 1 to 45, 1 to 44, 1 to 43, 1 to 42, 1 to 41, 1 to 40, 1 to 39, 1 to 38, 1 to 37, 1 to 36, 1 to 35, 1 to 34, 1 to 33,1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9(1 to several), 1 to 8, 1 to 7, 1 to 6, 1 to 5, 1 to 4, 1 to 3, 1 to 2, or 1 amino acid(s) in the amino acid sequence of SEQ ID NO: 2. In general, a fewer number of mutations is more preferred.

The polypeptide (g) can have 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity to the amino acid sequence of SEQ ID NO: 2. In general, higher sequence identity is more preferred.

In the present disclosure, the "function of degrading a nucleic acid molecule" means a function by which a polypeptide cleaves the nucleic acid molecule, in the presence of a metal ion if necessary. The nucleic acid molecule encompasses DNA and RNA. Whether or not a polypeptide has the function of degrading a nucleic acid molecule can be determined, for example, by allowing the polypeptide to act on the nucleic acid molecule (e.g., plasmid DNA), if necessary, in the presence of a metal ion, and observing the size of the nucleic acid molecule after a lapse of a predetermined time (e.g., 1 to 12 hours) by, for example, agarose gel electrophoresis. In case the fragmentation of the nucleic acid molecule is found after a lapse of a predetermined time, the polypeptide has the function of degrading a nucleic acid molecule.

The nucleic acid-degrading polypeptide of the present disclosure may form a complex with a substance that promotes the translocation thereof into a cell (e.g., into the cytoplasm or into the nucleus). Thus, the present disclosure provides a complex of the nucleic acid-degrading polypeptide of the present disclosure and a substance that promotes the translocation thereof into a cell (hereinafter, also referred to as the "nucleic acid-degrading polypeptide complex of the present disclosure"). Examples of the binding mode between the nucleic acid-degrading polypeptide of the present disclosure and the substance that promotes the translocation into a cell in the nucleic acid-degrading polypeptide complex of the present disclosure include, but are not particularly limited to, covalent bond and non-covalent bond (hydrogen bond, ionic bond, hydrophobic bond, disulfide bond, etc.). The nucleic acid-degrading polypeptide of the present disclosure and the substance that promotes the translocation into a cell may be bound directly or may be bound via an intervening element such as a linker. The linker may be degradable or nondegradable. In the case of allowing the nucleic acid-degrading polypeptide of the present disclosure to act in the cytoplasm, a linker that is cleaved by an intracytoplasmic enzyme or the like can be used. In the case of allowing the nucleic acid-degrading polypeptide of the present disclosure to act in the nucleus, a linker that is cleaved in the nucleus without being cleaved in the cytoplasm can be used.

In the nucleic acid-degrading polypeptide complex of the present disclosure, the substance that promotes the translocation of the nucleic acid-degrading polypeptide of the present disclosure into a cell is not particularly limited as long as the substance is capable of promoting the translocation of a polypeptide comprising approximately 240 to approximately 380 amino acids into a cell. Examples thereof include the intracellular delivery polypeptide of the present disclosure, cell-penetrating peptides, cell binding domains of proteinous toxins, virus vectors and non-viral vectors.

Examples of cell binding domain of proteinous toxin include, but are not limited to, the B domain of AB toxin. The AB toxin is a toxin having two domains (subunits), A and B, and diphtheria toxin, *Pseudomonas* exotoxin A, botulinum neurotoxin, cholera toxin, anthrax toxin, Shiga toxin, pertussis toxin, *E. coli* heat-labile enterotoxin (LT toxin), ricin, and the like are known (Odumosu et al., Toxins (Basel). 2010;2(7):1612-45). The B domain of the AB toxin has a function of binding to a receptor on cell membrane surface and delivering the A domain having toxicity into the cell. In a particular embodiment, the receptor binding domain of the proteinous toxin is selected from diphtheria toxin B fragment, *Pseudomonas* exotoxin A domain Ia and optionally domain II (Michalska and Wolf, Front Microbiol. 2015;6:963), botulinum neurotoxin HC domain and optionally HN domain (Williams and Tsai, Curr Opin Cell Biol. 2016;41:51-6), cholera toxin B subunit, anthrax toxin B subunit, Shiga toxin B subunit, pertussis toxin B subunit, E. *coli* LT toxin B subunit, and ricin B subunit.

The cell-penetrating peptide is a peptide that can pass through a cell membrane and translocate into a cell, and many types are known. Non-limiting examples of the cell-penetrating peptide include TAT, polyarginine (R5, R8, R9, etc.), Penetratin, Pep-1, proline-rich peptide (Pro), TAT-HA2, Hph-1, HP4, LAH4, LAH4-L1, Vectofusin-1, low-molecular-weight protamine (LMWP), LL-37, Pep-7, Pept1, Pept2, IVV-14, Transportant, Ig(v), pVEC, HRSV, TGN, Derived Ku-70, RW(n), RRRRRRGGRRRRG, SVS-1, L-CPP, RLW, K16ApoE, Angiopep-2, ACPP, KAFAK, hCT(9-32), VP22, and the like (see, e.g., Vanova et al., Materials (Basel). 2019;12(17). pii: E2671, Silva et al., Biomolecules. 2019;9(1). pii: E22, and Derakhshankhah and Jafari, Biomed Pharmacother. 2018;108:1090-1096).

Examples of the virus vector include, but are not limited to, vectors based on adenovirus, adeno-associated virus (AAV), retrovirus, vaccinia virus, pox virus, lentivirus, herpes virus, and bacteriophages.

Examples of the non-viral vector include, but are not limited to, particulate carriers such as polymer particles, lipid particles, and inorganic particles, and bacterial vectors. Nanoparticles of nano-level size can be used as the particulate carrier. Examples of polymer particles include, but are not limited to, particles containing polymers such as cationic polymers, polyamidoamine (PAMAM), chitosan, glycol chitosan, cyclodextrin, poly(lactic-co-glycolic acid) (PLGA), poly(lactic-co-caprolactic acid) (PLCA), poly(β amino ester), and atelocollagen. Lipid particles include liposomal and non-liposomal lipid particles, for example. Liposome is a small vesicle having an internal cavity enclosed by a lipid bilayer membrane. Non-liposomal lipid particles are lipid particles having no such structure. Examples of inorganic particles include gold nanoparticles, quantum dots, silica nanoparticles, iron oxide nanoparticles (e.g., superparamagnetic iron oxide nanoparticles (SPIONs)), nanotubes (e.g., carbon nanotubes (CNTs)), nanodiamonds, and fullerene. Examples of bacterial vector include, but are not limited to, vectors based on listeria, bifidus, and salmonella. The carrier particles can be variously modified. Examples of such modification include stealthing with PEG, targeting with a targeting molecule, and modification with a cell-penetrating peptide (CPP).

When the substance that promotes the translocation into a cell is a polypeptide, the nucleic acid-degrading polypeptide complex of the present disclosure can be configured as a fusion polypeptide. Thus, the present disclosure provides a fusion polypeptide comprising the nucleic acid-degrading polypeptide of the present disclosure and a polypeptide that promotes the translocation into a cell in a single molecule (hereinafter, also referred to as the "nucleic acid-degrading fusion polypeptide of the present disclosure"). The location of the polypeptide that promotes the translocation into a cell in the fusion polypeptide is not particularly limited, and the polypeptide is preferably located on the C-terminal side of the nucleic acid-degrading polypeptide of the present disclosure. The fusion protein can be configured such that a linker is interposed between the nucleic acid-degrading polypeptide of the present disclosure and the polypeptide that promotes the translocation into a cell. For the linker in the fusion polypeptide, see, for example, Chen et al. (supra). Hereinafter, the nucleic acid-degrading polypeptide complex of the present disclosure encompasses the nucleic acid-degrading polypeptide of the present disclosure, unless otherwise specified.

The present disclosure also provides a composition comprising the nucleic acid-degrading polypeptide of the present disclosure and/or the nucleic acid-degrading polypeptide complex of the present disclosure (hereinafter, also referred to as the "nucleic acid-degrading polypeptide composition of the present disclosure"). This composition may comprise an additive such as a buffer, an excipient, a stabilizer, a tonicity agent, or a preservative; a metal ion, a substance that promotes the translocation into a cell, and others, in addition to the nucleic acid-degrading polypeptide of the present disclosure and/or the nucleic acid-degrading polypeptide complex of the present disclosure. The additive may be pharmaceutically acceptable, and the composition may be a pharmaceutical composition.

The nucleic acid-degrading polypeptide of the present disclosure and/or the nucleic acid-degrading polypeptide complex of the present disclosure may be targeted to a cell or a tissue containing the nucleic acid molecule to be degraded. The targeting can be achieved by, for example, passive targeting or active targeting. Passive targeting can be achieved by, for example, but is not limited to, enclosure in a carrier having an EPR effect (e.g., carrier particles such as a liposome, and polymer micelle). The active targeting can be achieved by, for example, but is not limited to, the addition of a targeting molecule (e.g., a cell binding domain of an antibody or a proteinous toxin) that can interact with a cell surface molecule of interest, or enclosure in a carrier with this targeting molecule added thereto.

The nucleic acid-degrading polypeptide, the nucleic acid-degrading polypeptide complex and the nucleic acid-degrading polypeptide composition of the present disclosure may be for use in treating a disease that is improved by the degradation of a nucleic acid molecule. Examples of the disease that is improved by the degradation of a nucleic acid molecule include diseases that are improved by the damage or killing of causative cells. Such a disease encompasses, for example, but is not limited to, cell proliferative disease such as cancer, infection, autoimmune disease, endocrine hyperfunction such as hyperthyroidism, fibrous disease such as fibrosis, and hyperplasia.

The present disclosure also provides a method of degrading a nucleic acid molecule, the method comprising the step of allowing the nucleic acid-degrading polypeptide, the nucleic acid-degrading polypeptide complex and/or the nucleic acid-degrading polypeptide composition of the present disclosure to act on the nucleic acid molecule (hereinafter, also referred to as the "nucleic acid degradation method A of the present disclosure"). This method can be performed *in vitro, ex vivo* or *in vivo.* This method can be performed *in vitro,* for example, by adding the nucleic acid-degrading polypeptide, etc., of the present disclosure to a medium containing the target nucleic acid molecule or a cell, etc., containing this molecule, and *in vivo,* for example, by administering the nucleic acid-degrading polypeptide, etc., of the present disclosure to a living body containing the target nucleic acid molecule. When the target nucleic acid molecule is present in a cell, it is preferred to use the nucleic acid-degrading polypeptide of the present disclosure together with a substance that promotes the translocation into a cell, or to use the nucleic acid-degrading polypeptide complex of the present disclosure. A metal ion can coexist when allowing the nucleic acid-degrading polypeptide, etc., of the present disclosure to act on the nucleic acid molecule. Examples of the metal ion include, but are not limited to, calcium ions, magnesium ions, sodium ions, and zinc ions. A metal ion that is chelated by EDTA is preferred.

The present disclosure also provides a method of treating a disease that is improved by the degradation of a nucleic acid molecule, the method comprising the step of administering the nucleic acid-degrading polypeptide of the present disclosure, the nucleic acid-degrading polypeptide complex of the present disclosure and/or the nucleic acid-degrading polypeptide composition of the present disclosure to a subject in need thereof (hereinafter, also referred to as the "treatment method B of the present disclosure"). The administration in this method is in the same manner as in the treatment method A of the present disclosure. The disease that is improved by the degradation of a nucleic acid molecule is as mentioned above.

3. Nuclear translocating and nucleic acid-degrading polypeptide

In still another aspect, the present disclosure provides a polypeptide having a function of degrading a nucleic acid molecule and a function of translocating into the nucleus, the polypeptide being selected from

(i) a polypeptide comprising the amino acid sequence of SEQ ID NO: 3,
(j) a polypeptide that comprises an amino acid sequence containing a mutation of one or more amino acids in the amino acid sequence of SEQ ID NO: 3, and has the function of degrading a nucleic acid molecule and the function of translocating into the nucleus,
(k) a polypeptide that comprises an amino acid sequence having 80% or higher sequence identity to the amino acid sequence of SEQ ID NO: 3, and has the function of degrading a nucleic acid molecule and the function of translocating into the nucleus, and
(l) a polypeptide that comprises an amino acid sequence encoded by a polynucleotide hybridizing under highly stringent conditions to a polynucleotide encoding the amino acid sequence of SEQ ID NO: 3, and has the function of degrading a nucleic acid molecule and the function of translocating into the nucleus (hereinafter, also referred to as the "nuclear translocating and nucleic acid-degrading polypeptide of the present disclosure").

In the polypeptide (j), the mutation of amino acid(s) includes deletion, substitution and/or addition of the amino acid(s). The polypeptide (j) can contain a mutation of, for example, but is not limited to, 1 to 190, 1 to 185, 1 to 180, 1 to 175, 1 to 170, 1 to 165, 1 to 160, 1 to 155, 1 to 150, 1 to 145,1 to 140, 1 to 135, 1 to 130, 1 to 125, 1 to 120, 1 to 115, 1 to 110, 1 to 105, 1 to 100, 1 to 95, 1 to 90, 1 to 85, 1 to 80, 1 to 75, 1 to 70, 1 to 65, 1 to 60, 1 to 55, 1 to 50, 1 to 45, 1 to 40, 1 to 35, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, 1 to 12, 1 to 11, 1 to 10, 1 to 9 (1 to several), 1 to 8, 1 to 7, 1 to 6, 1 to 5, 1 to 4, 1 to 3, 1 to 2, or 1 amino acid(s) in the amino acid sequence of SEQ ID NO: 3. In general, a fewer number of mutations is more preferred.

The polypeptide (k) can have 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher sequence identity to the amino acid sequence of SEQ ID NO: 3. In general, higher sequence identity is more preferred.

The nuclear translocating and nucleic acid-degrading polypeptide of the present disclosure may be targeted to a cell containing a nucleic acid molecule to be degraded. In some embodiments, the targeting can be achieved by complexing the nuclear translocating and nucleic acid-degrading polypeptide of the present disclosure with a targeting molecule. Thus, the present disclosure provides a complex of the nucleic acid-degrading polypeptide of the present disclosure and a targeting molecule (hereinafter, also referred to as the "nuclear translocating and nucleic acid-degrading polypeptide complex of the present disclosure"). Examples of the binding mode between the nuclear translocating and nucleic acid-degrading polypeptide of the present disclosure and the targeting molecule in the nuclear translocating and nucleic acid-degrading polypeptide complex of the present disclosure include, but are not particularly limited to, covalent bond and non-covalent bond (hydrogen bond, ionic bond, hydrophobic bond, disulfide bond, etc.). The nuclear translocating and nucleic acid-degrading polypeptide of the present disclosure and the targeting molecule may be bound directly or may be bound via an intervening element such as a linker. The linker may be degradable or nondegradable. In one embodiment, the linker has a property of being cleaved in the cytoplasm.

In another embodiment, the targeting can be achieved by enclosing the nuclear translocating and nucleic acid-degrading polypeptide of the present disclosure in a carrier (e.g., a particulate carrier) targeted to a cell of interest. The particulate carrier is as mentioned in the section about the nucleic acid-degrading polypeptide. The targeting of the particulate carrier, particularly, to cancer cells is described in Yao et al., J Control Release. 2016 Oct 28;240:267-286, Yoo et al., Cancers (Basel). 2019;11(5). pii: E640, and the like.

Any known targeting molecule can be used. Non-limiting examples of the targeting molecule to cancer include transferrin, RGD peptide, APRPG peptide, NGR peptide, F3 peptide, CGKRK peptide, folic acid, lactoferrin, glycosaminoglycan, tumor-specific antibodies (e.g., HER2 and PSMA), antigen binding fragments thereof (e.g., Fab, Fab', $F(ab')_2$, Fd, Fcab, vNAR, VHH (nanobody), scFv, minibody, scFv-Fc, $scFv_2$ (diabody), $scFv_3$ (triabody), $scFv_4$ (tetrabody), Fv-clasp (Arimori et al., Structure. 2017;25(10):1611-1622), etc.), mimics thereof (e.g., monobody (adnectin), affibody, Affimer, affitin, Anticalin, Atrimer, fynomer, Armadillo repeat protein, Kunitz domain, knottin, avimer, DARPin, alphabody, O body, repebody, and the like (Simeon and Chen, Protein Cell. 2018;9(1):3-14, Yu et al., Annu Rev Anal Chem (Palo Alto Calif). 2017;10(1):293-320, Wuo and Arora, Curr Opin Chem Biol. 2018 Jun;44:16-22). The targeting molecule is described in Yao et al. (supra) and Yoo et al. (supra) as well as in Jahan et al., J Drug Deliv. 2017;2017:9090325, Leng et al., J Drug Deliv. 2017;2017:6971297, and the like.

The present disclosure also provides a composition comprising the nuclear translocating and nucleic acid-degrading polypeptide of the present disclosure and/or the nuclear translocating and nucleic acid-degrading polypeptide complex of the present disclosure (hereinafter, also referred to as the "nuclear translocating and nucleic acid-degrading polypeptide composition of the present disclosure"). This composition may comprise an additive such as a buffer, an excipient, a stabilizer, a tonicity agent, or a preservative; a metal ion, a targeting molecule, a carrier such as a particulate carrier, and others, in addition to the nuclear translocating and nucleic acid-degrading polypeptide of the present disclosure and/or the nuclear translocating and nucleic acid-degrading polypeptide complex of the present disclosure. The additive may be pharmaceutically acceptable, and the composition may be a pharmaceutical composition.

The nuclear translocating and nucleic acid-degrading polypeptide, the nuclear translocating and nucleic acid-degrading polypeptide complex and the nuclear translocating and nucleic acid-degrading polypeptide composition of the present disclosure may be for use in damaging a cell and/or for use in treating a disease that is improved by the damage of a cell. Examples of the cell to be damaged include, but are not limited to, cells causative of diseases. Non-limiting examples of such a cell encompass cancer cells, vascular endothelial cells, cancer-associated fibroblasts (CAFs), cells of pathogens such as bacteria and parasites, immunocytes, endocrine cells, and myofibroblasts. Examples of the disease that is improved by the damage of a cell include, but are not limited to, cell proliferative disease such as cancer, infection, autoimmune disease, endocrine hyperfunction such as hyperthyroidism, fibrous disease such as fibrosis, and hyperplasia.

The present disclosure also provides a method of degrading a nucleic acid molecule, the method comprising the step of allowing the nuclear translocating and nucleic acid-degrading polypeptide, the nuclear translocating and nucleic acid-degrading polypeptide complex and/or the nuclear translocating and nucleic acid-degrading polypeptide composition of the present disclosure to act on the nucleic acid molecule (hereinafter, also referred to as the "nucleic acid degradation method B of the present disclosure". This method can be performed *in vitro, ex vivo* or *in vivo*. This method can be performed *in vitro,* for example, by adding the nuclear translocating and nucleic acid-degrading polypeptide, etc., of the present disclosure to a medium containing the target nucleic acid molecule or a cell, etc., containing this molecule, and *in vivo,* for example, by administering the nuclear translocating and nucleic acid-degrading polypeptide, etc., of the present disclosure to a living body containing the target nucleic acid molecule. A metal ion can coexist when allowing the nuclear translocating and nucleic acid-degrading polypeptide, etc., of the present disclosure to act on the nucleic acid molecule. The type of the metal ion is as described for the nucleic acid degradation method A of the present disclosure.

The present disclosure also provides a method of damaging a cell, the method comprising the step of contacting the nuclear translocating and nucleic acid-degrading polypeptide, the nuclear translocating and nucleic acid-degrading polypeptide complex and/or the nuclear translocating and nucleic acid-degrading polypeptide composition of the present disclosure with the cell (hereinafter, also referred to as the "cell damaging method of the present disclosure"). This method can be performed *in vitro, ex vivo* or *in vivo.* The contact of the nuclear translocating and nucleic acid-degrading polypeptide, etc., of the present disclosure with the cell can be achieved *in vitro,* for example, by adding the nuclear translocating and nucleic acid-degrading polypeptide, etc., of the present disclosure to a medium containing the target cell, and *in vivo,* for example, by administering the nuclear translocating and nucleic acid-degrading polypeptide, etc., of the present disclosure to a living body containing the target cell.

The present disclosure also provides a method of treating a disease that is improved by the damage of a cell, the method comprising the step of administering the nuclear translocating and nucleic acid-degrading polypeptide, the nuclear translocating and nucleic acid-degrading polypeptide complex and the nuclear translocating and nucleic acid-degrading polypeptide composition of the present disclosure to a subject in need thereof (hereinafter, also referred to as the "treatment method C of the present disclosure"). The administration in this method is in the same manner as in the treatment method A of the present disclosure. The disease that is improved by the damage of a cell is as mentioned above.

4. Polynucleotide, vector and host cell

In yet another aspect, the present disclosure provides a polynucleotide encoding any of various polypeptides described above (i.e., each of the polypeptides (a) to (1)) (hereinafter, also referred to as the "polynucleotide of the present disclosure"). The polypeptides (a) to (1) are also collectively referred to as the "polypeptide of the present disclosure".

The polynucleotide encompasses DNA and RNA. The DNA encompasses genome DNA, genome DNA libraries, cDNA, cDNA libraries, synthetic DNA, and the like. The polynucleotide of the present disclosure may be isolated. The nucleotide sequence of the polynucleotide of the present disclosure can be determined from the amino acid sequence of the polypeptide encoded thereby. Examples of the nucleotide sequence of the polynucleotide encoding the polypeptide (a) include a nucleotide sequence comprising SEQ ID NO: 4 or 5. Examples of the nucleotide sequence of the polynucleotide encoding the polypeptide (e) include a nucleotide sequence comprising SEQ ID NO: 6 or 7. Examples of the nucleotide sequence of the polynucleotide encoding the polypeptide (i) include a nucleotide sequence comprising SEQ ID NO: 8 or 9.

The polynucleotide of the present disclosure can be expressed by the transfection of a host cell. In this respect, the polynucleotide of the present disclosure can be codon-optimized for use in order to attain efficient expression in the host cell to be transfected. An approach of optimizing codons for host (e.g., E. *coli*) cells is known. Examples of the codon-optimized sequences of the polynucleotides encoding the polypeptides (a), (e) and (i) include sequences comprising SEQ ID NOs: 5, 7 and 9, respectively.

The polynucleotide of the present disclosure can be obtained by artificial synthesis or by, for example, PCR using a primer set specific for the polynucleotide of the present disclosure from the gene of a sponge of the genus *Spongosorites* (particularly, those living at the coast of Iriomote Island). Examples of the sequences of the polynucleotides encoding the polypeptides (a), (e) and (i) contained in the sponge of the genus *Spongosorites* include sequences comprising SEQ ID NOs: 4, 6 and 8, respectively.

The present disclosure also provides a vector comprising the polynucleotide of the present disclosure (hereinafter, also referred to as the "vector of the present disclosure"). Examples of the vector include library vectors, cloning vectors, and expression vectors. The expression vector is not particularly limited as long as the vector permits expression of the polynucleotide of the present disclosure in a host. Examples thereof include virus vectors and non-viral vectors. Non-limiting examples of the virus vector are as mentioned above for the nucleic acid-degrading polypeptide of the present disclosure. Non-limiting examples of the non-viral vector include those mentioned above for the nucleic acid-degrading polypeptide of the present disclosure as well as plasmid vectors, cosmid vectors, fosmid vectors, and artificial chromosome vectors such as YAC, BAC, and PAC.

In the expression vector of the present disclosure, the polynucleotide of the present disclosure may be operably linked to a promoter. Examples of the promoter include, but are not limited to, promoters for bacteria such as trc promoter, tac promoter, and lac promoter, promoters for yeasts such as GAL1 promoter, GAL10 promoter, glyceraldehyde-3-phosphate dehydrogenase promoter, and PH05 promoter, and promoters for animal cells such as SV40 early promoter and SV40 late promoter.

The expression vector of the present disclosure may comprise, if desired, an enhancer, a splicing signal, a poly-A addition signal, a ribosomal binding sequence (SD sequence), a selective marker, a replication origin, and the like. Examples of the selective marker include dihydrofolate reductase gene, LacZ gene, and antibiotic resistance genes (e.g., ampicillin, neomycin, kanamycin, streptomycin, tetracycline or chloramphenicol resistance gene).

The present disclosure also provides a transformed cell comprising the polynucleotide of the present disclosure (hereinafter, also referred to as the "transformed cell of the present disclosure"). The transformed cell of the present disclosure can be obtained by transfecting a host cell with the polynucleotide of the present disclosure. The host cell is preferably a cell that can express the polynucleotide of the present disclosure. Examples thereof include, but are not limited to, cells of microbes such as bacteria (*E. coli,* etc.), actinomycetes, and yeasts, insect cells (SF9, etc.), and mammalian cells (e.g., human HEK293 embryonic kidney-derived cells, monkey COS cells, Chinese hamster ovary (CHO) cells, and myeloma cells). The transfection of the host cell with the polynucleotide of the present disclosure can be performed by any of various known methods, for example, a calcium phosphate method, lipofection, or electroporation. In the transformed cell of the present disclosure, the polynucleotide of the present disclosure may be contained in a vector or may be integrated in the genome of the transformed cell.

5. Method of producing polypeptide

The polypeptide of the present disclosure may be chemically synthesized by any of various synthesis methods such as a solid-phase peptide synthesis method, and typically, can be produced by allowing the transformed cell of the present disclosure to express the polynucleotide of the present disclosure. Thus, the present disclosure provides a method of producing the polypeptide of the present disclosure, the method comprising the step of culturing the transformed cell of the present disclosure under conditions suitable for the expression of the polypeptide of the present disclosure (hereinafter, also referred to as the "production method of the present disclosure"). The conditions suitable for the expression of the polypeptide of the present disclosure are typically the same as those suitable for the culture of the transformed cell and/or the parent host cell serving as a base. Non-limiting examples of the conditions suitable for the expression of the polypeptide of the present disclosure include culture at 37°C under 5% $CO_2$ in a culture medium. The production method of the present disclosure may optionally comprise the step of extracting the polypeptide of the present disclosure, the step of purifying the polypeptide of the present disclosure, and other steps. The extraction and purification of the polypeptide can employ any known approach such as salting out, dialysis, or chromatography (e.g., affinity column chromatography and size exclusion chromatography). The polypeptide of the present disclosure can be produced in a form with an affinity tag such as His tag, HA tag, myc tag, or FLAG tag added thereto in order to enhance purification efficiency.

6. Method of isolating polypeptide

The present disclosure also provides a method of isolating the polypeptide of the present disclosure from a sponge of the genus *Spongosorites* (hereinafter, also referred to as the "isolation method of the present disclosure").

The isolation method of the present disclosure comprises the step of obtaining aqueous extracts of the sponge of the genus *Spongosorites.* The step of obtaining aqueous extracts comprises subjecting a tissue of the sponge of the genus *Spongosorites* to extraction with an aqueous medium one to ten times, for example, 1, 2, 3, 4 or 5 times. The extraction temperature is not particularly limited as long as the temperature does not denature proteins. The temperature may be 1 to 50°C, 4 to 40°C, 10 to 30°C, 15 to 25°C or room temperature, etc. The aqueous medium is not particularly limited as long as the aqueous extraction of proteins can be performed. Examples thereof include water, brine, and aqueous buffers (e.g., a Tris-HCl buffer). In a particular embodiment, the aqueous medium contains a tyrosinase inhibitor. Examples of the tyrosinase inhibitor include, but are not limited to, kojic acid, hydroquinone and its derivatives, deoxyarbutin and its derivatives, resorcin, resorcinol, and vanillin. Other examples of the tyrosinase inhibitor are described, for example, in Zolghadri et al., J Enzyme Inhib Med Chem. 2019;34(1):279-309, etc.

As the sponge of the genus *Spongosorites,* those living preferably on the coastal ocean bed of Iriomote Island, more preferably on the ocean bed of 2 to 10 m below the coastal sea surface of Iriomote Island, particularly, the ocean bed of 3 to 8 m below the sea surface around a northern latitude of 24.35 degrees and an east longitude of 123.72 degrees, can be used. The sponge may be cryopreserved before extraction with an aqueous medium.

The isolation method of the present disclosure may optionally comprise the step of salting out the aqueous extracts, the step of fractionating the extracts by chromatography, the step of desalting the chromatography fractions, the step of evaluating the toxicity of the chromatography fractions, the step of fractionating a fraction having toxicity by chromatography, and other steps. Examples of the chromatography include, but are not limited to, hydrophobic interaction chromatography, ion-exchange chromatography, and gel filtration chromatography. In the case of performing chromatography a plurality of times, different types of chromatography methods may be combined.

Examples of the approach of evaluating toxicity include, but are not limited to, the evaluation of toxicity to brine shrimp (counting of the number of live individuals) and the evaluation of toxicity to a cell system (measurement of a survival rate), as described in Examples.

In a particular embodiment, the isolation method of the present disclosure comprises the steps of:

(i) subjecting the sponge of the genus *Spongosorites* to extraction with an aqueous medium containing a tyrosinase inhibitor;
(ii) salting out the aqueous extracts obtained in the step (i);
(iii) fractionating the salted-out product obtained in the step (ii) by chromatography;
(iv) evaluating the toxicity of the chromatography fractions obtained in the step (iii); and
(v) fractionating a fraction evaluated to have toxicity in the step (iv) by chromatography.

The steps (iv) to (v) may be repeated a plurality of times until a clear peak is obtained.

## Examples

Hereinafter, the present invention will be described in more detail with reference to Examples. However, the contents of the present invention are not limited by these examples.

### [Example 1] Toxicity evaluation of sponge extract

Aqueous extracts of a sponge of the genus *Spongosorites* from Iriomote Island and gel filtration fractions thereof were evaluated for their toxicity using larvae of brine shrimp and a cancer cell system.

<Preparation of sample>

A sponge sample was collected from the ocean bed of 6 m below the coastal sea surface (northern latitude: 24.35 degrees, east longitude: 123.72 degrees) of Iriomote Island and cryopreserved until use in experiments. 10 g of the sample was subjected to extraction with 10 mL of water three times at room temperature to obtain crude extracts. A portion of the crude extracts was freeze-dried and subjected to gel filtration chromatography with Sephadex LH-20 open column (2.5 × 120 cm, GE Healthcare) (eluent: water, flow rate: 0.5 mL/min). The obtained fractions (10 mL/fraction) were combined by TLC (thin-layer chromatography, butanol:acetic acid:water = 4:1:1) to obtain a total of 38 fractions.

<Evaluation using brine shrimp>

Dry eggs (commercially available product) of brine shrimp (*Artemia salina)* were incubated at 25°C for 24 hours in artificial seawater (ASW). Hatched larvae were placed at a density of 10 individuals/well in a 24-well plate containing 2 mL/well of brine. 20 μL of the crude extracts or each fraction diluted, if necessary, was added to each well, and the plate was kept at 25°C. The behavior of the larvae was observed every 12 hours up to 48 hours, and the number of live individuals was counted. An individual that was totally motionless during the observation was regarded as being dead. The exertion of toxicity by the crude extracts occurred late, and the toxicity was found later than 24 hours from crude extract addition. Also, toxicity was found in the second to ninth fractions close to a void volume among the chromatography fractions. The sixth fraction and the crude extracts killed all of 10 individuals of brine shrimp used in 36 hours at a final concentration of 100 μg/mL.

<Evaluation using cancer cell system>

HT29 cells (human colorectal adenocarcinoma), A549 cells (human alveolar basal epithelial adenocarcinoma) or MDA-MB 231 cells (human breast adenocarcinoma) were inoculated at a density of 5 × $10^3$ cells/well to a 96-well plate containing a medium and incubated for 18 hours in a $CO_2$ incubator (37°C, 5% $CO_2$). The crude extracts or the sixth fraction was dissolved in a solvent of DMSO:$H_2O$ (3:7), and added in an amount of 5 μL to each well, followed by further incubation for 72 hours. After the completion of incubation, the cells were fixed by the addition of trichloroacetic acid (50%) to each well and evaluated for cytotoxicity on the basis of an OD490 value of sulforhodamine B staining. Specifically, cytotoxicity A was determined according to the following formula.

$$A = 100 \times (T - T_0) / (C - T)$$

wherein $T_0$ represents an OD value before incubation, T represents an OD value at the completion of incubation, and C represents an OD value of a control well (medium alone). A positive value of A means a cell growth inhibitory effect (the number of cells increased from that before incubation), and a negative value of A means a cytotoxic effect (the number of cells decreased from that before incubation). The results are shown in the table below. The number within the parentheses represents the concentration (μg/mL) of the extracts or the fraction.

Table 1 Cytotoxicity of sponge extract and gel filtration fraction thereof

| | HT-29 | A549 | MDA-MB-231 |
|---|---|---|---|
| Crude extract | 20 (5) | -26 (5) | -1 (5) |
| Sixth fraction | 27 (25) | -1 (5) | -3 (5) |

<Influence of temperature on activity>

The crude extracts were heated at 40°C, 60°C or 80°C for 10 minutes, and toxicity to brine shrimp was evaluated in the same manner as above using these samples. As is evident from the results shown in Figure 1, toxicity to brine shrimp disappeared by heat treatment at 80°C.

<Size of active component>

The crude extracts were subjected to ultrafiltration with NMWL of 30 KDa, and a captured fraction (Retentate) and a flow-through fraction (Filtrate) were evaluated for their toxicity to brine shrimp in the same manner as above. As is evident from the results shown in Figure 2, the size of an active component was more than 30 KDa.

<Influence of pH on activity>

890 mg of the sponge sample was subjected to extraction with a Tris-HCl buffer (100 mM, pH 8.0) containing 200 mM NaCl and a 0.5% w/v tyrosinase inhibitor (kojic acid). The tyrosinase inhibitor was added to the extraction medium in order to suppress the production of melanin in the extracts. A tyrosinase inhibitor other than kojic acid is also substitutable. Since black discoloration was suppressed in the extracts supplemented with kojic acid (Figure 3), the life of a column can be extended. In addition, the activity (e.g., toxicity to brine shrimp shown in Figure 4) of the extracts also exhibited a slightly strong tendency.

HiTrap Desalting column (5 mL, GE Healthcare) was loaded with the extracts, followed by elution with 0.6 M NaCl in AKTA FPLC system (GE Healthcare) to obtain desalted crude extracts. 250 μL of the crude extracts, 50 μL each of buffers at various pH values (pH 4.0, 5.0, and 6.0: 0.5 M sodium citrate, pH 7.0: 0.5 M HEPES, pH 8.0 and 9.0: 0.5 M Tris-HCl, pH 10.0: 0.5 M glycine), and 200 μL of distilled water were mixed and injected to a 24-well plate. The plate was incubated for 2 days in an ice bath and then subjected to the same evaluation using brine shrimp as above. Life and death were determined 36 hours after addition of the animals to the wells. The results are shown in Figure 5.

[Example 2] Purification of toxic component

A toxic component in aqueous extracts of a sponge was purified by the following treatment. First, 100 g of a sponge sample was subjected to extraction with a Tris-HCl buffer (100 mM, pH 8.0) containing 200 mM NaCl and 0.5% w/v kojic acid. 12.5 mL of 4 M ammonium sulfate was added to 37.5 mL of the obtained crude extracts, and HiTrap Butyl FF column for hydrophobic interaction chromatography (5 mL, GE Healthcare) was loaded with the mixture. The column was washed with 1 M ammonium sulfate until the absorbance of the eluate at 280 nm reached less than 0.05 AU. Subsequently, the column was subjected to gradient elution with 1 M ammonium sulfate-water (Figure 6), and the toxicity of each fraction thus obtained was evaluated using brine shrimp. Fractions having toxicity were combined, and HiPrep 26/10 Desalting column (53 mL, GE Healthcare) was loaded therewith for desalting, followed by elution with a Tris-HCl buffer (100 mM, pH 8.0). RESOURCE-ISO column for hydrophobic interaction chromatography (1 mL, GE Healthcare) was loaded with protein fractions, followed by elution with 2 M ammonium sulfate-water (Figure 7). Fractions having toxicity were combined and desalted in the same manner as above, and RESOURCE-Q column for ion-exchange chromatography (1 mL, GE Healthcare) was loaded with protein fractions, followed by gradient elution with a Tris-HCl buffer (100 mM, pH 8.0, containing 0.1 to 0.4 M NaCl) (Figure 8). Fractions having toxicity were combined and concentrated into 100 μL by ultrafiltration, and Superdex 200 10/300 GL column for gel filtration (GE Healthcare) was loaded therewith, followed by elution with a 50 mM Tris-HCl buffer (pH 9.0, containing 0.2 M NaCl). Activity was confirmed in a peak fraction at an elution volume of 12.3 mL (fraction A) (Figure 9). AKTA FPLC system (GE Healthcare) was used in each of the chromatography methods described above.

Table 2 shows the total amount of proteins and the degree of toxicity at each stage of purification. The total amount of proteins was quantified with γ globulin as a standard substance using BCA Protein Assay Kit (Thermo Fisher Scientific Inc.). One unit related to total activity represents activity that kills 50% animals within 36 hours in brine shrimp assay. The activity recovery rate represents the ratio of the total activity of fractions at each purification stage to the total activity of crude extracts.

Table 2 Activity at each purification stage

| Purification stage | Total amount of proteins (mg) | Total activity (Unit) | Activity recovery rate (%) | Relative activity (Unit/mg) |
|---|---|---|---|---|
| Crude extract | 2400 | 556,000 | 100 | 230 |
| HiTrap Butyl FF | 294 | 234,000 | 42 | 796 |
| RESOURCE-ISO | 32 | 15,400 | 2.8 | 481 |
| RESOURCE-Q | 2.5 | 18,500 | 3.3 | 7370 |
| Superdex | 0.14 | 19,400 | 3.5 | 137,588 |

The molecular weight of the active component was predicted to be 145 KDa from the distribution coefficients (Kav) of the fraction A and standard substances (glutamate dehydrogenase (290 kDa), lactose dehydrogenase (142 kDa), enolase (67 kDa), myokinase (32 kDa) and cytochrome C (12.4 kDa)) calculated by gel filtration chromatography (Figure 10).

As a result of analyzing the fraction A by SDS-PAGE, the darkest band was found at a position of 120 kDa (Figure 11, arrow in the right lane).

[Example 3] Isolation of toxic component

A method of purifying a toxic component was developed that permits a more rapid, large-scale process. First, 620 g of a sponge sample was subjected to extraction with a Tris-HCl buffer (100 mM, pH 8.0) containing 200 mM NaCl and 0.5% w/v kojic acid. Ammonium sulfate (final concentration: 80%) was added at 0°C to the obtained crude extracts, and the mixture was stirred at 0°C for 1 hour and subjected to centrifugation at 8500 rpm at 4°C for 50 minutes. The precipitates were redissolved in a 50 mM Tris-HCl buffer (pH 8.0) containing 1 M ammonium sulfate (redissolved solution). 100 mL of the redissolved solution was added to a suspension of 100 mL of TOYOPEARL® Butyl-650 M gel (Tosoh Corp., for hydrophobic interaction chromatography, support particle diameter: 40 to 90 μm) in 50 mM Tris-HCl (pH 8.0) containing 1 M sodium sulfate, followed by overnight stirring for adsorption to the gel. A column was filled with the obtained gel preparation, and the adsorbed protein was eluted with a 50 mM Tris-HCl buffer (pH 8.0) containing 0.9 M, 0.7 M and 0.2 M sodium sulfate until the absorbance of each eluate at 280 nm reached less than 0.05 AU. Active fractions eluted with the buffer containing 0.2 M sodium sulfate were dialyzed against 50 mM Tris-HCl (pH 9.0) containing 0.1 M NaCl, and RESOURCE-Q column (1 mL) was loaded therewith. The adsorbed protein was eluted in a gradient of a Tris-HCl buffer (100 mM, pH 8.0, 0.1 to 0.6 M NaCl). Final purification was performed by HPLC (pump: PU-890 (JASCO), detector: MD-4015 (JASCO), eluent: 50 mM Tris-HCl, pH 8.0, containing 0.2 M NaCl) using BioSec 5 column for gel filtration (pore size: 500 angstroms, Agilent Technologies, Inc.). Figure 12 shows results of analyzing samples at each purification stage by SDS-PAGE. Figure 13 shows results of purification by HPLC. Lane 4 of Figure 12 was loaded with only the toxic component (soritesidine; also abbreviated to SOR) finally isolated by HPLC.

[Example 4] Physicochemical characteristics of toxic component

SOR isolated in Example 3 was analyzed with MALDI-TOF (AXIMA-CFR plus (Shimadzu Corp.), sinapic acid was used as a matrix). As shown in Figure 14, the spectrum of SOR contained a cluster of singly charged ions centered at m/z = 108,766 and a cluster of doubly charged ions centered at m/2z = 54,874. These results also fall in with the results of SDS-PAGE of Examples 2 and 3.

Next, the isoelectric point of SOR was determined by two-dimensional polyacrylamide gel electrophoresis (2-DE). First, 443 μg of a sample was mixed with a rehydration solution (8 M urea, 0.5% 3-[(3-cholamidopropyl)dimethylammonio]-propanesulfonic acid (CHAPS), 0.5% Bio-Lyte 3/10 (Bio-Rad Laboratories, Inc.), 0.2% dithiothreitol, 0.002% bromophenol blue). The obtained solution was applied to IPG Strip 3-6 (Bio-Rad Laboratories, Inc.). Isoelectric focusing electrophoresis was performed at 20°C as follows: rehydration for 12 hours, 300 V for 30 minutes, 1000 V for 30 minutes, and 5000 V for 2 hours. After electrophoresis, the strip was equilibrated for 15 minutes with shaking in 2% SDS, 50 mM Tris-HCl, pH 8.8, 6 M urea, 30% glycerol, and 0.25% DTT. Subsequently, the strip was re-equilibrated for 15 minutes with shaking in 2% SDS, 50 mM Tris-HCl, pH 8.8, 6 M urea, 30% glycerol, and 4.5% iodoacetamide. The strip was put on a 12% polyacrylamide gel, and the second electrophoresis was performed in the same manner as in SDS-PAGE. The gel was stained with a silver staining kit (Wako Pure Chemical Industries, Ltd.). As shown in Figure 15, the band of SOR was found around an isoelectric point (pI) of approximately 4.5.

[Example 5] Study on bioactivity of toxic component

(1) Cytotoxicity

The cytotoxicity of SOR was evaluated using L1210 (RBRC-RCB2844, Riken) and HeLa (RBRC-RCB0007, Riken) cells. RPMI1640 medium supplemented with 10% FBS was used for the L1210 cells, and EMEM supplemented with 10% FBS was used for the HeLa cells. The cells were precultured for 3 days (L1210) or 7 days (HeLa) using a $CO_2$ incubator (37°C, 5% $CO_2$). For L1210, the cells that reached confluency ($1 \times 10^5$ cells/mL) were inoculated to a 96-well microtiter plate (50 $\mu$L/well). SOR dissolved in a medium (200 $\mu$g/mL) was filtered (0.22 $\mu$m), and serially diluted 2-fold, and the diluted products were added to wells in an amount of 50 $\mu$L/well such that the final concentration of SOR was 50 to 0.024 $\mu$g/mL. For the HeLa cells, cells that reached confluency ($2 \times 10^4$ cells/mL) were inoculated to a 96-well microtiter plate (100 $\mu$L/well) and incubated for 24 hours. SOR dissolved in a medium (200 $\mu$g/mL) was filtered (0.22 $\mu$m), and serially diluted 2-fold, and the diluted products were added to wells in an amount of 10 $\mu$L/well such that the final concentration of SOR was 50 to 0.024 $\mu$g/mL. The cells of each line were incubated for 48 hours, and the growth of the cells was quantified using Cell Counting Kit-8 (Dojindo Laboratories Co., Ltd.) based on the WST-8 method according to manufacturer's protocol. Absorption at 450 nm was measured using a microplate reader, and a mean of triplicate data was plotted to prepare a concentration-response curve, followed by analysis using Prism software. As shown in Figure 16, SOR exhibited strong cytotoxicity to both the cells ($IC_{50}$ value: 12.11 ng/mL (110 pM) for the L1210 cells and 0.062 ng/mL (0.517 pM) for the HeLa cells). Also, SOR exhibited selectivity approximately 200 times higher for the HeLa cells than for the L1210 cells. For reference, Table 3 shows comparison with the previously reported cytotoxicity of other compounds.

Table 3 Cytotoxicity of SOR

| Compound | $IC_{50}$ | Reference |
|---|---|---|
| SOR | 0.517pM (HeLa) 100pM (L1210) | |
| Paclitaxel | 2.6nM (HeLa) | Liebmann et al., Br J Cancer. 1993;68(6):1104-9 |
| Cytochalasin D | 0.1$\mu$M (HeLa) | Miranda et al., J Cell Biol. 1974;61(2):481-500 |
| Swinholide A | 22nM (L1210) | Kobayashi et al., Chem Pharm Bull (Tokyo). 1994;42(1):19-26 |
| Palytoxin | ~ 0.7pM (A549) | Sawelew et al., BioRxiv 2018, 292219 |
| Maitotoxin | ~ 58pM (smooth muscle cell) | Frew et al., Toxicon. 2008;51(8): 1400-8 |

(2) Toxicity in brine shrimp

The $LC_{50}$ value of isolated SOR for brine shrimp was determined in the same manner as in Example 1. The results are shown in Figure 17. For comparison, the $LC_{50}$ value was also determined 48 hours after exposure to paclitaxel, cytochalasin D and swinholide A. The results are shown in Table 4.

Table 4 Toxicity of SOR in brine shrimp

| Compound | $LC_{50}$ ($\mu$g/mL, $\mu$M) |
|---|---|
| SOR | 0.34, 0.0031 |
| Paclitaxel | 0.86, 0.99 |
| Cytochalasin D | 0.29, 0.34 |
| Swinholide A | 0.55,0.40 |

(3) Toxicity in *Aplysia kurodai* egg

Eggs were taken out of a newly deposited egg mass of *Aplysia kurodai,* and influence on cleavage was examined using the collected eggs. Eggs were taken out of the egg mass in artificial seawater (ASW) and gently centrifuged by hand centrifuge apparatus. The precipitated eggs were rinsed with ASW twice. Filtered natural seawater was added to a 24-well plate (2 mL/well), to which SOR (10 μL/well) and subsequently the liquid containing the eggs (20 μL/well) were added. Then, egg development was observed under microscope every few hours. As shown in the results of Figure 18, SOR had concentration-dependent influence on the morphology of the eggs. Untreated control eggs underwent the second cleavage 6 hours after fertilization and developed into morula in 24 hours; however, only two blastomeres were observed even 24 hours later in eggs treated with a high concentration of SOR (1.3 μg/mL), suggesting that the second cleavage was inhibited. On the other hand, in eggs treated with a high concentration of SOR (1.3 ng/mL), overall shape of the cells was shrunken and bubble-like protrusions on the cell surface were often observed, though development progressed.

(4) Toxicity in mice

Toxicity in mice was assessed by intracerebroventricularly injecting varying concentrations of SOR to mice (ddY, male, 3 to 4 weeks old, Japan SLC, Inc.) (Sakai et al., J Pharmacol Exp Ther. 2001;296(2):650-8). Although no acute toxicity was observed, very potent behavioral toxicity developed over time. For example, the administration of 574 ng of SOR resulted in loss of voluntary movement 14 hours later, made the mice lose their normal posture 17 hours later, and killed the mice 18 hours later. Reddish ring around eyes was observed in mice given SOR 14 hours or later after administration (Figure 19). Administration at a dosage of 5.74 ng/mouse (1.60 pmol/kg) was also found to cause lethal toxicity by no later than 48 to 54 hours after administration. For reference, Table 5 shows comparison with the previously reported toxicity of other compounds.

Table 5 Toxicity of SOR in mice

| Compound | Lethal dose | References |
|---|---|---|
| SOR | 1.60 pmol/kg (i.c.v.) | |
| Maitotoxin | 37.9 pmol/kg (i.p.) | Yokoyama et al., J Biochem. 1988;104(2):184-7 |
| Palytoxin | 56.0 pmol/kg (i.v.) | Moore and Scheuer, Science. 1971;172(3982):495-8 |
| CrTX-A | 465 pmol/kg (i.v.) | Nagai et al., Biochem Biophys Res Commun. 2000;275(2):582-8 |

[Example 6] Determination of amino acid sequence of toxic component

(1) Determination of N-terminal amino acid sequence and amino acid sequence of digest

The N-terminal amino acid sequence of SOR was determined by Edman degradation. A semi-pure sample of SOR (hydrophobic interaction chromatography fraction at the second stage) was separated by SDS-PAGE and then blotted to PVDF membrane by electroblotting, and the membrane was stained with Ponceau S solution. A band portion of 120 kDa was excised, and the N-terminal amino acid sequence (KLGDQRQIDIASWNTFDGGVXKAN, SEQ ID NO: 10) was determined using a protein sequencer (PPSQ-21, Shimadzu Corp.).

In order to obtain an internal sequence of SOR, the band of 120 KDa described above was placed in a microtube, immersed in a very small amount of methanol, and then subjected to reduction treatment in a DTT solution. Next, 3.0 mg of monoiodoacetic acid and 10 μg of 1 M NaOH were added thereto, and the mixture was stirred for 20 minutes in the dark. The PVDF membrane was washed by immersing in pure water and subsequently 5% acetonitrile for 5 minutes. 50 μL of a Lys-C digestion solution (30% 20 mM Tris-HCl, pH 9.0, 70% acetonitrile) was added to the obtained membrane, and 1 μL of *Achromobacter* protease I (Wako Pure Chemical Industries, Ltd., 1 pmol/mL) was added thereto, followed by treatment at room temperature for 1 hour. The digests (50 μL) were separated by HPLC (Cadenza CD-C18 column, 0.05% TFA-acetonitrile). Then, peaks were separated, and measurement by MALDI-TOFMS was performed therefor. Fractions found to have ions were each subjected to automatic Edman degradation using a protein sequencer (PPSQ-21, Shimadzu Corp.) to determine a total 20 peptides and a total of 160 N-terminal residues, as shown in Table 6.

Table 6 Amino acid sequences of SOR digests

| | |
|---|---|
| XGH | SEQ ID NO: 11 |

(continued)

| | |
|---|---|
| TGAXXXE | SEQ ID NO: 12 |
| SGXST | SEQ ID NO: 13 |
| (Q/G)AGFVPNXTXDXT | SEQ ID NO: 14 |
| NFDY | SEQ ID NO: 15 |
| LALEVPLRTVNXT | SEQ ID NO: 16 |
| GDGENXNDNXDXYD | SEQ ID NO: 17 |
| ASTGSTIPXGXXT | SEQ ID NO: 18 |
| ESAAETEN (E/G) | SEQ ID NO: 19 |
| ASAAPTNN(N/A)XTSLSSGXD(E/G) | SEQ ID NO: 20 |
| XLD(D/E)ETLE | SEQ ID NO: 21 |
| PLDVRGTY (D/E)XXXV | SEQ ID NO: 22 |
| HXQDRIQPAXPPXH | SEQ ID NO: 23 |
| ANTGIGNVVIERTDNPNTVPYIPA(A/G)GXNNNHXH | SEQ ID NO: 24 |
| FAVITLGDLNADGXH | SEQ ID NO: 25 |
| LPG | SEQ ID NO: 26 |

(2) Determination of gene sequence

Next, an attempt was made to determine the gene sequence of SOR by use of the amino acid sequence. The sequencing is summarized in Figure 20, and the primers used are shown in Table 7.

Table 7 List of primers used in sequencing

| Name | Sequence | SEQ ID NO |
|---|---|---|
| SOR1Fw | CARMGNCARATHGAYATHGCN | 27 |
| SOR1Rev | A TNGYNSWNCCNGTNSWNGC | 28 |
| SOR2Fw | TGGAAYACNTTYGAYTTYGG | 29 |
| SOR4Rev | CCNGCNGCNGGDATRTANGG | 30 |
| SOR3Fw2 | CAAATGTCACAAATCCATGTAATCCTGC | 31 |
| SOR4Fw1 | ATGATCCTACCATACTGCAAGC | 32 |
| SOR4Fw2 | GATACTGGCATTGTAGGCTCTGG | 33 |
| SOR5Fw1 | GGCACTCTCTGATCATAGACTG | 34 |
| SOR5Fw2 | CAGCTGATGAGGTATACGTCAAAGG | 35 |
| SOR-inv1 | AATCTCTTGAACCAAAATGACATCATGC | 36 |
| SOR-inv2 | CTTACATACACCGCCAAACTTGC | 37 |
| SOR3'-1 | GTGCTGGTTTTAGATGTGTGGCTTGTGC | 38 |
| SOR3'-2 | TCTGGACATATCAAGGCCCTGCAGGTGG | 39 |
| SOR5'-4 | CCATTAAACTTGAAAACATGACAGGGCC | 40 |
| SOR5'-5 | TGATCTTACAGTACAACCAGCAGCACTG | 41 |
| UPM (Long) | CTAATACGACTCACTATAGGGCAAGCAGTGGTATCAACGCAGAGT | 42 |
| UPM (Short) | CTAATACGACTCACTATAGGGC | 43 |
| NUP | AAGCAGTGGTATCAACGCAGAGT | 44 |

(continued)

| Name | Sequence | SEQ ID NO |
|---|---|---|
| rSORFw | GTCGACAAGCTTGGAGATCAGCGACAG | 45 |
| rSORRev | GAATTCCTAACAGCCAGTACCTGGAAG | 46 |

(2-1) Extraction and purification of total RNA, preparation of cDNA and extraction and purification of genome DNA

A sponge *Spongosorites* sp. collected in Iriomote Island of Okinawa, Japan in 2002 was used as a sample for cDNA preparation and for genome DNA extraction. The sponge sample was immersed in RNAlater (Qiagen N.V.) immediately after collection, preserved at -20°C for a few days, and then preserved at -80°C until use. Total RNA was prepared using RNeasy mini kit (Qiagen N.V.). Purified nucleic acids were assayed with NanoDrop (Thermo Fisher Scientific Inc.), and the presence or absence of the nucleic acid was confirmed by 0.8% agarose gel electrophoresis. 1$^{st}$ strand cDNA was synthesized from the total RNA using SMARTer® RACE cDNA Amplification Kit (Takara Bio Inc.). DNA Isolation Kit ISOHAIR (Nippon Gene Co., Ltd.) was used in genome DNA extraction.

(2-2) 1$^{st}$ strand PCR

First, in order to obtain information on a short gene sequence by PCR, degenerate primers (SOR1w, SOR1ev, SOR2Fw, and SOR4Rev) were prepared (Table 7) on the basis of the N-terminal amino acid sequence and internal sequence information (Table 6) obtained in the section (1). RNA was extracted from the sponge preserved in RNAlater immediately after collection, and 1$^{st}$ strand cDNA was synthesized therefrom and used as a template in nested PCR. 1$^{st}$ PCR was performed using SOR1Fw and SOR1Rev, and 2$^{nd}$ PCR was performed with the 1$^{st}$ PCR product as a template using SOR2Fw and SOR4Rev. KOD Dash (Toyobo Co., Ltd.) was used as a PCR enzyme. The PCR product of the 2$^{nd}$ PCR was electrophoresed in an agarose gel. Then, a band around 300 bp was excised and purified using AxyPrep DNA Gel Extraction Kit (Axygen Biosciences, Inc.). A sequence of 115 amino acids long (SEQ ID NO: 48) corresponding to the nucleotide sequence of the obtained PCR product of 344 bp (SEQ ID NO: 47) contained a moiety well consistent with the sequence information obtained by Edman degradation (amino acid residues indicated in red in Figure 21).

(2-3) 3' RACE (Miss priming PCR)

Next, in order to obtain a 3' total sequence, 3' RACE was attempted. First, primers (SOR4Fw1 and SOR4Fw2) were prepared (Table 7) on the basis of the nucleotide sequence obtained above by 1$^{st}$ strand PCR, and nested PCR was performed with 3' RACE ready cDNA as a template. 1$^{st}$ PCR was performed using SOR4Fw1, SOR4Fw2 and UPM (Universal Primer Mix, Clontech Laboratories, Inc.), and 2$^{nd}$ PCR was performed with the 1$^{st}$ PCR product as a template using SOR4Fw2 and a universal primer NUP (nested universal primer). Phusion™ High-Fidelity DNA polymerase (NEB) was used as a PCR enzyme. The PCR product of the 2$^{nd}$ PCR was electrophoresed in an agarose gel. Then, a band around 1000 bp was excised and purified using AxyPrep DNA Gel Extraction Kit.

As a result, expected 3'-terminal poly-A was absent, and a PCR product of 884 bp (SEQ ID NO: 49) containing a sequence complementary to SOR4Fw2 was instead obtained. Although this might be a PCR error, this nucleotide sequence was converted, to be safe, to an amino acid sequence (SEQ ID NO: 50), which consequently contained a sequence well consistent with the amino acid sequence information obtained by amino acid sequencing in the section (1) (amino acid residues indicated in red in Figure 22). This revealed that this PCR product resulted from the 3' miss priming of the primer SOR4Fw2 used because the 3' nucleotide sequence was incidentally complementary to the primer. This PCR approach is designated as "Miss priming PCR".

(2-4) 3'-RACE

In order to amplify the 3'-terminal moiety of the SOR-derived gene by use of RACE, primers (SOR5Fw1 and SOR5Fw2) were prepared on the basis of the nucleotide sequence of 884 bp obtained by "Miss priming PCR" mentioned above, and nested PCR was performed with 3' RACE ready cDNA as a template. 1$^{st}$ PCR was performed using SOR5Fw1 and the same UPM as in the section (2-3), and 2$^{nd}$ PCR was performed with the 1$^{st}$ PCR product as a template using SOR5Fw2 and the same NUP as in the section (2-3). Phusion™ High-Fidelity DNA polymerase (NEB) was used as a PCR enzyme. The PCR product of the 2$^{nd}$ PCR was electrophoresed in an agarose gel. Then, a band around 2.5 kbp was excised and purified using AxyPrep DNA Gel Extraction Kit. As a result, 4 clones of PCR products having poly-A at the 3' end were obtained. Figure 23 shows the sequence (SEQ ID NO: 51) of one of the clones. Some sequences were inconsistent with the sequence information obtained by Miss priming PCR, though most of sequences were consistent therewith. This nucleotide sequence was converted to an amino acid sequence (SEQ ID NO: 52), which consequently contained a sequence well consistent with the amino acid sequence information obtained by amino acid sequencing. The amino acid sequence indicated in red in the sequence information shown in Figure 23 is the sequence well consistent with the amino acid sequencing.

(2-5) Inverse PCR

Next, in order to examine the 5' upstream nucleotide sequence of SOR, a plurality of attempts were made to amplify the 5'-terminal sequence by use of RACE with the 1st strand cDNA obtained in the section (2-1) as a template. However, no product was obtained. Accordingly, inverse PCR was performed with the genome DNA obtained in the section (2-1) as a direct template to amplify the 5'terminal sequence of the SOR-derived gene. The inverse PCR is an approach of performing PCR by using, as a template, circular DNA obtained by the ligation of both ends of a template cleaved with a restriction enzyme, and has the advantage that the whole nucleotide sequence in the ring can be read from a portion of sequence information (Figure 24).

First, a sample solution containing the genome DNA and a restriction enzyme PstI was prepared and incubated at 16°C for 5 hours. Then, 10 μL of a purified product was obtained using AxyPrep PCR Clean-up Kit (Axygen Biosciences, Inc.). The product was mixed with 10 μL of Ligation Mix (Takara Bio Inc.) and self-ligated by overnight incubation at 4°C to prepare a plasmid, which was in turn used as a template in inverse PCR.

Next, nucleic acid amplification was performed by nested PCR using primers (SOR3Fw2, SOR-inv1, and SOR-inv2) prepared on the basis of the sequence determined by 1st strand PCR and SOR4Fw2 (Table 7) used in the section (2-3). Phusion™ High-Fidelity DNA polymerase (NEB) was used as a PCR enzyme. The PCR product amplified by 1st PCR (SOR3Fw2 and SOR-inv1) was used as a template in 2nd PCR (SOR4Fw2 and SOR-inv2). The PCR product of the 2nd PCR was electrophoresed in an agarose gel. Then, a band around 1.5 kbp was excised and purified using AxyPrep DNA Gel Extraction Kit.

As a result, a PCR product of 1607 bp (SEQ ID NO: 53) was obtained. The nucleotide sequence information was converted to an amino acid sequence (SEQ ID NO: 54, 55), which consequently contained a sequence consistent with the sequence information obtained by amino acid sequencing in the section (1). The amino acid sequence indicated in red in the sequence information given below is the sequence consistent with the amino acid sequencing (Figure 25). The nucleotide sequence of this PCR product was also well consistent with the nucleotide sequences of the PCR products obtained by 1st strand PCR and Miss priming PCR. However, this nucleotide sequence had no PstI digestion site to be produced during self-ligation. A nucleotide sequence up to 4 bases upstream of the PstI digestion site supposed to reside was completely consistent with the nucleotide sequences obtained from the other PCR products. The sequence indicated in red arrow in Figure 25 is the sequence different from the other PCR products, and the sequence boxed in orange is the sequence supposed to be the PstI site. A start codon for methionine was absent before a stop codon upstream from the N-terminal amino acid sequence determined by amino acid sequencing.

(2-6) Full length PCR for determining whole translated region of SOR

The experiments described above were able to produce fragmentary nucleotide sequence information from a plurality of PCR products, and however, presented the problems of the absence of a start codon in their respective nucleotide sequences and no restriction site confirmed in inverse PCR. Accordingly, in order to confirm the sequence information thus obtained, the gene of SOR was obtained as one consecutive nucleotide sequence. Primers (SOR5'-5, SOR3'-2, SOR5'-4, and SOR3'-1) were prepared (Table 7) for 5' and 3' untranslated regions on the basis of the nucleotide sequences amplified by inverse PCR and 3'-RACE. Both the 1st strand cDNA and the genome DNA were used as PCR templates and each subjected to nested PCR. 1st PCR was performed using SOR5'-5 and SOR3'-2, and 2nd PCR was performed with the 1st PCR product as a template using SOR5'-4 and SOR3'-1. As a result, PCR products of 3309 bp were obtained as 14 clones from the templated 1st strand cDNA and as 4 clones from the templated genome DNA. One (SEQ ID NO: 56) of these clones was completely consistent between the nucleotide sequences of the amplified products from the 1st strand cDNA and the genome DNA as templates except for primer-derived sequences (Figure 26). This nucleotide sequence was well consistent with the nucleotide sequences of the other PCR methods, and however, differed by 2 bases from the nucleotide sequence obtained by 3'-RACE, and an amino acid predicted therefrom also differed (bases and amino acid indicated in blue in Figure 26). The whole 947-amino acid sequence (SEQ ID NO: 57) of SOR was predicted by this PCR. An amino acid sequence up to N-terminal residue 311 was found to have homology with deoxyribonuclease I (DNaseI). The homology between positions 1 to 259 of bovine-derived DNaseI and positions 8 to 310 of SOR was approximately 20%. A molecular weight of 103911.07 and an isoelectric point of 4.83 were calculated from the whole amino acid sequence of the SOR translated region and were well consistent with results of preceding research. No existing protein had homology with the C-terminal amino acid sequence of SOR. The full-length amino acid sequence of SOR exhibited homology on the order of approximately 30% with the amino acid sequence of a predicted protein (GenBank Accession No. WP_022696775) of *Alphaproteobacteria Euryhalocaulis caribicus.*

[Example 7] Recombinant expression of SOR

In order to ligate the sequence of SOR with a vector for expression, recognition sites of restriction enzymes were introduced to the N terminus and C terminus of SOR by PCR using, as a template, a pTAC-1 plasmid harboring 5'-RACE ready cDNA of the SOR gene obtained in Example 6. The gene-specific primers used were rSORFw and rSORRev (Table 7) for introducing a recognition site (GTCGAC) of SalI (Takara Bio Inc.) to the N-terminal sequence of SOR and a recognition site (GAATTC) of EcoRI (Takara Bio Inc.) to the C-terminal sequence thereof. Phusion™ High-Fidelity DNA polymerase (NEB) was used as a PCR enzyme. This reaction produced rSOR.

rSOR harboring the recognition sites of the restriction enzymes and a plasmid vector pEcoli-Nterm-6×HN Vector (Clontech Laboratories, Inc.) for expression were digested with SalI and EcoRI. Specifically, 2.5 μL (15 U/μL) of SalI, 2.5 μL (15 U/μL) of EcoRI, 5.0 μL of 10 × H buffer (Takara Bio Inc.), and 10 μL of rSOR/pEcoli-Nterm-6×HN Vector were each adjusted to 50 μL with $dH_2O$ and incubated at 37°C for 1 hour. Each restriction enzyme digestion product was subjected to agarose electrophoresis, and a band was excised and purified. After confirmation of concentrations using NanoDrop, the vector and the insert were mixed at vector:insert = 1:3 to prepare a DNA solution. Subsequently, Ligation Mix and the DNA solution were mixed at Ligation Mix:DNA solution = 1:1 and incubated at 25°C for 3 hours for ligation reaction.

Competent cells NEB® 10-beta Competent E. coli (NEB) for cloning were transformed with the obtained ligation reaction solution, and plasmids were purified (see Example 6(2)). Shuffle® T7 Express lysY Competent E. coli (NEB) was transformed with the purified plasmids by electroporation (see Example 6(2)), then smeared over the surface of LB agar medium containing ampicillin (100 μg/mL), and cultured at 30°C for approximately 16 hours. A very small amount of the obtained colony was picked up using a bamboo skewer, inoculated to a liquid medium containing ampicillin, and shake-cultured at 30°C until $OD_{600}$ = 0.4 to 0.8. Then, IPTG (final concentration: 0.4 mM) was added thereto, followed by shake culture at 30°C for 4 hours. The obtained culture solution was centrifuged at 6000 rpm for 10 minutes to collect E. *coli,* which was then suspended in a homogenization buffer (50 mM Tris-HCl, pH 7.4, 150 mM NaCl) in a volume of 1/20 times the culture solution. *E. coli* was lysed by repetitive sonication. The suspension was centrifuged again to obtain liquid cell extracts as a supernatant and cell homogenates as precipitates. The liquid cell extracts and the cell homogenates were subjected to SDS-PAGE. The results are shown in Figure 27. A band around 116 kDa corresponding to SOR was found in the cell homogenates.

[Example 8] Cleavage of DNA by toxic component

(1) Natural SOR

Plasmid DNA (pTAC-1, 4 ng, BioDynamics Laboratory, Inc.), or the plasmid DNA and EDTA (12.5 mM) were added to and mixed with a hydrophobic interaction chromatography fraction containing SOR (8.8 μg/mL) or a Tris-HCl buffer (pH 8.3) containing 0.1 M NaCl. The mixture was left at room temperature for 24 hours, separated by agarose electrophoresis, stained with ethidium bromide (15 min), and subsequently observed under UV light (260 nm). Thermo scientific GeneRuler 1 kb DNA Ladder (Thermo Fisher Scientific Inc.) was used as a marker. The results of Figure 28 show that SOR cleaved DNA in a metal ion-dependent manner.

(2) Recombinant SOR N-terminal moiety

DNA cleaving activity was evaluated in the same manner as in the section (1) using a recombinantly expressed polypeptide from residues 1 to 310 (SEQ ID NO: 2) of SOR. An artificial gene (rN-SOR, 930 bp (SEQ ID NO: 58), harboring a SalI recognition site (GTCGAC) on the N-terminal side and a HindIIIrecognition site (AAGCTT) on the C-terminal side, full-length insert sequence: 948 bp, SEQ ID NO: 59) having the gene sequence of N-terminal 310 residues of SOR optimized for E. *coli* codons was incorporated into pUCFa vector (2966 bp, FASMAC, SEQ ID NO: 60) having ampicillin resistance to prepare plasmid DNA (prN-SOR, 3914 bp).

NEB® 10-beta Competent E. coli (NEB) was transformed with prN-SOR by electroporation, then inoculated to LBA agar medium, and cultured at 37°C for 16 hours. Then, the obtained colony was inoculated to LBA medium and cultured at 37°C for 16 hours, followed by prN-SOR extraction using AxyPrep Plasmid Miniprep kit. Then, 2.5 μL (15 U/μL) of SalI, 2.5 μL (15 U/μL) of HindIII, 5.0 μL of 10 × H buffer, and 10 μL of prN-SOR/pET-22b(+) Vector (Novagen) were each brought up to a constant volume of 50 μL with ultrapure water and incubated at 37°C for 3 hours. Each restriction enzyme digestion product was subjected to agarose electrophoresis, and bands of rN-SOR (948 bp, insert) and pET-22b(+) Vector (5493 bp, vector) were excised and purified. Light absorption at 260 nm was measured to confirm concentrations. Then, the vector and the insert were mixed at vector:insert = 1:3 to prepare a DNA solution. Subsequently, Ligation Mix and the DNA solution were mixed at Ligation Mix:DNA solution = 1:1 and incubated at 16°C for 3 hours for ligation reaction.

NEB® 10-beta Competent E. coli was transformed with the ligation product, and the obtained E. *coli* strain was inoculated to LBA agar medium and cultured, followed by plasmid DNA extraction using AxyPrep Plasmid Miniprep kit. SHuffle T7 Express LysY Competent E. coli (NEB) was transformed with the obtained plasmid DNA. The expression strain thus obtained was suspended in 80% glycerol and preserved at -80°C.

The expression strain was smeared over the surface of LBA agar medium and cultured at 30°C for approximately 16 hours. The obtained colony was inoculated to liquid LBA medium and shake-cultured at 30°C for 16 hours. Then, IPTG (final concentration: 0.4 mM) was added thereto, followed by shake culture at 30°C for 4 hours. The obtained cultures were collected by centrifugation. The pellets were suspended in a homogenization buffer (50 mM Tris-HCl, pH 7.5/150 mM NaCl) in an amount of 1/20 times the culture solution and then subjected to repetitive sonication. The suspension was frozen and thawed and then centrifuged again to obtain a soluble fraction and a precipitated fraction.

The precipitated fraction was suspended in a 20-fold amount of 4% octyl phenol ethoxylate and 50 mM Tris-HCl, pH 9.0/0.1 M NaCl and shaken at room temperature for 30 minutes. Then, precipitates were recovered by centrifugation at 8000 rpm for 20 minutes. This operation was repeated four times. Then, the precipitated fraction was suspended in a 20-fold amount of ultrapure water, and precipitates were recovered by centrifugation at 8000 rpm for 10 minutes. This operation was repeated four times to remove octyl phenol ethoxylate. Next, the resulting precipitates were suspended in an approximately 20-fold amount of 8 M urea and 50 mM Tris-HCl, pH 9.0/0.1 M NaCl and shaken overnight at room temperature, and a supernatant was recovered by centrifugation. This supernatant was added to Ni column (Profinity IMAC Resin, Ni-Charged, Bio-Rad Laboratories, Inc.). Elution was performed first with 8 mL of 8 M urea and 50 mM Tris-HCl, pH 9.0/0.5 M NaCl and subsequently with 8 mL of 8 M urea, 50 mM Tris-HCl, pH 9.0/0.5 M NaCl, and 50 mM imidazole. Final elution was performed with 4 mL of 8 M urea, 50 mM Tris-HCl, pH 9.0/0.5 M NaCl, and 0.5 M imidazole. Each fraction was separated by 4 mL and subjected to SDS-PAGE to recover fractions containing the band of interest. Then, imidazole was removed by centrifugal ultrafiltration, and the internal solution was recovered with 1 mL of 8 M urea, 50 mM Tris-HCl, pH 9.0/0.1 M NaCl, and 10 mM DTT.

The sample was put in a cellulose tube (UC36-32-100, molecular weight cut-off: 14000, pore size: 50 angstroms, Sanko Junyaku Co., Ltd.) and dialyzed for 5 days under conditions of 20°C and 4°C for refolding. The refolding buffer used was 50 mM Tris-HCl, pH 9.0/0.1 M NaCl and replaced with a fresh one once a day. After dialysis for 5 days, and the internal dialysate was recovered and centrifuged at 8000 rpm for 10 minutes. Then, the supernatant was used as an expressed protein sample after refolding.

Plasmid DNA (4 ng) and EDTA (12.5 mM) were added to and mixed with the expressed protein (1 mg/mL) or a Tris-HCl buffer (pH 8.3) containing 0.1 M NaCl. The mixture was left at room temperature for 5 hours, separated by agarose electrophoresis, stained with ethidium bromide (15 min), and subsequently observed under UV light (260 nm). The results of Figure 29 show that the N-terminal moiety of SOR cleaved DNA in a metal ion-dependent manner.

[Example 9] Intracellular translocation of toxic component

Whether SOR could translocate into cells and further into the nuclei was studied using fluorescently labeled SOR. The sample solution containing SOR after hydrophobic column purification in Example 4 was dialyzed against 0.1 M sodium carbonate (pH 9.4) for buffer replacement, and 30 μL of FITC-I (fluorescein-4-isothiocyanate, Dojindo Laboratories Co. Ltd., 1 mg/mL in DMSO) was added to 300 μL of the solution and reacted at 4°C for 8 hours to obtain FITC-labeled SOR. Also, BSA labeled with FITC in the same manner as above was used as a control.

(1) Intracellular translocation into Ba/F3 cell

FITC-labeled SOR or FITC-labeled BSA was added in an amount of 18 μg/mL or 0.56 μg/mL in terms of protein to RPMI1640 medium containing 10% FBS and containing Ba/F3 cells at a density of 10000 cells/mL, and incubated at 37°C for 22 hours under 5% $CO_2$. Then, the cells were washed with PBS and fixed in 4% PFA and 4% sucrose/PBS(-) at room temperature for 10 minutes. Subsequently, the cells thus fixed were observed under a confocal laser microscope TCS-SP5 (Leica Camera AG). The results of Figures 30 and 31 show that FITC was localized in the cells.

(2) Intracellular translocation into HeLa cell

The same experiment as in the section (1) was also conducted for HeLa cells (derived from human uterine cervix cancer). A HeLa suspension prepared at approximately $2.2 \times 10^4$ cells/mL was added at 270 μL/well to 8-well chamber cover glass (Eppendorf) to make approximately $6.0 \times 10^3$ cells/well, followed by preculture at 37°C for 24 hours in a 5% $CO_2$ incubator. Then, 30 μL of the FITC-SOR sample (final concentration: 18 μg/well) was added to each well and incubated at 37°C in a 5% $CO_2$ incubator. The cells thus supplemented with the sample was subjected to fixing and permeabilization treatment over each time period from 30 minutes to 16 hours, and stained with DAPI. Then, a mount was prepared. The mount was observed under a confocal laser microscope (TCS-SP5), and the intracellular localization of FITC fluorescence was observed at each of the time periods after sample addition. The results are shown in Figures 32 to 34. The fluorescence of FITC was mostly localized around the cell membrane 30 minutes after addition, and some SOR molecules translocated into the cytoplasm 1 hour after addition. The localization of FITC fluorescence was consistent with the localization of DAPI fluorescence 12 hours or later thereafter. Thus, the intracellular translocation of SOR was confirmed.

The results described above suggested that: SOR has a structure similar to that of AB toxin (Odumosu et al., supra) constituted by an A subunit involved in toxicity and a B subunit involved in intracellular translocation; and the C-terminal moiety probably corresponding to the B subunit contributes to intracellular translocation and further to nuclear translocation. The results described above also indicate that SOR, particularly, its C-terminal moiety, is capable of functioning as a cellular and nuclear translocation carrier of various substances, in cooperation with the size of its molecular weight.

Those skilled in the art should understand that many various modifications can be made without departing from the spirit of the present invention. Thus, it should be understood that the embodiments of the present inventions described herein are merely given for illustrative purposes and are not intended to limit the scope of the present invention.

## Claims

1. A polypeptide having a function of delivering a substance into a cell, the polypeptide being selected from

(a) a polypeptide comprising the amino acid sequence of SEQ ID NO: 1,
(b) a polypeptide that comprises an amino acid sequence containing a mutation of one or more amino acids in the amino acid sequence of SEQ ID NO: 1, and has the function of delivering a substance into a cell,
(c) a polypeptide that comprises an amino acid sequence having 80% or higher sequence identity to the amino acid sequence of SEQ ID NO: 1, and has the function of delivering a substance into a cell, and
(d) a polypeptide that comprises an amino acid sequence encoded by a polynucleotide hybridizing under highly stringent conditions to a polynucleotide encoding the amino acid sequence of SEQ ID NO: 1, and has the function of delivering a substance into a cell.

2. The polypeptide according to claim 1, wherein the polypeptide has a function of delivering a substance into the nucleus.

3. The polypeptide according to claim 1 or 2, wherein the cell is a mammalian cell.

4. A complex of the polypeptide according to any one of claims 1 to 3 and a substance that functions intracellularly.

5. The complex according to claim 4, wherein the polypeptide according to any one of claims 1 to 3 and the substance that functions intracellularly are bound through a covalent bond and/or a non-covalent bond.

6. The complex according to claim 4 or 5, wherein the substance that functions intracellularly is selected from a toxin, an enzyme, a cell growth-inhibiting substance, an inhibitory nucleic acid molecule, a genome editing molecule, an antibody or an antigen binding fragment thereof, a signal transduction-regulating substance, a transcriptional factor, a gene and a label.

7. A fusion polypeptide comprising, in a single molecule, the polypeptide according to any one of claims 1 to 3 and a polypeptide that functions intracellularly.

8. A composition for use in delivering a substance into a cell, comprising the polypeptide according to any one of claims 1 to 3.

9. A composition for use in treating a disease that is improved by the delivery of a substance that functions intracellularly into a cell, comprising the complex according to any one of claims 4 to 6 or the polypeptide according to claim 7.

10. A method of delivering a substance that functions intracellularly into a cell, comprising the step of contacting a complex of the substance and the polypeptide according to any one of claims 1 to 3 with the cell.

11. A polypeptide having a function of degrading a nucleic acid molecule, the polypeptide being selected from

(e) a polypeptide comprising the amino acid sequence of SEQ ID NO: 2,
(f) a polypeptide that comprises an amino acid sequence containing a mutation of one or more amino acids in the amino acid sequence of SEQ ID NO: 2, and has the function of degrading a nucleic acid molecule,
(g) a polypeptide that comprises an amino acid sequence having 80% or higher sequence identity to the amino acid sequence of SEQ ID NO: 2, and has the function of degrading a nucleic acid molecule, and
(h) a polypeptide that comprises an amino acid sequence encoded by a polynucleotide hybridizing under highly stringent conditions to a polynucleotide encoding the amino acid sequence of SEQ ID NO: 2, and has the function of degrading a nucleic acid molecule.

12. A complex of the polypeptide according to claim 11 and a substance that promotes the translocation of the polypeptide into a cell.

13. The complex according to claim 12, wherein the polypeptide according to claim 11 and the substance that promotes the translocation of the polypeptide into a cell are bound through a covalent bond and/or a non-covalent bond.

14. The complex according to claim 12 or 13, wherein the substance that promotes the translocation of the polypeptide into a cell is selected from the polypeptide according to any one of claims 1 to 3, a cell-penetrating peptide, a cell binding domain of a proteinous toxin, a virus vector and a non-viral vector.

15. A fusion polypeptide comprising, in a single molecule, the polypeptide according to claim 11 and a polypeptide that promotes the translocation of the polypeptide according to claim 11 into a cell.

16. A composition for use in treating a disease that is improved by the degradation of a nucleic acid molecule, comprising the polypeptide according to claim 11 or 15 or the complex according to any one of claims 12 to 14.

17. A method of degrading a nucleic acid molecule, comprising the step of allowing the polypeptide according to claim 11 or 15 or the complex according to any one of claims 12 to 14 to act on the nucleic acid molecule.

**18.** A polypeptide having a function of degrading a nucleic acid molecule and a function of translocating into the nucleus, the polypeptide being selected from

(i) a polypeptide comprising the amino acid sequence of SEQ ID NO: 3,
(j) a polypeptide that comprises an amino acid sequence containing a mutation of one or more amino acids in the amino acid sequence of SEQ ID NO: 3, and has the function of degrading a nucleic acid molecule and the function of translocating into the nucleus,
(k) a polypeptide that comprises an amino acid sequence having 80% or higher sequence identity to the amino acid sequence of SEQ ID NO: 3, and has the function of degrading a nucleic acid molecule and the function of translocating into the nucleus, and
(l) a polypeptide that comprises an amino acid sequence encoded by a polynucleotide hybridizing under highly stringent conditions to a polynucleotide encoding the amino acid sequence of SEQ ID NO: 3, and has the function of degrading a nucleic acid molecule and the function of translocating into the nucleus.

**19.** A complex of the polypeptide according to claim 18 and a targeting molecule.

**20.** A composition for use in damaging a cell, comprising the polypeptide according to claim 18 or the complex according to claim 19.

**21.** A method of damaging a cell, comprising the step of contacting the polypeptide according to claim 18 or the complex according to claim 19 with the cell.

**22.** A polynucleotide encoding the polypeptide according to any one of claims 1 to 3, 7, 11, 15 and 18.

**23.** A vector comprising the polynucleotide according to claim 22.

**24.** A transformed cell comprising the polynucleotide according to claim 23.

**25.** A method of producing the polypeptide according to any one of claims 1 to 3, 7, 11, 15 and 18, comprising the step of culturing the transformed cell according to claim 24 under conditions suitable for the expression of the polypeptide according to any one of claims 1 to 3, 7, 11, 15 and 18.

FIG. 1

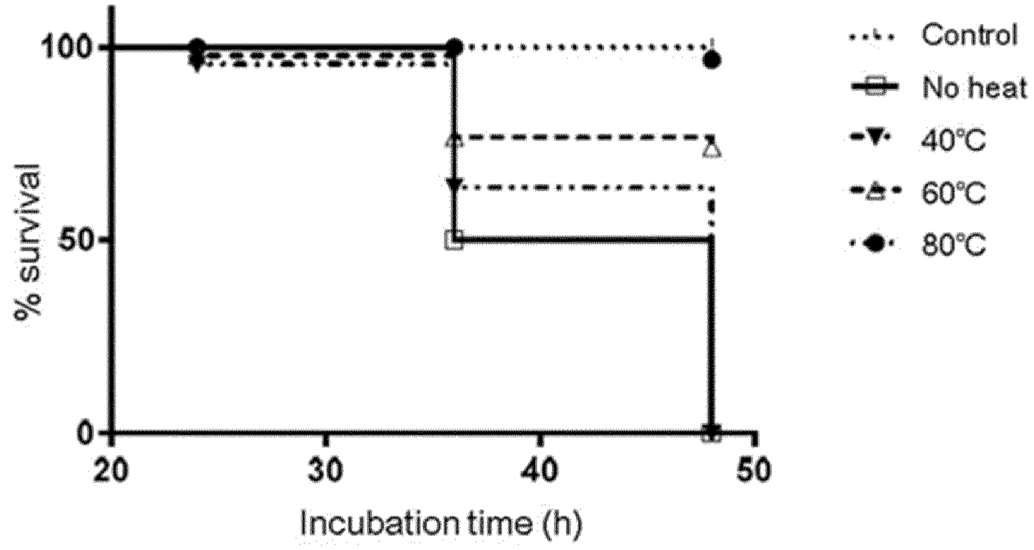
100
50
0
20
30
40
50
% survival
Incubation time (h)
Control
No heat
40°C
60°C
80°C

FIG. 2

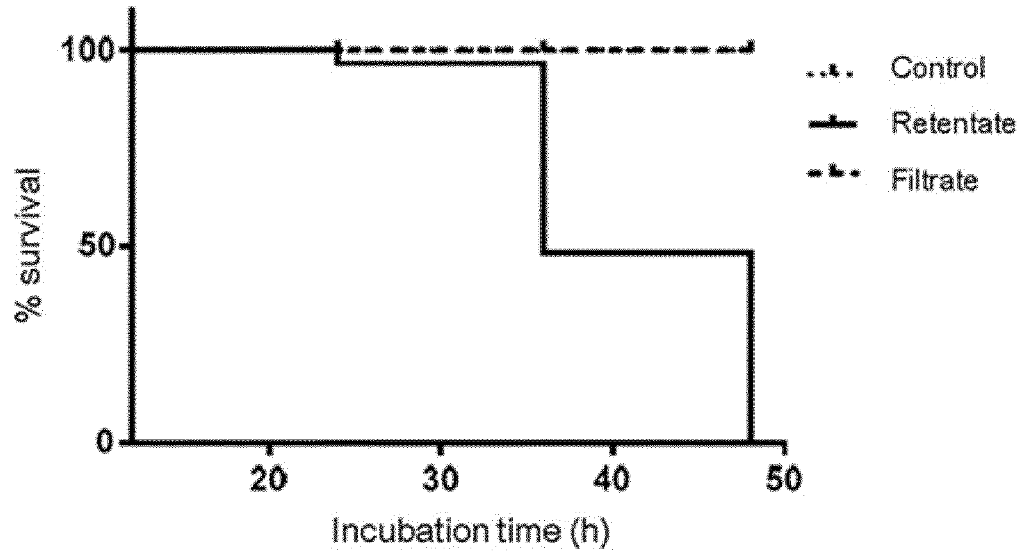
100
50
0
20
30
40
50
% survival
Incubation time (h)
Control
Retentate
Filtrate

FIG. 3

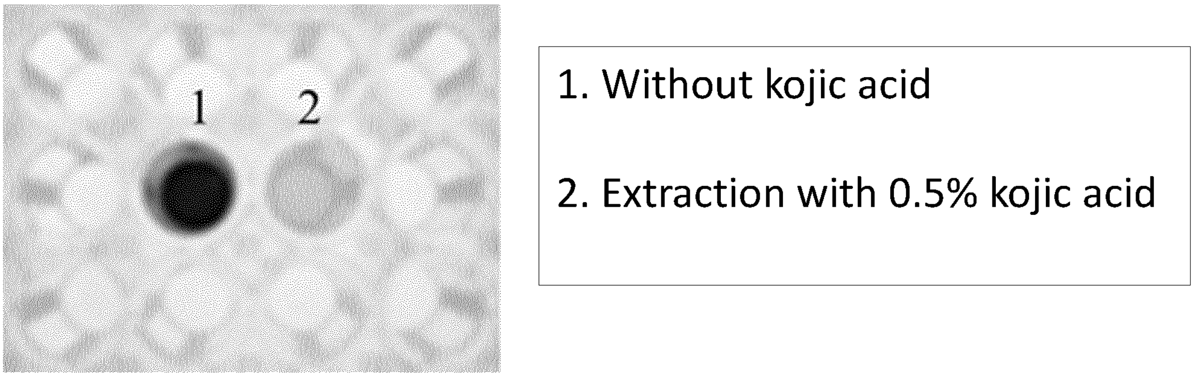
1
2
1. Without kojic acid
2. Extraction with 0.5% kojic acid

FIG. 4

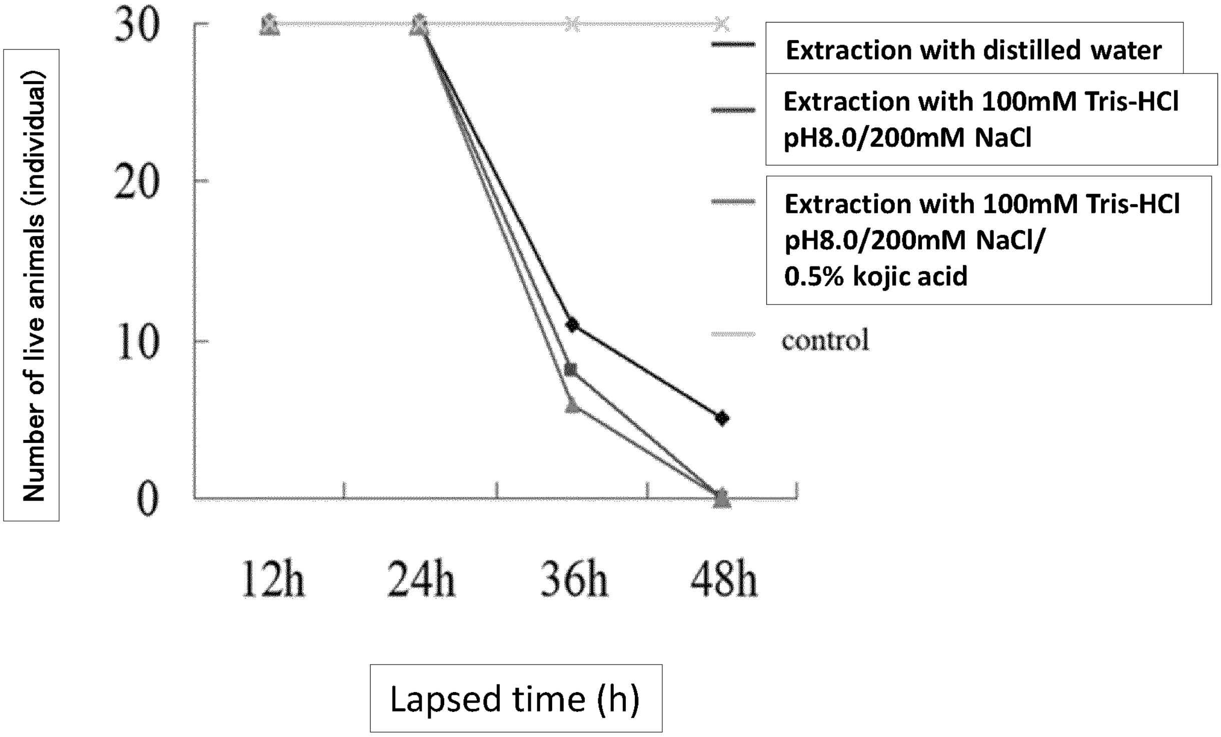
30
20
10
0
Number of live animals (individual)
12h
24h
36h
48h
Lapsed time (h)
Extraction with distilled water
Extraction with 100mM Tris-HCl pH8.0/200mM NaCl
Extraction with 100mM Tris-HCl pH8.0/200mM NaCl/ 0.5% kojic acid
control

FIG. 5

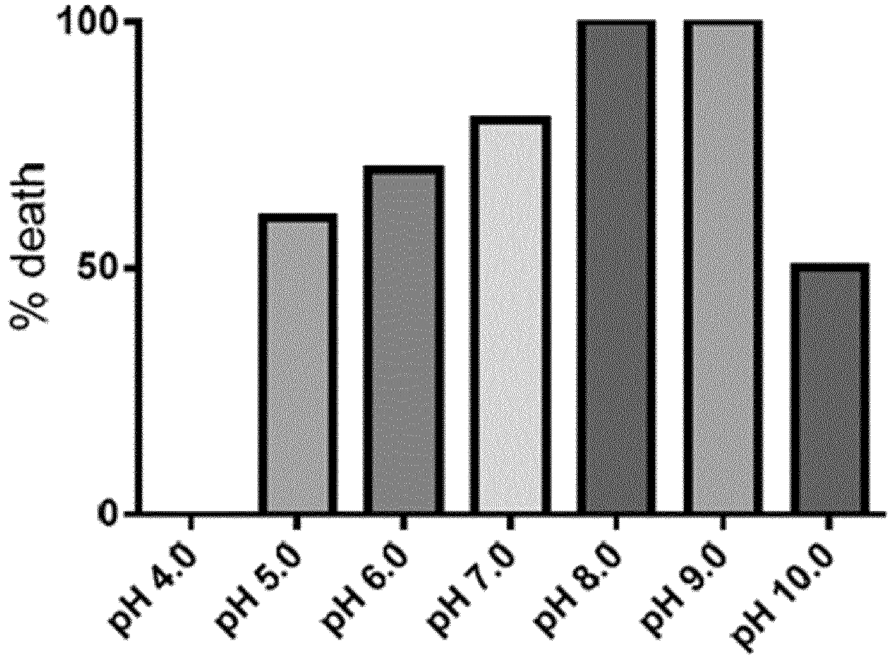
100
50
0
% death
pH 4.0
pH 5.0
pH 6.0
pH 7.0
pH 8.0
pH 9.0
pH 10.0

FIG. 6

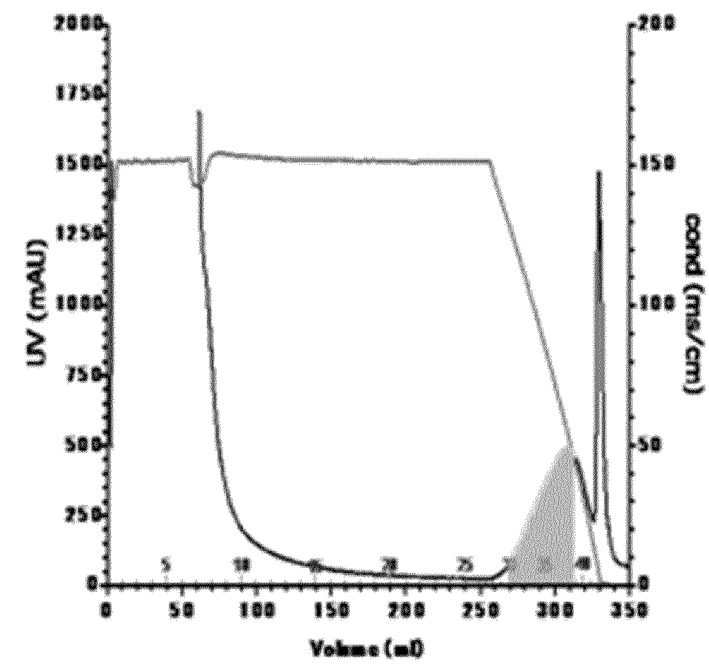
UV (mAU)
cond (ms/cm)
Volume (ml)

FIG. 7

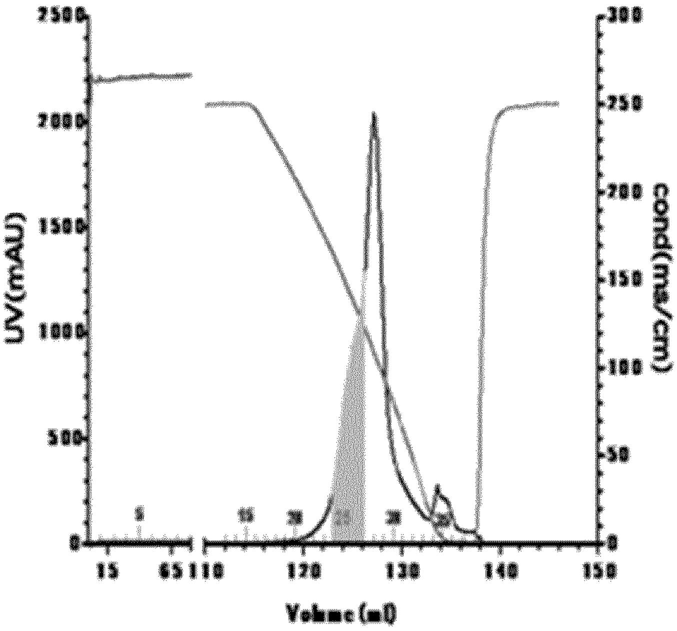


FIG. 8

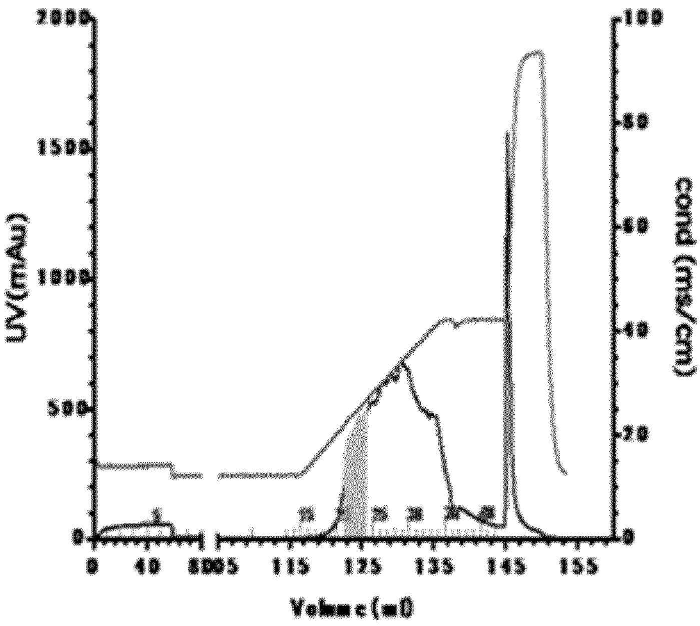

FIG. 9

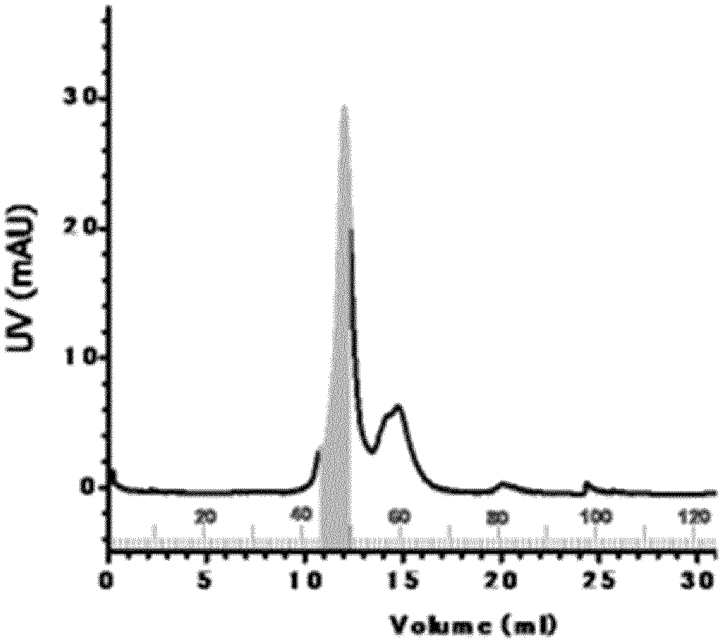
UV (mAU)
30
20
10
0
20
40
60
80
100
120
0
5
10
15
20
25
30
Volume (ml)

FIG. 10

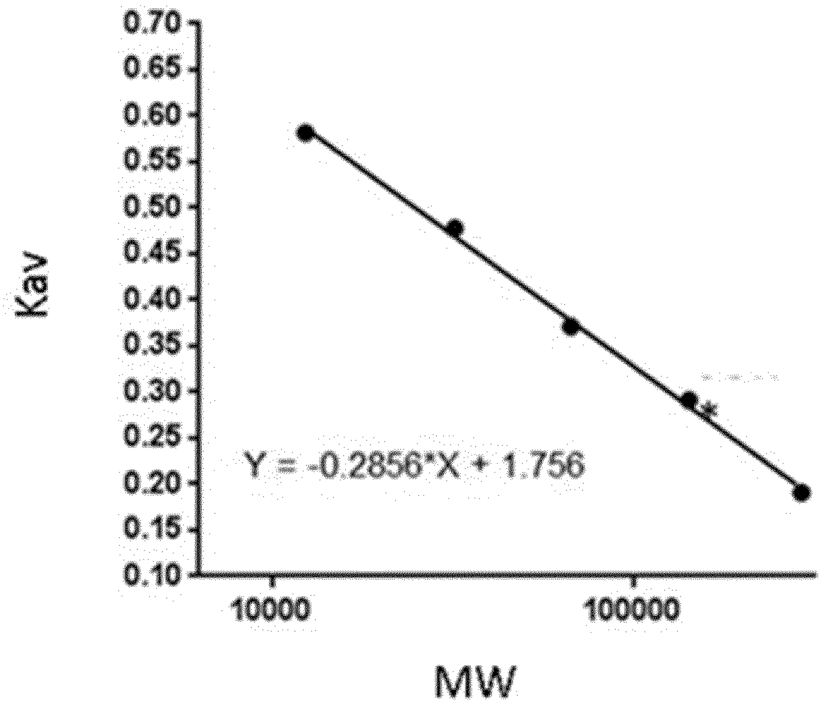
Kav
0.70
0.65
0.60
0.55
0.50
0.45
0.40
0.35
0.30
0.25
0.20
0.15
0.10
Y = -0.2856*X + 1.756
10000
100000
MW

FIG. 11

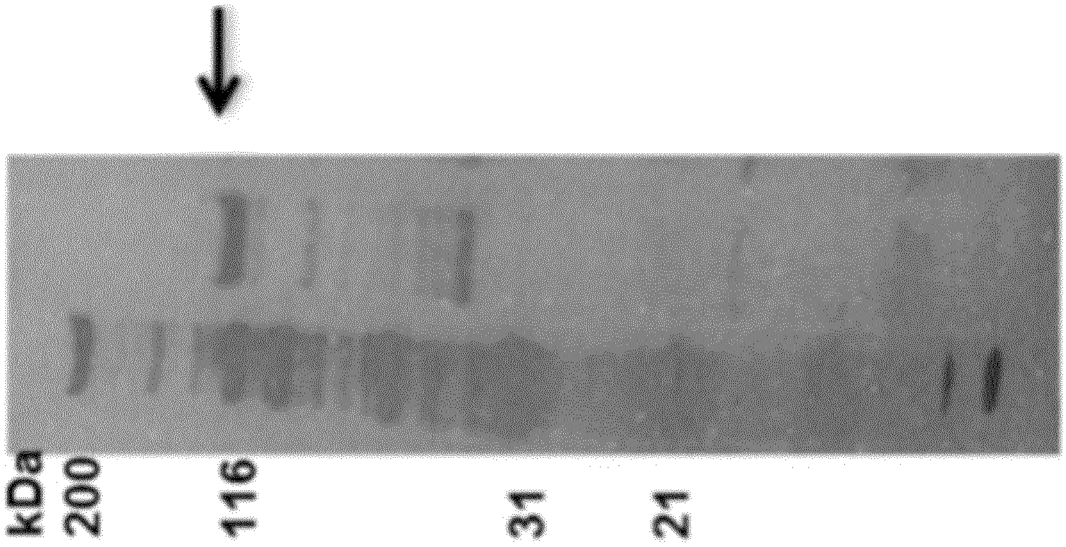
kDa
200
116
31
21

FIG. 12

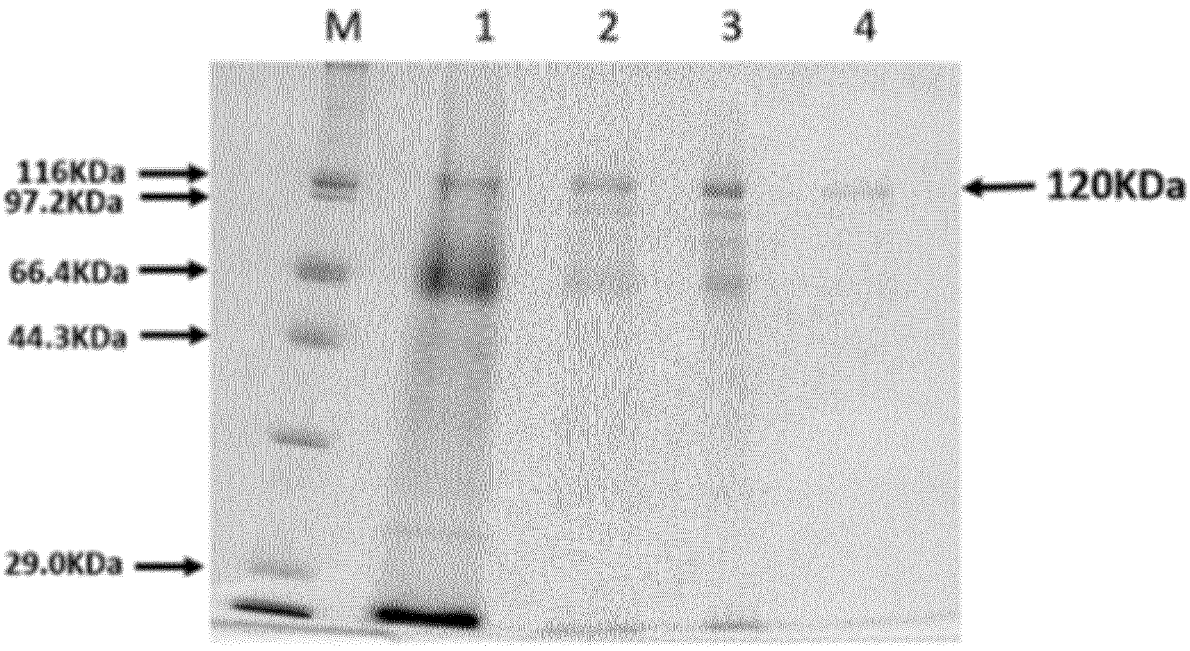
M
1
2
3
4
116KDa
97.2KDa
66.4KDa
44.3KDa
29.0KDa
120KDa

FIG. 13

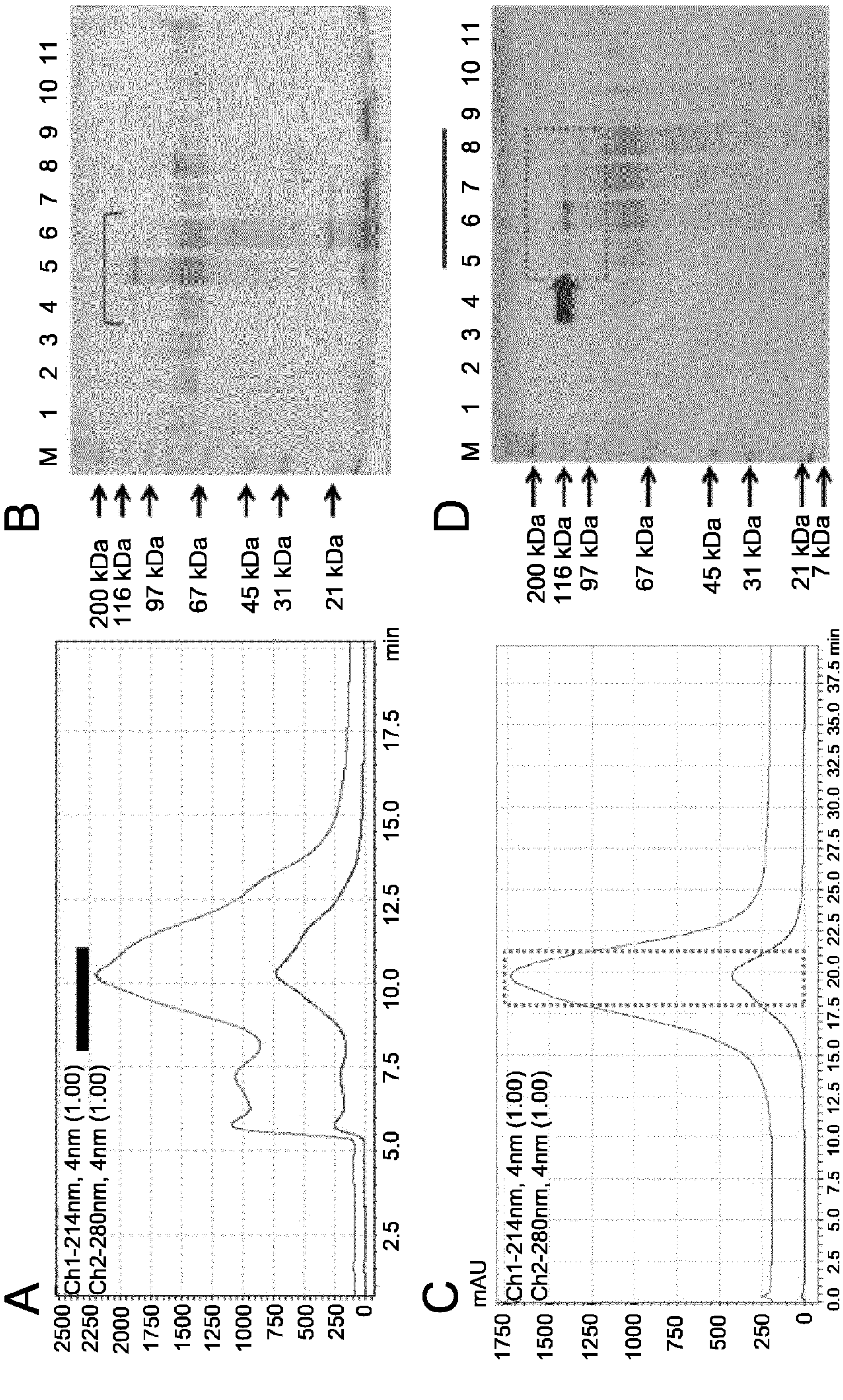
A
Ch1-214nm, 4nm (1.00)
Ch2-280nm, 4nm (1.00)
2500 2250 2000 1750 1500 1250 1000 750 500 250 0
2.5 5.0 7.5 10.0 12.5 15.0 17.5 min
B
200 kDa
116 kDa
97 kDa
67 kDa
45 kDa
31 kDa
21 kDa
M 1 2 3 4 5 6 7 8 9 10 11
C
mAU
Ch1-214nm, 4nm (1.00)
Ch2-280nm, 4nm (1.00)
1750 1500 1250 1000 750 500 250 0
0.0 2.5 5.0 7.5 10.0 12.5 15.0 17.5 20.0 22.5 25.0 27.5 30.0 32.5 35.0 37.5 min
D
200 kDa
116 kDa
97 kDa
67 kDa
45 kDa
31 kDa
21 kDa
7 kDa
M 1 2 3 4 5 6 7 8 9 10 11

FIG. 14

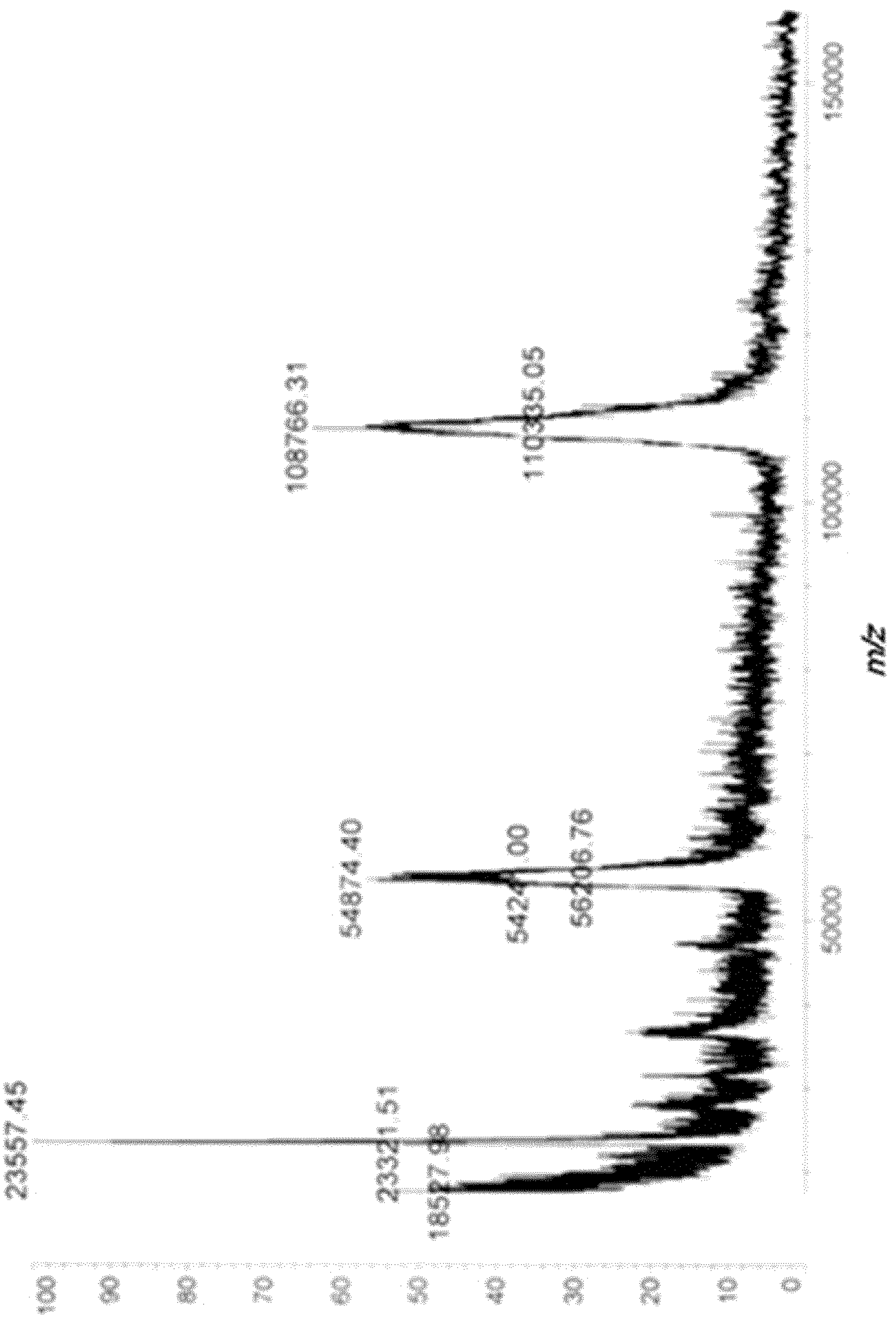
23557.45
23321.51
18527.98
54874.40
56206.76
108766.31
110335.05
100
90
80
70
60
50
40
30
20
10
0
50000
100000
150000
m/z

FIG. 15

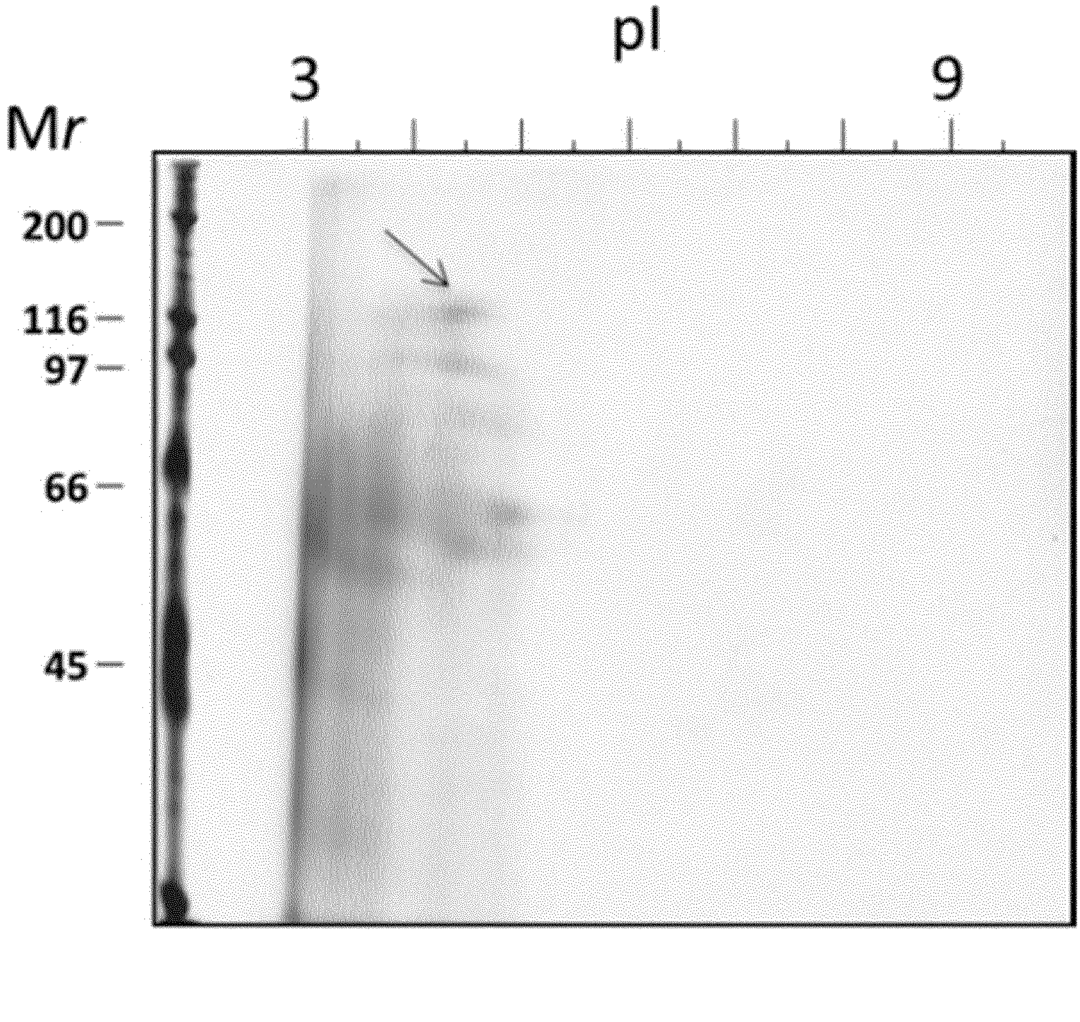

FIG. 16

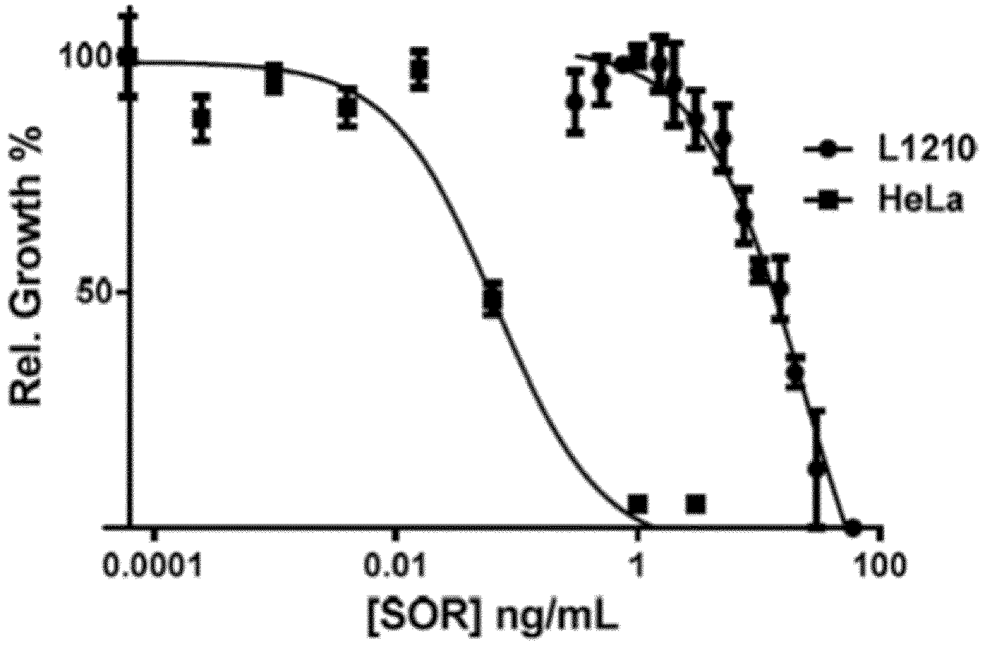
100
50
Rel. Growth %
0.0001
0.01
1
100
[SOR] ng/mL
L1210
HeLa

FIG. 17

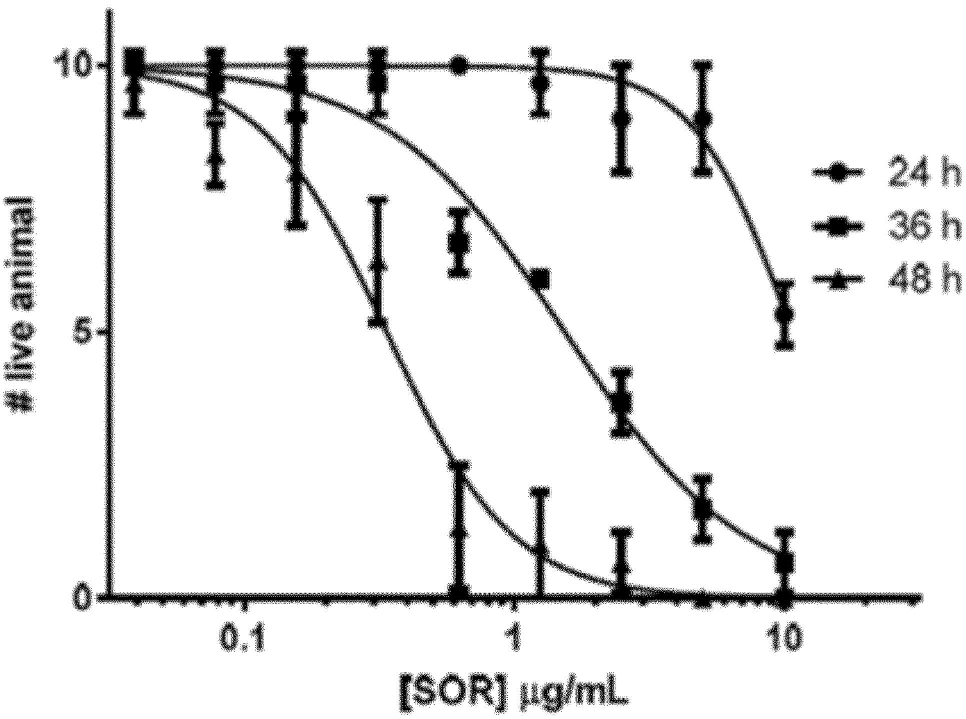
10
5
0
# live animal
0.1
1
10
[SOR] μg/mL
24 h
36 h
48 h

FIG. 18

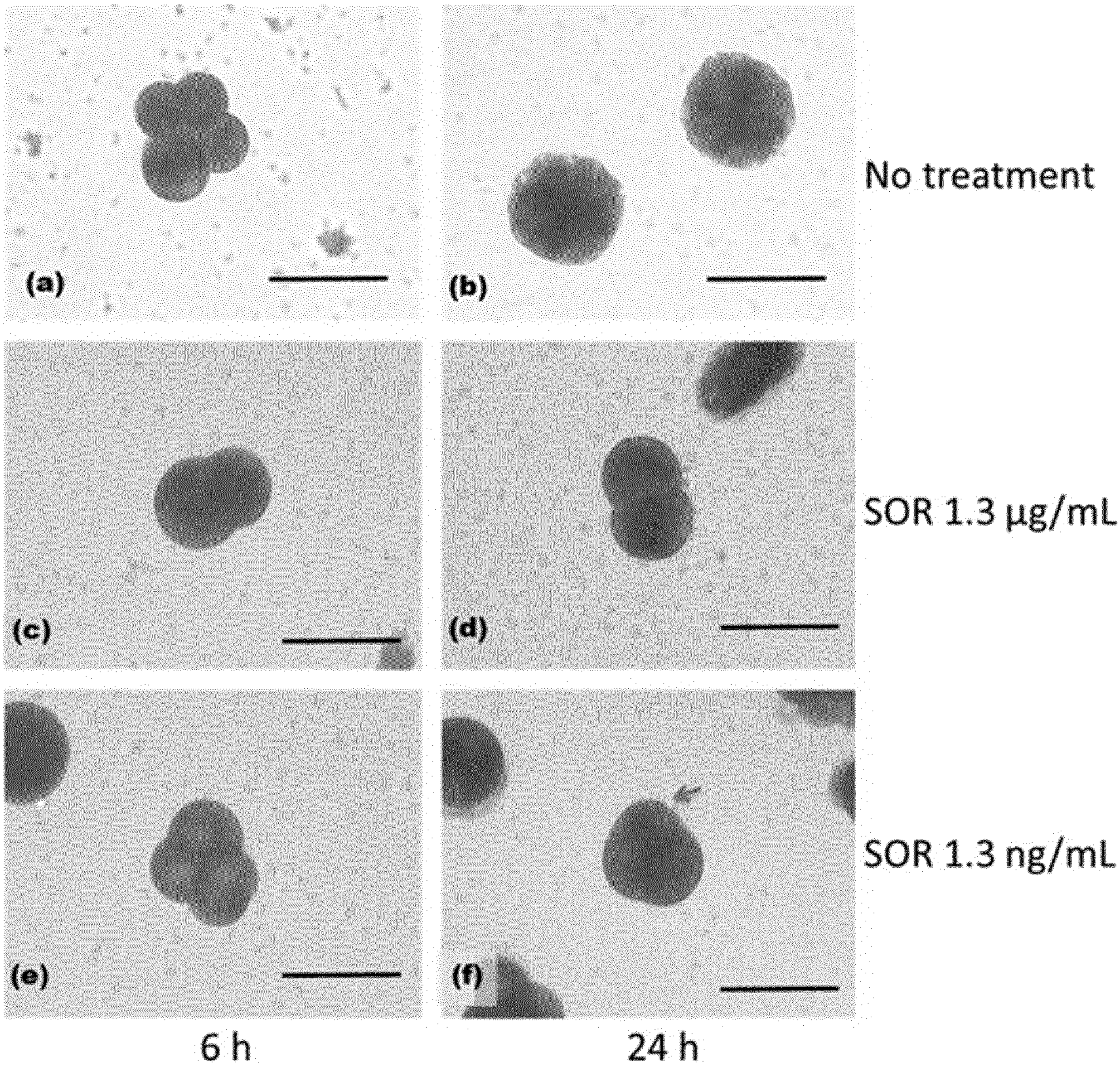

**FIG. 19**

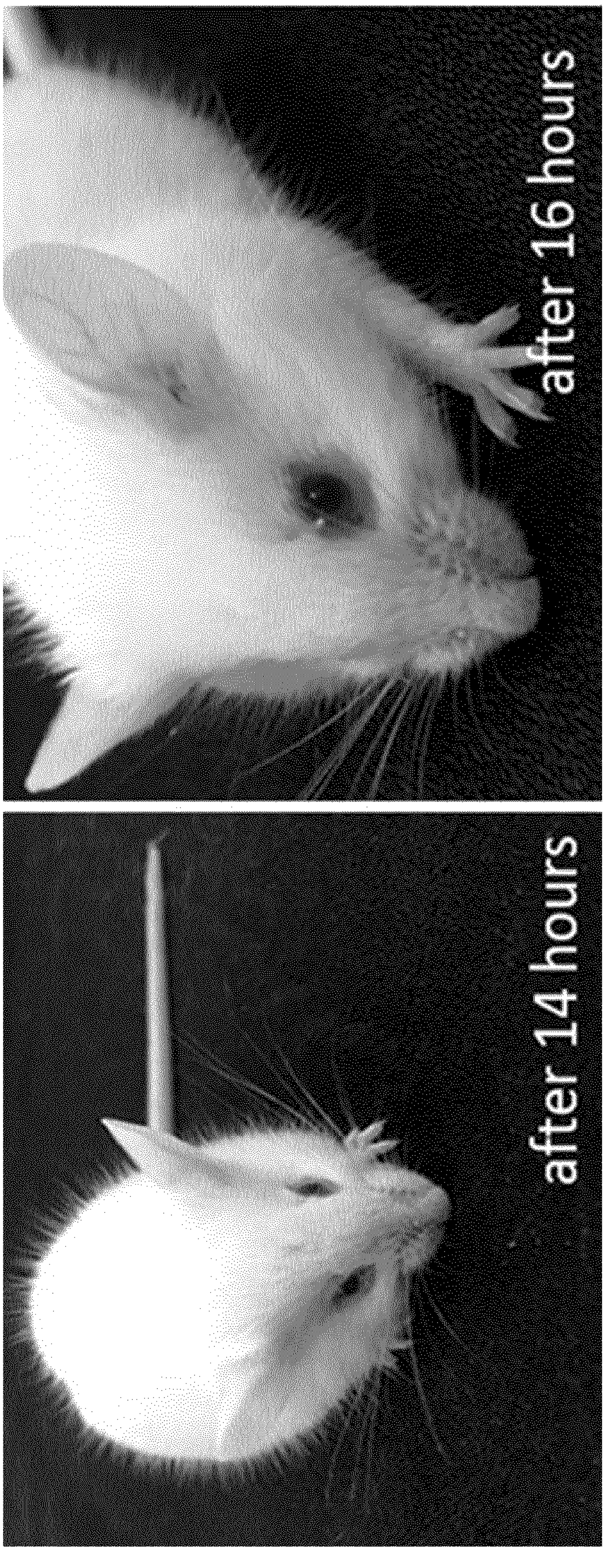

FIG. 20

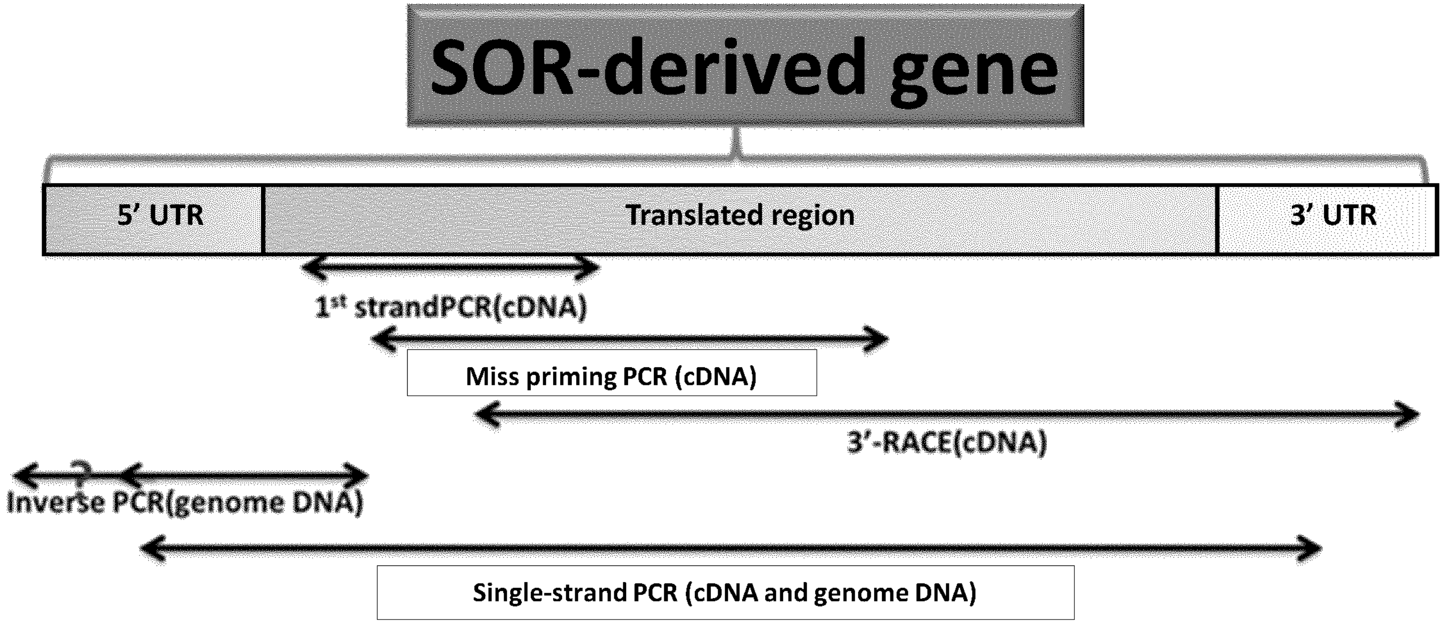


1. Degenerate primer PCR (cDNA)
1st: SOR1Fw, SOR1Rev 2nd: SOR2Fw, SOR4Rev
Results: SOR2Fw to SOR4Rev
From 13AA to 127AA

2. Miss priming PCR (cDNA)
1st: SOR4Fw1, UPM 2nd: SOR4Fw2, NUP
Results: SOR4Fw2 to SOR4Fw2 (Rev is miss priming)
From 86AA to 380AA (2nd base)

3. 3'-RACE(cDNA)
1st: SOR5Fw1, UPM 2nd: SOR5Fw2, NUP
Results: SOR5Fw1 to NUP (started from 1st primer)
From 298AA (3rd base) to poly-A

4. Inverse PCR (genome DNA)
1st: SOR-inv1, SOR3Fw2 2nd: SOR-inv2, SOR4Fw2
Results: SOR-inv2 to SOR4Fw2 (PstI site was absent despite cleavage with PstI)
From approximately 1291 bp upstream of 1AA to approximately 122AA (not definitive due to absence of PstI site)

5. Single-strand PCR (cDNA and genome DNA)
1st: SOR5'-5, SOR3'-2 2nd: SOR5'-4, SOR3'-1
Results: SOR5'-4 to SOR3'-1
From 193 bp upstream of 1AA to 62 bp from stop codon

FIG. 21

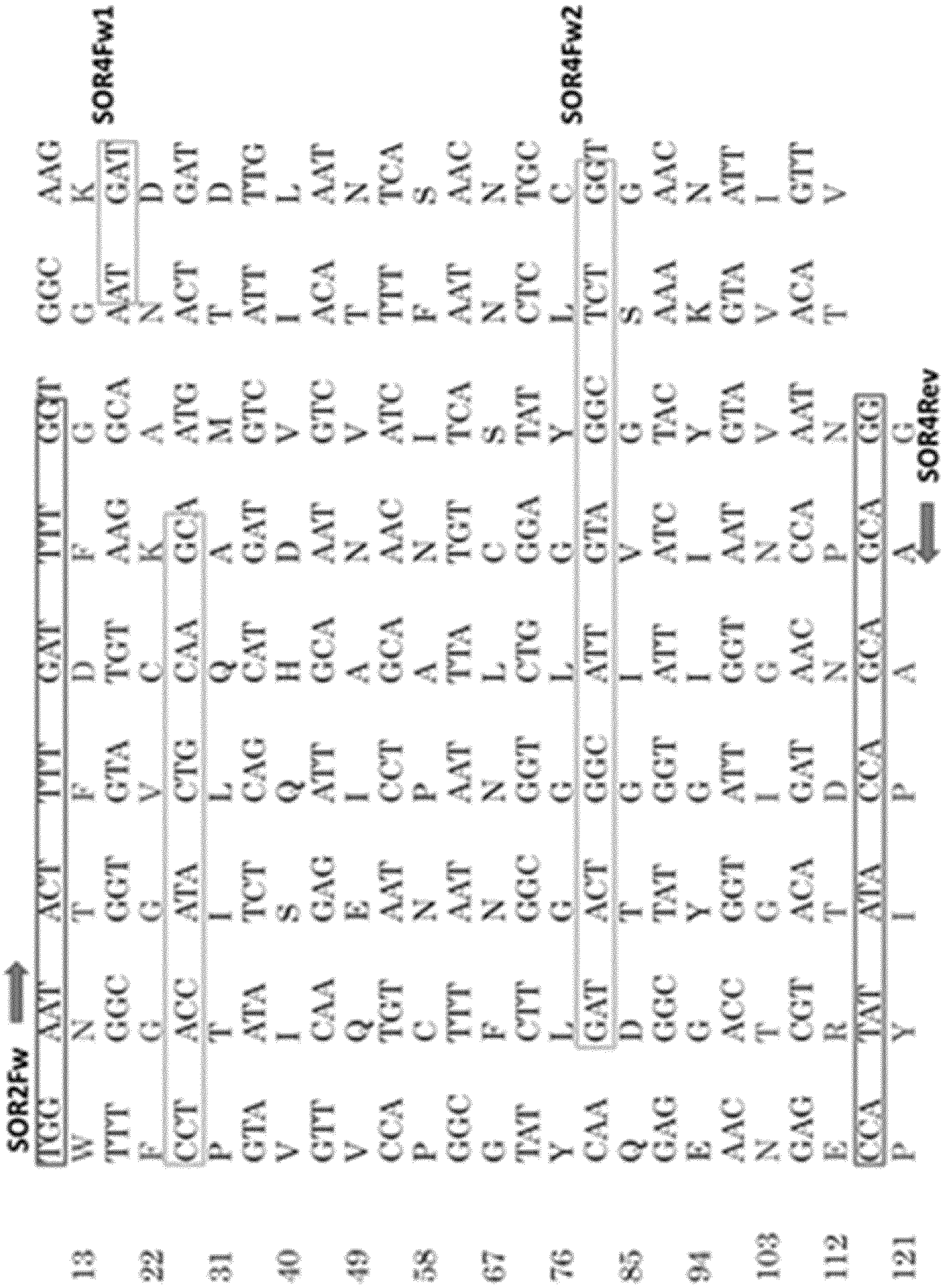
SOR2Fw
SOR4Fw1
SOR4Fw2
SOR4Rev
13 TGC AAT ACT TTT GAT TTT GGT GGC AAG
W N T F D F G G K
22 TTT GGC GGT GTA TGT AAG GCA AAT GAT
F G G V C K A N D
31 CCT ACC ATA CTG CAA GCA ATG ACT GAT
P T I L Q A M T D
40 GTA ATA TCT CAG CAT GAT GTC ATT TTG
V I S Q H D V I L
49 GTT CAA GAG ATT GCA AAT GTC ACA AAT
V Q E I A N V T N
58 CCA TGT AAT CCT GCA AAC ATC TTT TCA
P C N P A N I F S
67 GGC TTT AAT AAT TTA TGT TCA AAT AAC
G F N N L C S N N
76 TAT CTT GGC GGT CTG GGA TAT CTC TGC
Y L G G L G Y L C
85 CAA GAT ACT GGC ATT GTA GGC TCT GGT
Q D T G I V G S G
94 GAG GGC TAT GGT ATT ATC TAC AAA AAC
E G Y G I I Y K N
103 AAC ACC GGT ATT GGT AAT GTA GTA ATT
N T G I G N V V I
112 GAG CGT ACA GAT AAC CCA AAT ACA GTT
E R T D N P N T V
121 CCA TAT ATA CCA GCA GCA GG
P Y I P A A G

# FIG. 22

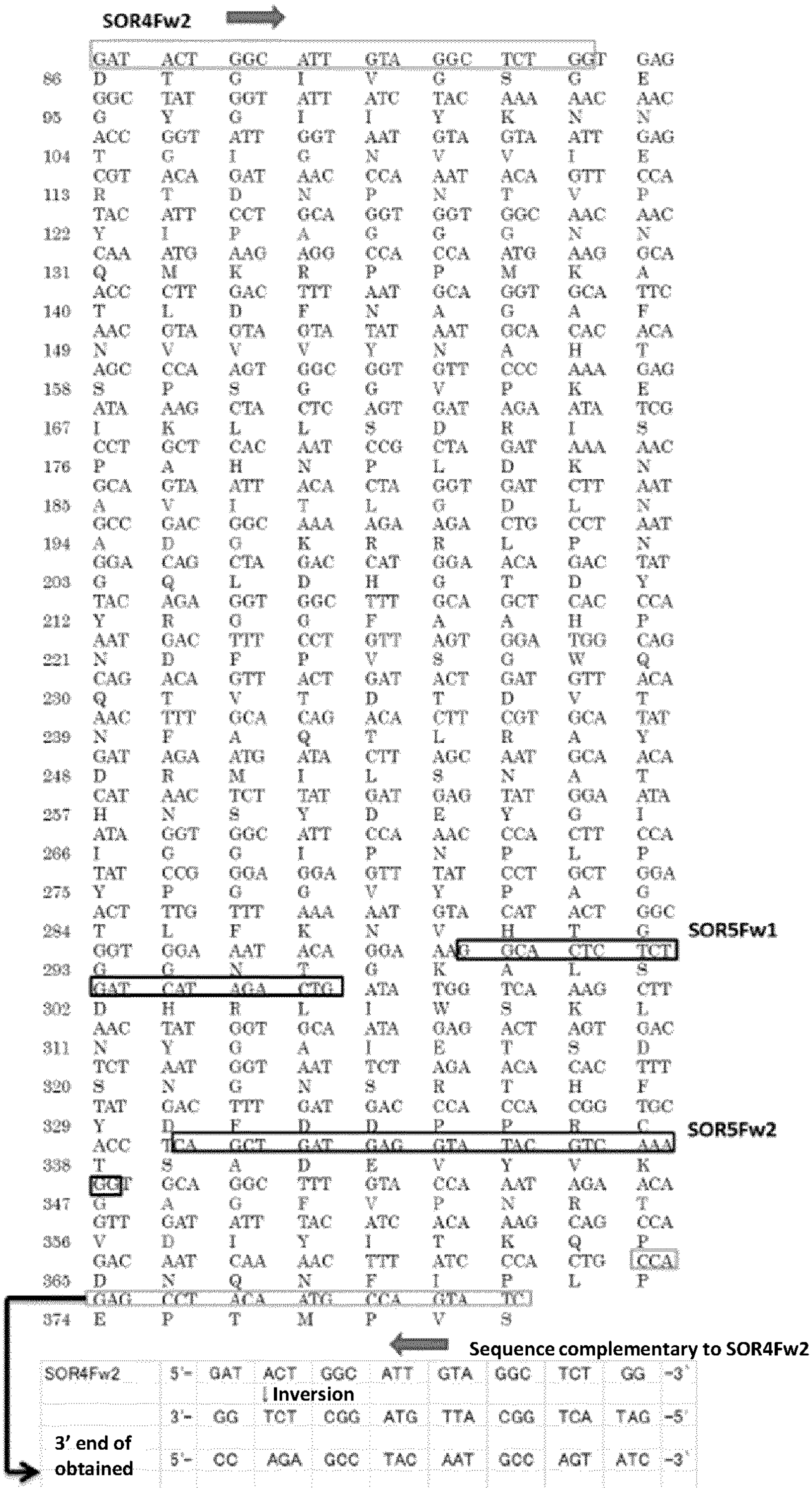


| | | | | | | | | | | |
|---|---|---|---|---|---|---|---|---|---|---|
| SOR4Fw2 | 5'- | GAT | ACT | GGC | ATT | GTA | GGC | TCT | GG | -3' |
| | | | Inversion | | | | | | | |
| | 3'- | GG | TCT | CGG | ATG | TTA | CGG | TCA | TAG | -5' |
| 3' end of obtained sequence | 5'- | CC | AGA | GCC | TAC | AAT | GCC | AGT | ATC | -3' |

# FIG. 23

SOR5Fw1 →

| | | | | | | | | | |
|---|---|---|---|---|---|---|---|---|---|
| | G | GCA | CTC | TCT | GAT | CAT | AGA | CTG | ATA |
| 299 | | A | L | S | D | H | R | L | I |
| | TGG | TCA | AAG | CTT | AAC | TAT | GGT | GCA | ATA |
| 307 | W | S | K | L | N | Y | G | A | I |
| | GAG | ACT | ACC | GAT | AAT | AAC | GGC | AAT | ACA |
| 316 | E | T | T | D | N | N | G | N | T |
| | AAG | TCC | AAT | TTT | GTT | GAA | TTT | ACA | AAC |
| 325 | K | S | N | F | V | E | F | T | N |
| | CCA | CCA | CAA | TGT | ACT | GCA | CCA | GAT | ACC |
| 334 | P | P | Q | C | T | A | P | D | T |
| | GTA | TAT | GCA | AAG | GGT | GCA | GAC | TTT | GTA |
| 343 | V | Y | A | K | G | A | D | F | V |
| | CCA | AAT | AGA | ACA | GTT | GAT | ATT | TAC | ATC |
| 352 | P | N | R | T | V | D | I | Y | I |
| | ACA | AAG | CAG | CCA | GAC | AAT | CAA | AAC | TTT |
| 361 | T | K | Q | P | D | N | Q | N | F |
| | ATC | CCA | CTG | CCA | CAG | CCA | CAA | AAC | CCA |
| 370 | I | P | L | P | Q | P | Q | N | P |
| | ACA | AAC | CCT | GGG | CAG | TTC | AAG | CAA | GTT |
| 379 | T | N | P | G | Q | F | K | Q | V |
| | GGC | GAA | TCA | TAC | CAG | CTA | ACA | GAT | GTA |
| 388 | G | E | S | Y | Q | L | T | D | V |
| | AGG | ACT | ACA | GGC | AAA | AGT | ACG | CAC | AAC |
| 397 | R | T | T | G | K | S | T | H | N |
| | ATT | GAT | GCA | AAC | GGC | AAC | ATT | GCA | AAT |
| 406 | I | D | A | N | G | N | I | A | N |
| | ACT | GCT | GTA | TGG | GTA | CAG | CCA | ACA | CCA |
| 415 | T | A | V | W | V | Q | P | T | P |
| | AAC | CAG | CAG | TAC | TAC | CTC | ATA | GTT | GAT |
| 424 | N | Q | Q | Y | Y | L | I | V | D |
| | GTG | GAC | CGT | GAT | GGA | TAC | TTT | ACA | AAC |
| 433 | V | D | R | D | G | Y | F | T | N |
| | AGC | ATA | GAT | ATA | GTT | GAT | GCA | TTT | AAC |
| 442 | S | I | D | I | V | D | A | F | N |
| | ACA | CAA | GGC | TTT | GAT | GTA | GAC | CCA | TGC |
| 451 | T | Q | G | F | D | V | D | P | C |
| | CTT | CGA | AAG | AGA | CTA | GCA | GCA | TCA | CTT |
| 460 | L | R | K | R | L | A | A | S | L |
| | TCT | GAT | GGA | ATA | CCA | GCA | GGC | GAT | GGC |
| 469 | S | D | G | I | P | A | G | D | G |
| | ACA | CGT | GAC | TTT | GTA | AAA | CCT | CCC | AAA |
| 478 | T | R | D | F | V | K | P | P | K |
| | ATA | TTT | ATT | TCC | GGT | GAC | TGG | TTC | CCA |
| 487 | I | F | I | S | G | D | W | F | P |
| | CAG | GGT | TCT | GCA | CGT | ATC | TAC | ATT | ATC |
| 496 | Q | G | S | A | R | I | Y | I | I |
| | ATA | TAT | GAC | AAG | AAC | TTT | GAT | TAT | AAC |
| 505 | I | Y | D | K | N | F | D | Y | N |
| | GGC | AAT | GAC | TCC | AAA | GTA | TTA | ACT | GAC |
| 514 | G | N | D | S | K | V | L | T | D |
| | GTC | CGT | GGA | ACT | TAT | GAC | ACA | ATT | ACA |
| 523 | V | R | G | T | Y | D | T | I | T |
| | GTG | GGT | GAG | AAC | CAC | AAG | TTT | ACA | GGA |
| 532 | V | G | E | N | H | K | F | T | G |

# FIG. 23 (cont.)

| | | | | | | | | | |
|---|---|---|---|---|---|---|---|---|---|
| | CTA | GAA | TGG | TCC | TCA | CCA | CAT | GTT | GGT |
| 541 | L | E | W | S | S | P | H | V | G |
| | AAC | TAT | AAC | ATA | ATA | TTT | GAT | GCA | AAC |
| 550 | N | Y | N | I | I | F | D | A | N |
| | TCT | GAT | GGA | GTA | TAC | AAA | AAA | TCC | GAT |
| 559 | S | D | G | V | Y | K | K | S | D |
| | GGT | GAC | ATT | GTA | GAT | CAC | ATA | AAC | GAG |
| 568 | G | D | I | V | D | H | I | N | E |
| | ATA | GGC | TTT | GCA | ATG | CTC | GAA | TCG | TAT |
| 577 | I | G | F | A | M | L | E | S | Y |
| | CCA | CAT | CAC | AGC | CAT | ATT | GTA | GAT | TTA |
| 586 | P | H | H | S | H | I | V | D | L |
| | GAT | GAC | AAT | GCA | AAC | CAG | CGA | GAA | GAC |
| 595 | D | D | N | A | N | Q | R | E | D |
| | TTT | AAC | AGT | GGA | ATT | GCA | AAA | AAC | GTC |
| 604 | F | N | S | G | I | A | K | N | V |
| | TAC | GTT | CTT | GCA | ACT | AAT | CTT | CCA | AAC |
| 613 | Y | V | L | A | T | N | L | P | N |
| | CCA | AGC | ACA | ATA | GTT | GAC | GTT | TAT | GTA |
| 622 | P | S | T | I | V | D | V | Y | V |
| | ATT | AGT | GAG | AAA | CTC | TTA | AAG | CTA | AAC |
| 631 | I | S | E | K | L | L | K | L | N |
| | AAT | CCT | GGA | TGG | ACC | ACA | TGG | CAG | CAA |
| 640 | N | P | G | W | T | T | W | Q | Q |
| | TCC | TCT | AAT | GTG | CTA | CTA | AAG | GAG | AGT |
| 649 | S | S | N | V | L | L | K | E | S |
| | GCA | GCA | CCA | ACT | AAC | AAT | GGA | GAC | AGG |
| 658 | A | A | P | T | N | N | G | D | R |
| | CTA | CAC | CCT | ACT | TGG | GTA | ACT | CCA | CAA |
| 667 | L | H | P | T | W | V | T | P | Q |
| | AAG | ACA | GTA | TTT | GGT | TCA | GTA | TGG | CAT |
| 676 | K | T | V | F | G | S | V | W | H |
| | ATG | CCC | GGT | GAT | ACA | TTT | GAT | CCA | CCA |
| 685 | M | P | G | D | T | F | D | P | P |
| | TAC | ATT | AAT | TAT | TAC | GGC | AAA | AAA | TTC |
| 694 | Y | I | N | Y | Y | G | K | K | F |
| | ACT | ATA | GTA | ATT | GAT | GTG | AAT | CGT | GAT |
| 703 | T | I | V | I | D | V | N | R | D |
| | GGT | AAG | TAT | GAT | CCT | AGT | ATA | GAT | ATG |
| 712 | G | K | Y | D | P | S | I | D | M |
| | GTA | GAT | ACT | CAC | GAT | ATA | GGA | GAC | ATG |
| 721 | V | D | T | H | D | I | G | D | M |
| | ACA | GAC | TGG | TTT | ATC | ACA | CAG | GGT | CAC |
| 730 | T | D | W | F | I | T | Q | G | H |
| | CAT | GAC | CTA | AGC | CCA | ACT | AGT | GGA | AAC |
| 739 | H | D | L | S | P | T | S | G | N |
| | GGA | CAG | CCT | GCC | GTA | TCT | GAA | TAC | AAA |
| 748 | G | Q | P | A | V | S | E | Y | K |
| | GAG | TAT | CTC | AAC | TCA | AAG | CTA | GAC | TTG |
| 757 | E | Y | L | N | S | K | L | D | L |
| | GAG | AAC | CCG | CTT | GAT | AAC | ACC | AAC | AAC |
| 766 | E | N | P | L | D | N | T | N | N |
| | TAT | GAT | GCT | GCA | ACT | GAG | GCA | GCT | TCT |
| 775 | Y | D | A | A | T | E | A | A | S |

# FIG. 23 (cont.)

| | | | | | | | | | |
|---|---|---|---|---|---|---|---|---|---|
| | GCA | CAA | TAT | CTA | TGC | CGT | ACC | AAT | CTT |
| 784 | A | Q | Y | L | C | R | T | N | L |
| | CCA | ATC | AAT | CTA | TTT | GAG | AAT | GTC | ATA |
| 793 | P | I | N | L | F | E | N | V | I |
| | GAG | CTG | GGA | GCT | CAG | ACA | GGC | TTT | ATC |
| 802 | E | L | G | A | Q | T | G | F | I |
| | ATG | TTT | AAC | CCT | GAT | GAG | TAT | TAT | GCA |
| 811 | M | F | N | P | D | E | Y | Y | A |
| | GGC | CGT | GAT | CTA | AAT | TCT | GGA | CGC | CAC |
| 820 | G | R | D | L | N | S | G | R | H |
| | ATT | TAT | GAT | GCT | GTA | GAT | TTT | GAT | GGC |
| 829 | I | Y | D | A | V | D | F | D | G |
| | TTG | GAG | ATA | AAT | GAC | GGA | TAC | ACA | TCA |
| 838 | L | E | I | N | D | G | Y | T | S |
| | GTA | ATC | TCT | GCA | AAA | GAG | ATG | ACC | CTA |
| 847 | V | I | S | A | K | E | M | T | L |
| | AAC | AGA | TTA | GGT | GTT | GGC | CAA | AAC | TCC |
| 856 | N | R | L | G | V | G | Q | N | S |
| | AAT | CTG | CTT | ACC | TGC | GGA | GCT | GAC | ACA |
| 865 | N | L | L | T | C | G | A | D | T |
| | GTA | ACG | ATA | GAT | GGC | ATG | TCA | ACA | CAA |
| 874 | V | T | I | D | G | M | S | T | Q |
| | AAA | GAT | TCA | ACT | ACA | AAT | ATT | GTT | GCA |
| 883 | K | D | S | T | T | N | I | V | A |
| | AAG | ACC | ATA | TCT | CAC | AAA | AGT | AAC | ATC |
| 892 | K | T | I | S | H | K | S | N | I |
| | TCA | CTA | GAG | GCA | GAC | ACT | TGT | ACA | CAT |
| 901 | S | L | E | A | D | T | C | T | H |
| | TAT | ACT | CAC | AAT | CTC | TAC | TTT | GAG | TCA |
| 910 | Y | T | H | N | L | Y | F | E | S |
| | GAC | ACC | AAC | ATC | TTA | GTT | AAG | GCC | ATT |
| 919 | D | T | N | I | L | V | K | A | I |
| | GAG | AGT | CCA | TTT | GCA | TGG | ATA | ATT | GAC |
| 928 | E | S | P | F | A | W | I | I | D |
| | GTA | GGT | GAC | AGA | ATA | CTT | CCA | GGT | ACT |
| 937 | V | G | D | R | I | L | P | G | T |
| | GGC | TGT | TAG | | | | | | |
| 946 | G | C | * | | | | | | |

SOR3'-1
ATGAATCGTGTGTGTGGGTTCTTCTAATATGATTGCACAAGCCACACATCTAAAACC
AGCACAATGCACACACACCTTCTGCTGTTTATAACTTTAATACATTATGACATTTTA
CTCTAAATTGCAATAATAGTTAATCAGTGTATATACTCAAAAACAAAAAACTATCCCT
CATACCATTGAGTGCTATGATATTATTTTTGGGAATAATAATAATAATAATGGGGGG
GGCATCTTCGCAGTCCTTTTCTCAAACTGATATATAGATAAACTAAATGACAATTCA
CAAGATCTGGGTAATCTATAGATTACTTAACATGATGTTATCATGCTATCCTTTCTAT SOR3'-2
GGGGGCCACCTGCAGGGCCTTGATATGTCCAGATAAAAAACAGGTCAAATTACGTC
CCACCTTTTGTACAGATCTCCTACTATCGAATATCTAAAATAACGGCTAAAATAGCC
AAACGCTAGGAAGGTTATCTAATGAGCTTAAAAAAAAAACAAGAAACAAAAAAAAA
AAAAAAAAAAAAAAAAAAAAGTACTCTGCGTTGATACCACTGCTT

NUP

FIG. 24

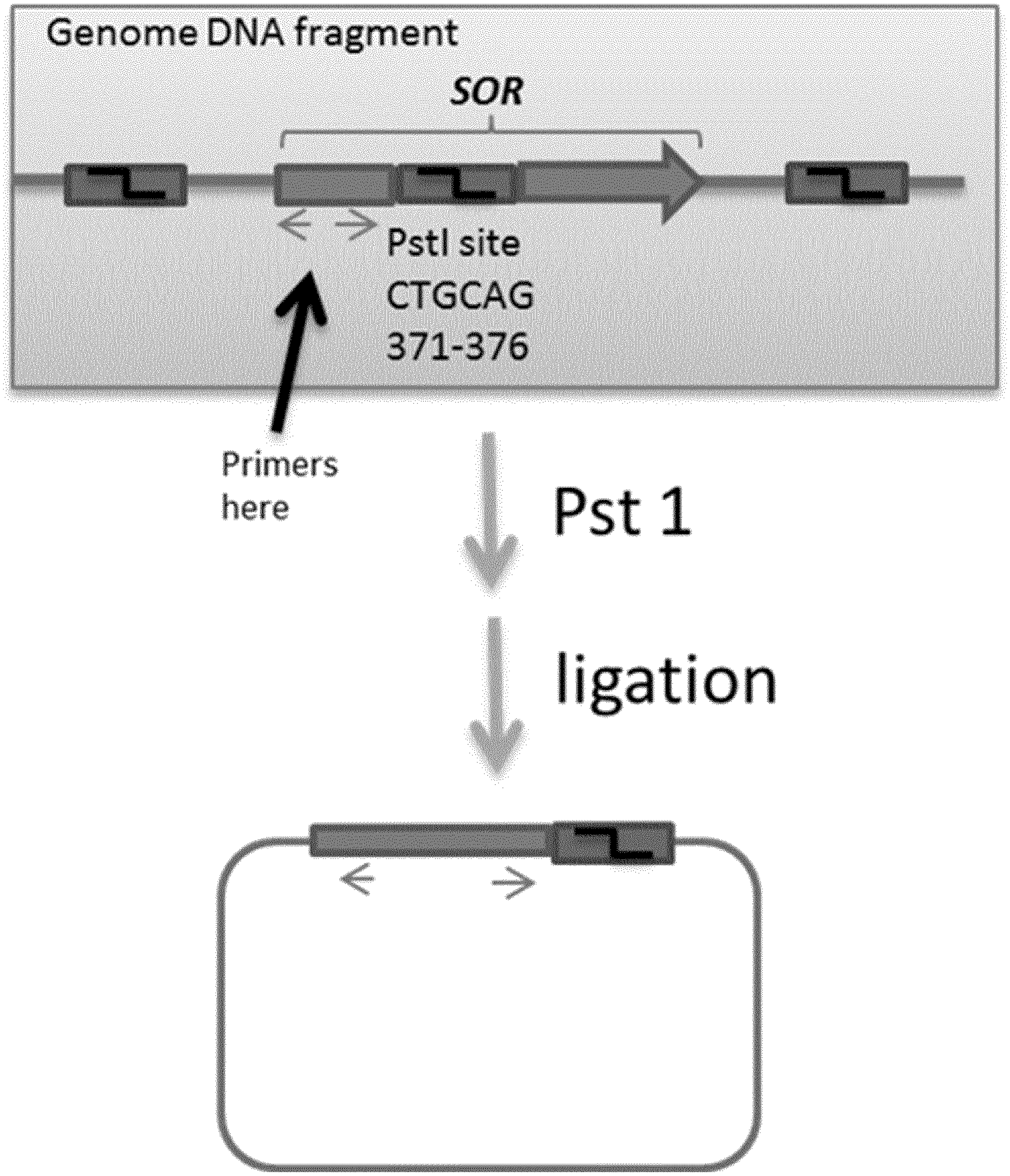
Genome DNA fragment
SOR
PstI site
CTGCAG
371-376
Primers
here
Pst 1
ligation

# FIG. 25

SOR4Fw2

GAT ACT GGC ATT GTA GGC TCT GGT GAG
86 D T G I V G S G E
GGC TAT GGT ATT ATC TAC AAA AAC AAC
95 G Y G I I Y K N N
ACC GGT ATT GGT AAT GTA GTA ATT GAG
104 T G I G N V V I E
CGT ACA GAT AAC CCA AAT ACA GTT CCA
113 R T D N P N T V P
TAC A
122 Y

Pst I site should be here

GGCAATGACTATAGATAGTACAGGCAAGGTAGGAATTGGAACTACTAGTCCAAATG
CAAAATTACATGTACAAGGATCATTTAGAGTCCAAGGCTATAACATCTTAACTGAC
CCTGATGAACAATACAACAGTCATTTTCCTCATTCAAGTAATAATGTCTACATCTCC
GGTGATAAAACATATCTTAGAAGTGGCAAACCAAACGGTTACTCCACTACAATGAC
TGTTGATAACATCAATGGTAATGTAGGCATTGGACTAAAAGAGCCCAAAGTAAAAC
TTCATATGTTTGGTGATGCCTTTTTGCAAACAGGCTCTTCTGGCGATTCAAGAAAT
TTGCAATTAAGCAGTTTCTATACTTCACAACCAAAAGCTATCAATCTTGAGAGTCG
TTACTTTGATACGATAGAGGGATTCAAACTTGGAGCAAGTGGAGATAGTTTTGTCC
ATGACAAGTTCTACATAAATAGATACACTGCCCATGTAAATGACAATCCCGGAATT
ACAACTAACAACGTTGATTCAACCAGACTCGTTACAGTTACAGCAACTGGGAATGT
AGGAATTGGAACTACTAGTCCAAGTAAGACACTTGATGTAAATGGCGATGCCATTG
CAAATAACTGGCTTAGACATTCCAGCCAGCAATGGAAAACAGACATAGCACCACT
AGATAACTCCCTAAGCAAGCTAGAGCAGCTACAAGGCATAAGCTACAAGTGGAAA
GCAGATGAATTCGCAGACATGGGATTTCACAATAAAACAACTCTTGGGTTTATTGC
CGAAGATGTAGAGACTATACTTCCACAGCTAATCCATACTGACCAAAATGGTGAAA
AATCAATGGACTATGGTAAACTAACACCAATTCTAGTTGAAGCAATCAAAGAGCAG
CAAAACACCATAGAACAGCTAACCACTGCAATATGTAAAGACAGCCCAAAACATG SOR5'-5
ACGTGTGCAGCAGCTAAAAAACACTTTTTTTTAATTTTTTGATCTTACAGTACAAC
CAGCAGCACTGAAACATTTTTGGCATCGAATCTAAAAAGAAAAAAAAATCATACTA
TCACATAATGATGATATGCTGTATTGTGAGAAACTCAAAGACACTGAAAAACATTT
TTGGCATCGAATCTAAAAAGAAAAAAAAATCATACTATCACATAATGATGATGATG SOR5'-4
ATGCTGTATTGTGAGAAACTCAAAGACACTGAAACATTTTATACAATCCATTAAAC
TTGAAAACATGACAGGGCCAACAAATCTCAAAATACTTCCTATATACCTTACACGC
CATAA

CAC TTC TCA TTG AAA CGA CAA GAT AGT
H F S L K R Q D S
AGT ATT ACA CAT CAG ACT GTG TCA ATT
S I T H Q T V S I
TTT TGC ATT ACC ATC TTG CTT TTA TCA
F C I T I L L L S
GTT AGT GCA TAC TTT CCT AAT TCT ATT
V S A Y F P N S I
CCA GAG GCG CAA GCT
P E A Q A
AAG CTT GGA GAT CAG CGA CAG ATA GAT
1 K L G D Q R Q I D
ATT GCA AGC TGG AAC ACA TTT GAT TTT
10 I A S W N T F D F
GGT GGC AAG TTT GGC GGT GTA TGT AAG
19 G G K F G G V C K

SOR-inv2

# FIG. 26

SOR5'-4

CCATTAAACTTGAAAACATGACAGGGCCAACAAATCTCAAAATACTTCCTATATACC
TTACACGCCA

| | | | | | | | | | |
|---|---|---|---|---|---|---|---|---|---|
| | TAA | CAC | TTC | TCA | TTG | AAA | CGA | CAA | GAT |
| | * | H | F | S | L | K | R | Q | D |
| | AGT | AGT | ATT | ACA | CAT | CAG | ACT | GTG | TCA |
| | S | S | I | T | H | Q | T | V | S |
| | ATT | TTT | TGC | ATT | ACC | ATC | TTG | CTT | TTA |
| | I | F | C | I | T | I | L | L | L |
| | TCA | GTT | AGT | GCA | TAC | TTT | CCT | AAT | TCT |
| | S | V | S | A | Y | F | P | N | S |
| | ATT | CCA | GAG | GCG | CAA | GCT | | | |
| | I | P | E | A | Q | A | | | |
| | AAG | CTT | GGA | GAT | CAG | CGA | CAG | ATA | GAT |
| 1 | K | L | G | D | Q | R | Q | I | D |
| | ATT | GCA | AGC | TGG | AAC | ACA | TTT | GAT | TTT |
| 10 | I | A | S | W | N | T | F | D | F |
| | GGT | GGC | AAG | TTT | GGC | GGT | GTA | TGT | AAG |
| 19 | G | G | K | F | G | G | V | C | K |
| | GCA | AAT | GAT | CCT | ACC | ATA | CTG | CAA | GCA |
| 28 | A | N | D | P | T | I | L | Q | A |
| | ATG | ACT | GAT | GTA | ATA | TCT | CAG | CAT | GAT |
| 37 | M | T | D | V | I | S | Q | H | D |
| | GTC | ATT | TTG | GTT | CAA | GAG | ATT | GCA | AAT |
| 46 | V | I | L | V | Q | E | I | A | N |
| | GTC | ACA | AAT | CCA | TGT | AAT | CCT | GCA | AAC |
| 55 | V | T | N | P | C | N | P | A | N |
| | ATC | TTT | TCA | GGC | TTT | AAT | AAT | TTA | TGT |
| 64 | I | F | S | G | F | N | N | L | C |
| | TCA | AAT | AAC | TAT | CTT | GGC | GGT | CTG | GGA |
| 73 | S | N | N | Y | L | G | G | L | G |
| | TAT | CTC | TGC | CAA | GAT | ACT | GGC | ATT | GTA |
| 82 | Y | L | C | Q | D | T | G | I | V |
| | GGC | TCT | GGT | GAG | GGC | TAT | GGT | ATT | ATC |
| 91 | G | S | G | E | G | Y | G | I | I |
| | TAC | AAA | AAC | AAC | ACC | GGT | ATT | GGT | AAT |
| 100 | Y | K | N | N | T | G | I | G | N |
| | GTA | GTA | ATT | GAG | CGT | ACA | GAT | AAC | CCA |
| 109 | V | V | I | E | R | T | D | N | P |
| | AAT | ACA | GTT | CCA | TAC | ATT | CCT | GCA | GGT |
| 118 | N | T | V | P | Y | I | P | A | G |
| | GGT | GGC | AAC | AAC | CAA | ATG | AAG | AGG | CCA |
| 127 | G | G | N | N | Q | M | K | R | P |
| | CCA | ATG | AAG | GCA | ACC | CTT | GAC | TTT | AAT |
| 136 | P | M | K | A | T | L | D | F | N |
| | GCA | GGT | GCA | TTC | AAC | GTA | GTA | GTA | TAT |
| 145 | A | G | A | F | N | V | V | V | Y |
| | AAT | GCA | CAC | ACA | AGC | CCA | AGT | GGC | GGT |
| 154 | N | A | H | T | S | P | S | G | G |
| | GTT | CCC | AAA | GAG | ATA | AAG | CTA | CTC | AGT |
| 163 | V | P | K | E | I | K | L | L | S |
| | GAT | AGA | ATA | TCG | CCT | GCT | CAC | AAT | CCG |
| 172 | D | R | I | S | P | A | H | N | P |
| | CTA | GAT | AAA | AAC | GCA | GTA | ATT | ACA | CTA |
| 181 | L | D | K | N | A | V | I | T | L |

# FIG. 26 (cont.)

| | | | | | | | | | |
|---|---|---|---|---|---|---|---|---|---|
| | GGT | GAT | CTT | AAT | GCC | GAC | GGC | AAA | AGA |
| 190 | G | D | L | N | A | D | G | K | R |
| | AGA | CTG | CCT | AAT | GGA | CAG | CTA | GAC | CAT |
| 199 | R | L | P | N | G | Q | L | D | H |
| | GGA | ACA | GAC | TAT | TAC | AGA | GGT | GGC | TTT |
| 208 | G | T | D | Y | Y | R | G | G | F |
| | GCA | GCT | CAC | CCA | AAT | GAC | TTT | CCT | GTT |
| 217 | A | A | H | P | N | D | F | P | V |
| | AGT | GGA | TGG | CAG | CAG | ACA | GTT | ACT | GAT |
| 226 | S | G | W | Q | Q | T | V | T | D |
| | ACT | GAT | GTT | ACA | AAC | TTT | GCA | CAG | ACA |
| 235 | T | D | V | T | N | F | A | Q | T |
| | CTT | CGT | GCA | TAT | GAT | AGA | ATG | ATA | CTT |
| 244 | L | R | A | Y | D | R | M | I | L |
| | AGC | AAT | GCA | ACA | CAT | AAC | TCT | TAT | GAT |
| 253 | S | N | A | T | H | N | S | Y | D |
| | GAG | TAT | GGA | ATA | ATA | GGT | GGC | ATT | CCA |
| 262 | E | Y | G | I | I | G | G | I | P |
| | AAC | CCA | CTT | CCA | TAT | CCG | GGA | GGA | GTT |
| 271 | N | P | L | P | Y | P | G | G | V |
| | TAT | CCT | GCT | GGA | ACT | TTG | TTT | AAA | AAT |
| 280 | Y | P | A | G | T | L | F | K | N |
| | GTA | CAT | ACT | GGC | GGT | GGA | AAT | ACA | GGA |
| 289 | V | H | T | G | G | G | N | T | G |
| | AAG | GCA | CTC | TCT | GAT | CAT | AGA | CTG | ATA |
| 298 | K | A | L | S | D | H | R | L | I |
| | TGG | TCA | AAG | CTT | AAC | TAT | GGT | GCA | ATA |
| 307 | W | S | K | L | N | Y | G | A | I |
| | GAG | ACT | ACC | GAT | AAT | AAC | GGC | AAT | ACA |
| 316 | E | T | T | D | N | N | G | N | T |
| | AAG | TCC | AAT | TTT | GTT | GAA | TTT | ACA | AAC |
| 325 | K | S | N | F | V | E | F | T | N |
| | CCA | CCA | CAA | TGT | ACT | GCA | CCA | GAT | ACC |
| 334 | P | P | Q | C | T | A | P | D | T |
| | GTA | TAT | GCA | AAG | GGT | GCA | GAC | TTT | GTA |
| 343 | V | Y | A | K | G | A | D | F | V |
| | CCA | AAT | AGA | ACA | GTT | GAT | ATT | TAC | ATC |
| 352 | P | N | R | T | V | D | I | Y | I |
| | ACA | AAG | CAG | CCA | GAC | AAT | CAA | AAC | TTT |
| 361 | T | K | Q | P | D | N | Q | N | F |
| | ATC | CCA | CTG | CCA | CAG | CCA | CAA | AAC | CCA |
| 370 | I | P | L | P | Q | P | Q | N | P |
| | ACA | AAC | CCT | GGG | CAG | TTC | AAG | CAA | GTT |
| 379 | T | N | P | G | Q | F | K | Q | V |
| | GGC | GAA | TCA | TAC | CAG | CTA | ACA | GAT | GTA |
| 388 | G | E | S | Y | Q | L | T | D | V |
| | AGG | ACT | ACA | GGC | AAA | AGT | ACG | CAC | AAC |
| 397 | R | T | T | G | K | S | T | H | N |
| | ATT | GAT | GCA | AAC | GGC | AAC | ATT | GCA | AAT |
| 406 | I | D | A | N | G | N | I | A | N |
| | ACT | GCT | GTA | TGG | GTA | CAG | CCA | ACA | CCA |
| 415 | T | A | V | W | V | Q | P | T | P |
| | AAC | CAG | CAG | TAC | AAC | CTC | ATA | GTT | GAT |
| 424 | N | Q | Q | Y | N | L | I | V | D |

# FIG. 26 (cont.)

| | | | | | | | | | |
|---|---|---|---|---|---|---|---|---|---|
| | GTG | GAC | CGT | GAT | GGA | TAC | TTT | ACA | AAC |
| 433 | V | D | R | D | G | Y | F | T | N |
| | AGC | ATA | GAT | ATA | GTT | GAT | GCA | TTT | AAC |
| 442 | S | I | D | I | V | D | A | F | N |
| | ACA | CAA | GGC | TTT | GAT | GTA | GAC | CCA | TGC |
| 451 | T | Q | G | F | D | V | D | P | C |
| | CTT | CGA | AAG | AGA | CTA | GCA | GCA | TCA | CTT |
| 460 | L | R | K | R | L | A | A | S | L |
| | TCT | GAT | GGA | ATA | CCA | GCA | GGC | GAT | GGC |
| 469 | S | D | G | I | P | A | G | D | G |
| | ACA | CGT | GAC | TTT | GTA | AAA | CCT | CCC | AAA |
| 478 | T | R | D | F | V | K | P | P | K |
| | ATA | TTT | ATT | TCC | GGT | GAC | TGG | TTC | CCA |
| 487 | I | F | I | S | G | D | W | F | P |
| | CAG | GGT | TCT | GCA | CGT | ATC | TAC | ATT | ATC |
| 496 | Q | G | S | A | R | I | Y | I | I |
| | ATA | TAT | GAC | AAG | AAC | TTT | GAT | TAT | AAC |
| 505 | I | Y | D | K | N | F | D | Y | N |
| | GGC | AAT | GAC | TCC | AAA | GTA | TTA | ACT | GAC |
| 514 | G | N | D | S | K | V | L | T | D |
| | GTC | CGT | GGA | ACT | TAT | GAC | ACA | ATT | ACA |
| 523 | V | R | G | T | Y | D | T | I | T |
| | GTG | GGT | GAG | AAC | CAC | AAG | TTT | ACA | GGA |
| 532 | V | G | E | N | H | K | F | T | G |
| | CTA | GAA | TGG | TCC | TCA | CCA | CAT | GTT | GGT |
| 541 | L | E | W | S | S | P | H | V | G |
| | AAC | TAT | AAC | ATA | ATA | TTT | GAT | GCA | AAC |
| 550 | N | Y | N | I | I | F | D | A | N |
| | TCT | GAT | GGA | GTA | TAC | AAA | AAA | TCC | GAT |
| 559 | S | D | G | V | Y | K | K | S | D |
| | GGT | GAC | ATT | GTA | GAT | CAC | ATA | AAC | GAG |
| 568 | G | D | I | V | D | H | I | N | E |
| | ATA | GGC | TTT | GCA | ATG | CTC | GAA | TCG | TAT |
| 577 | I | G | F | A | M | L | E | S | Y |
| | CCA | CAT | CAC | AGC | CAT | ATT | GTA | GAT | TTA |
| 586 | P | H | H | S | H | I | V | D | L |
| | GAT | GAC | AAT | GCA | AAC | CAG | CGA | GAA | GAC |
| 595 | D | D | N | A | N | Q | R | E | D |
| | TTT | AAC | AGT | GGA | ATT | GCA | AAA | AAC | GTC |
| 604 | F | N | S | G | I | A | K | N | V |
| | TAC | GTT | CTT | GCA | ACT | AAT | CTT | CCA | AAC |
| 613 | Y | V | L | A | T | N | L | P | N |
| | CCA | AGC | ACA | ATA | GTT | GAC | GTT | TAT | GTA |
| 622 | P | S | T | I | V | D | V | Y | V |
| | ATT | AGT | GAG | AAA | CTC | TTA | AAG | CTA | AAC |
| 631 | I | S | E | K | L | L | K | L | N |
| | AAT | CCT | GGA | TGG | ACC | ACA | TGG | CAG | CAA |
| 640 | N | P | G | W | T | T | W | Q | Q |
| | TCC | TCT | AAT | GTG | CCA | CTA | AAG | GAG | AGT |
| 649 | S | S | N | V | P | L | K | E | S |
| | GCA | GCA | CCA | ACT | AAC | AAT | GGA | GAC | AGG |
| 658 | A | A | P | T | N | N | G | D | R |
| | CTA | CAC | CCT | ACT | TGG | GTA | ACT | CCA | CAA |
| 667 | L | H | P | T | W | V | T | P | Q |

# FIG. 26 (cont.)

| | | | | | | | | | |
|---|---|---|---|---|---|---|---|---|---|
| | AAG | ACA | GTA | TTT | GGT | TCA | GTA | TGG | CAT |
| 676 | K | T | V | F | G | S | V | W | H |
| | ATG | CCC | GGT | GAT | ACA | TTT | GAT | CCA | CCA |
| 685 | M | P | G | D | T | F | D | P | P |
| | TAC | ATT | AAT | TAT | TAC | GGC | AAA | AAA | TTC |
| 694 | Y | I | N | Y | Y | G | K | K | F |
| | ACT | ATA | GTA | ATT | GAT | GTG | AAT | CGT | GAT |
| 703 | T | I | V | I | D | V | N | R | D |
| | GGT | AAG | TAT | GAT | CCT | AGT | ATA | GAT | ATG |
| 712 | G | K | Y | D | P | S | I | D | M |
| | GTA | GAT | ACT | CAC | GAT | ATA | GGA | GAC | ATG |
| 721 | V | D | T | H | D | I | G | D | M |
| | ACA | GAC | TGG | TTT | ATC | ACA | CAG | GGT | CAC |
| 730 | T | D | W | F | I | T | Q | G | H |
| | CAT | GAC | CTA | AGC | CCA | ACT | AGT | GGA | AAC |
| 739 | H | D | L | S | P | T | S | G | N |
| | GGA | CAG | CCT | GCC | GTA | TCT | GAA | TAC | AAA |
| 748 | G | Q | P | A | V | S | E | Y | K |
| | GAG | TAT | CTC | AAC | TCA | AAG | CTA | GAC | TTG |
| 757 | E | Y | L | N | S | K | L | D | L |
| | GAG | AAC | CCG | CTT | GAT | AAC | ACC | AAC | AAC |
| 766 | E | N | P | L | D | N | T | N | N |
| | TAT | GAT | GCT | GCA | ACT | GAG | GCA | GCT | TCT |
| 775 | Y | D | A | A | T | E | A | A | S |
| | GCA | CAA | TAT | CTA | TGC | CGT | ACC | AAT | CTT |
| 784 | A | Q | Y | L | C | R | T | N | L |
| | CCA | ATC | AAT | CTA | TTT | GAG | AAT | GTC | ATA |
| 793 | P | I | N | L | F | E | N | V | I |
| | GAG | CTG | GGA | GCT | CAG | ACA | GGC | TTT | ATC |
| 802 | E | L | G | A | Q | T | G | F | I |
| | ATG | TTT | AAC | CCT | GAT | GAG | TAT | TAT | GCA |
| 811 | M | F | N | P | D | E | Y | Y | A |
| | GGC | CGT | GAT | CTA | AAT | TCT | GGA | CGC | CAC |
| 820 | G | R | D | L | N | S | G | R | H |
| | ATT | TAT | GAT | GCT | GTA | GAT | TTT | GAT | GGC |
| 829 | I | Y | D | A | V | D | F | D | G |
| | TTG | GAG | ATA | AAT | GAC | GGA | TAC | ACA | TCA |
| 838 | L | E | I | N | D | G | Y | T | S |
| | GTA | ATC | TCT | GCA | AAA | GAG | ATG | ACC | CTA |
| 847 | V | I | S | A | K | E | M | T | L |
| | AAC | AGA | TTA | GGT | GTT | GGC | CAA | AAC | TCC |
| 856 | N | R | L | G | V | G | Q | N | S |
| | AAT | CTG | CTT | ACC | TGC | GGA | GCT | GAC | ACA |
| 865 | N | L | L | T | C | G | A | D | T |
| | GTA | ACG | ATA | GAT | GGC | ATG | TCA | ACA | CAA |
| 874 | V | T | I | D | G | M | S | T | Q |
| | AAA | GAT | TCA | ACT | ACA | AAT | ATT | GTT | GCA |
| 883 | K | D | S | T | T | N | I | V | A |
| | AAG | ACC | ATA | TCT | CAC | AAA | AGT | AAC | ATC |
| 892 | K | T | I | S | H | K | S | N | I |
| | TCA | CTA | GAG | GCA | GAC | ACT | TGT | ACA | CAT |
| 901 | S | L | E | A | D | T | C | T | H |
| | TAT | ACT | CAC | AAT | CTC | TAC | TTT | GAG | TCA |
| 910 | Y | T | H | N | L | Y | F | E | S |

## FIG. 26 (cont.)

| | | | | | | | | | |
|---|---|---|---|---|---|---|---|---|---|
| | GAC | ACC | AAC | ATC | TTA | GTT | AAG | GCC | ATT |
| 919 | D | T | N | I | L | V | K | A | I |
| | GAG | AGT | CCA | TTT | GCA | TGG | ATA | ATT | GAC |
| 928 | E | S | P | F | A | W | I | I | D |
| | GTA | GGT | GAC | AGA | ATA | CTT | CCA | GGT | ACT |
| 937 | V | G | D | R | I | L | P | G | T |
| | GGC | TGT | TAG | | | | | | |
| 946 | G | C | * | | | | | | |

ATGAATCGTGTGTGTGGGTTCTTCTAATATGATTGCACAAGCCACACATCTAAAACC
AGCAC
SOR3'-1

## FIG. 27

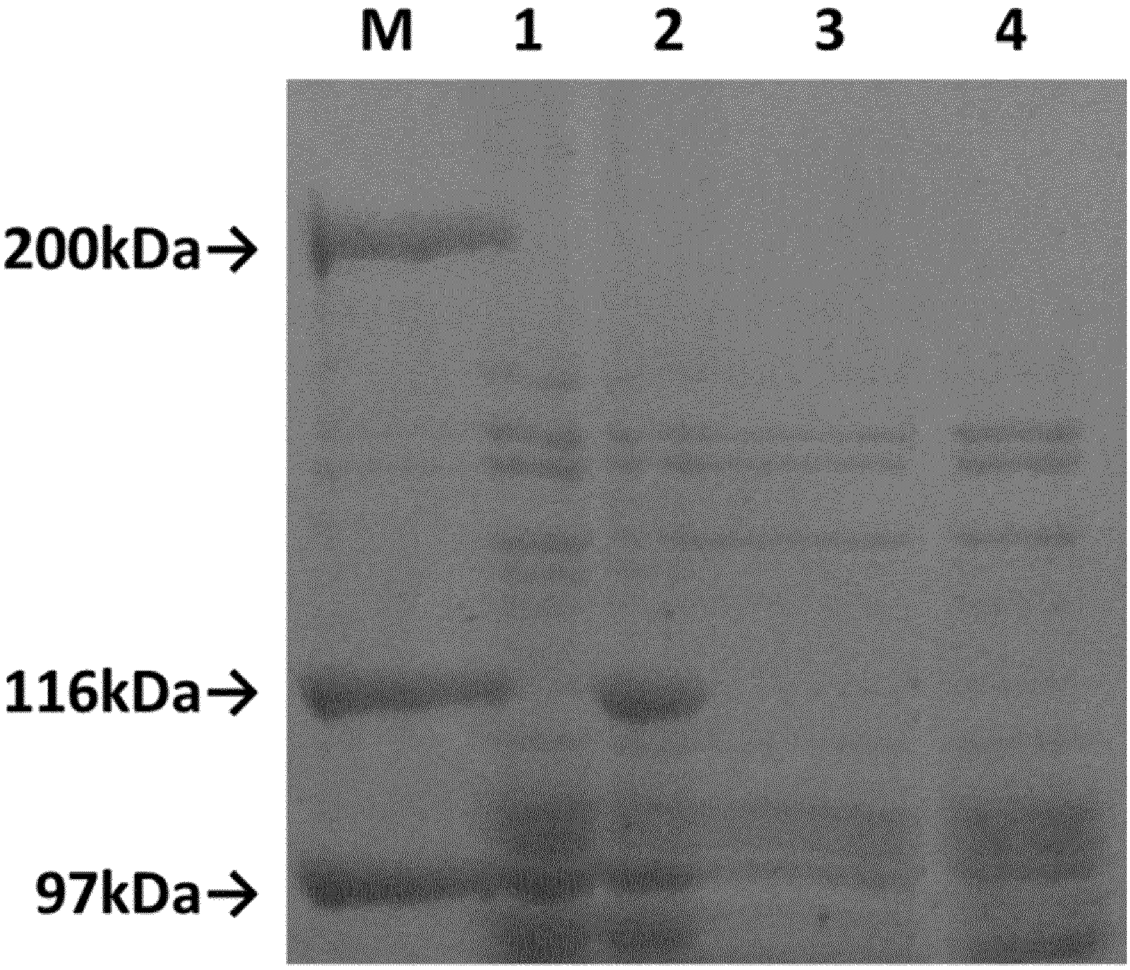

FIG. 28

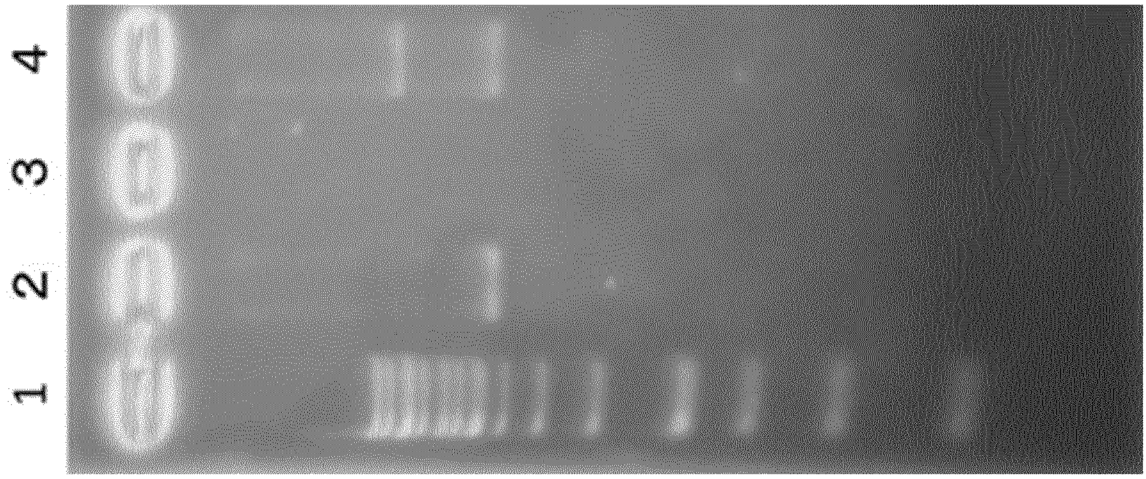


FIG. 29

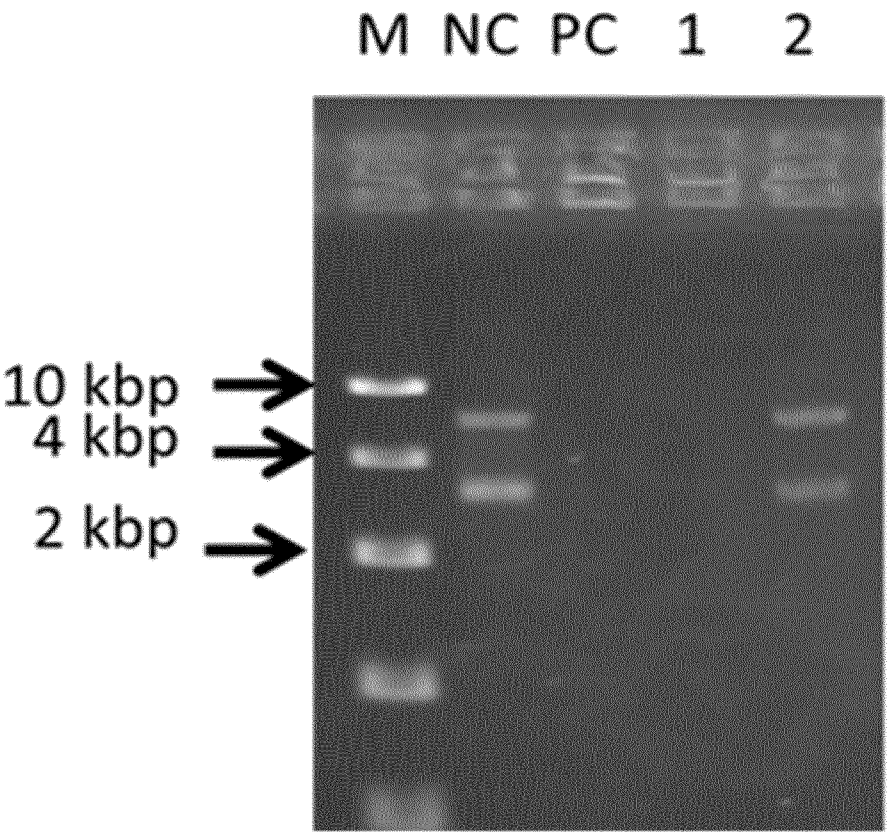

FIG. 30

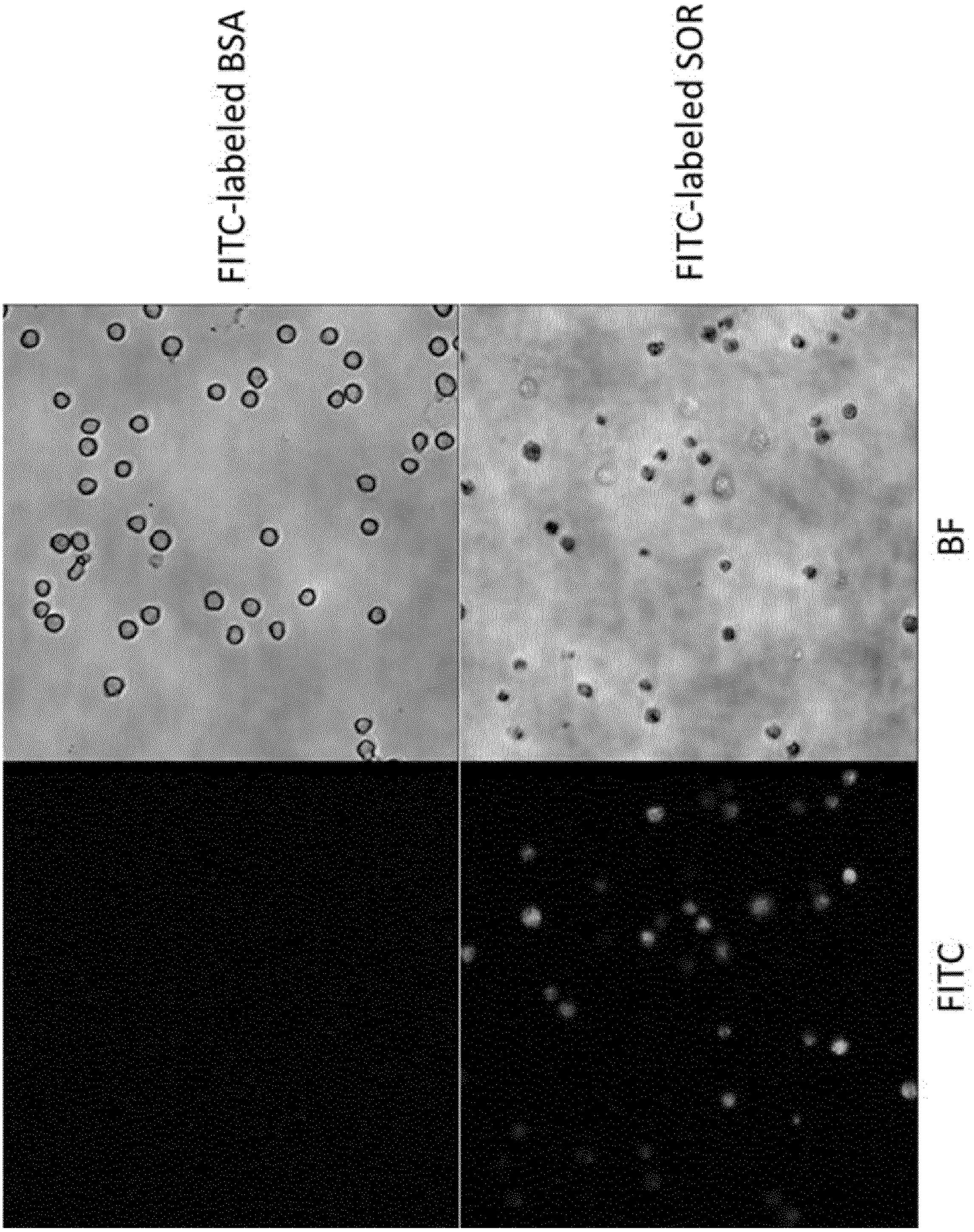

FIG. 31

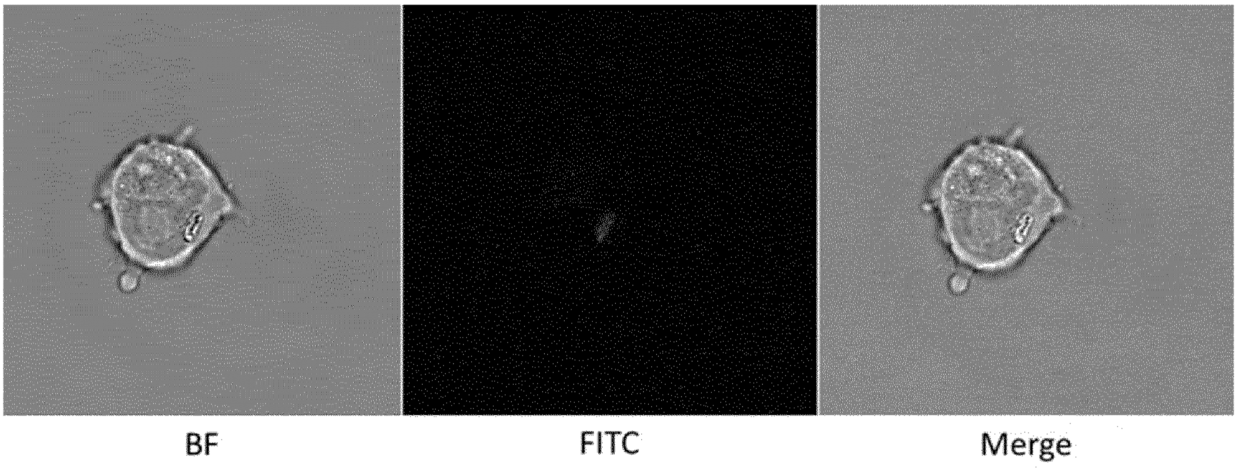


FIG. 32

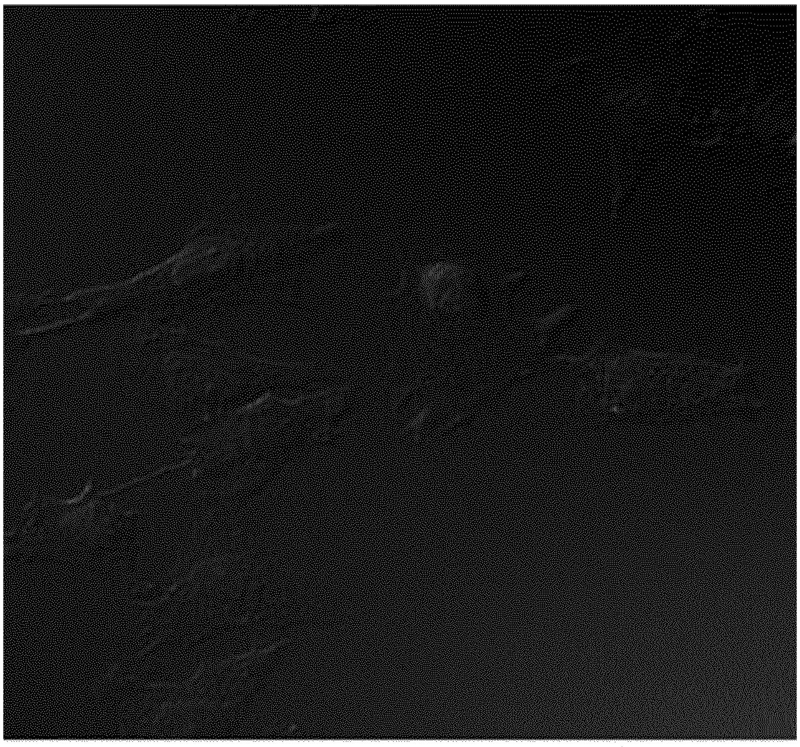

FIG. 33

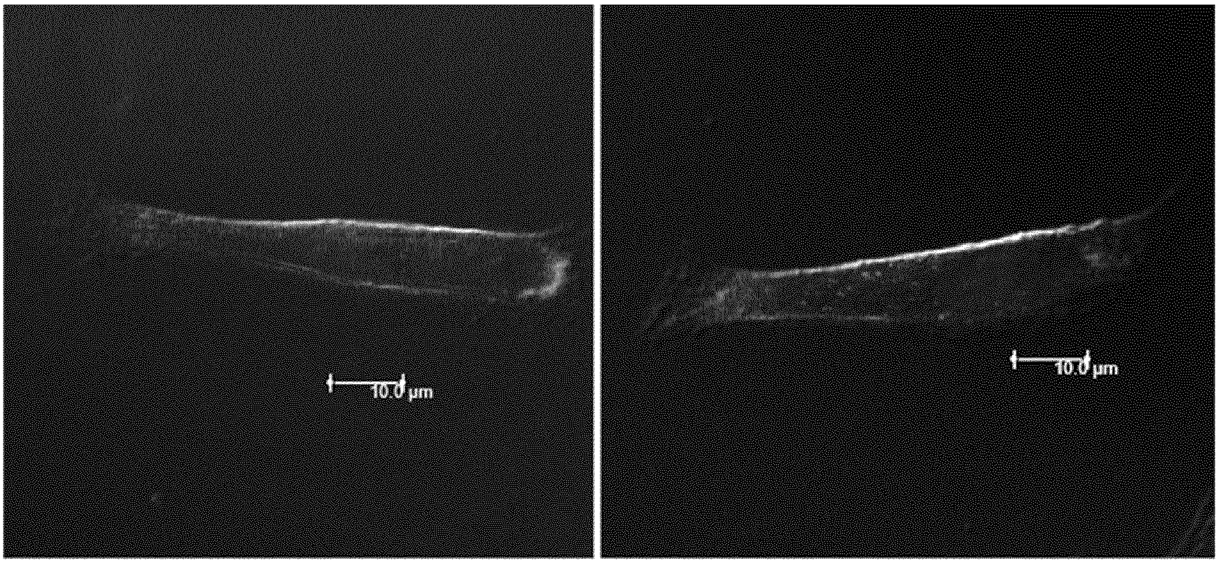


0.5 h 1 h

FIG. 34

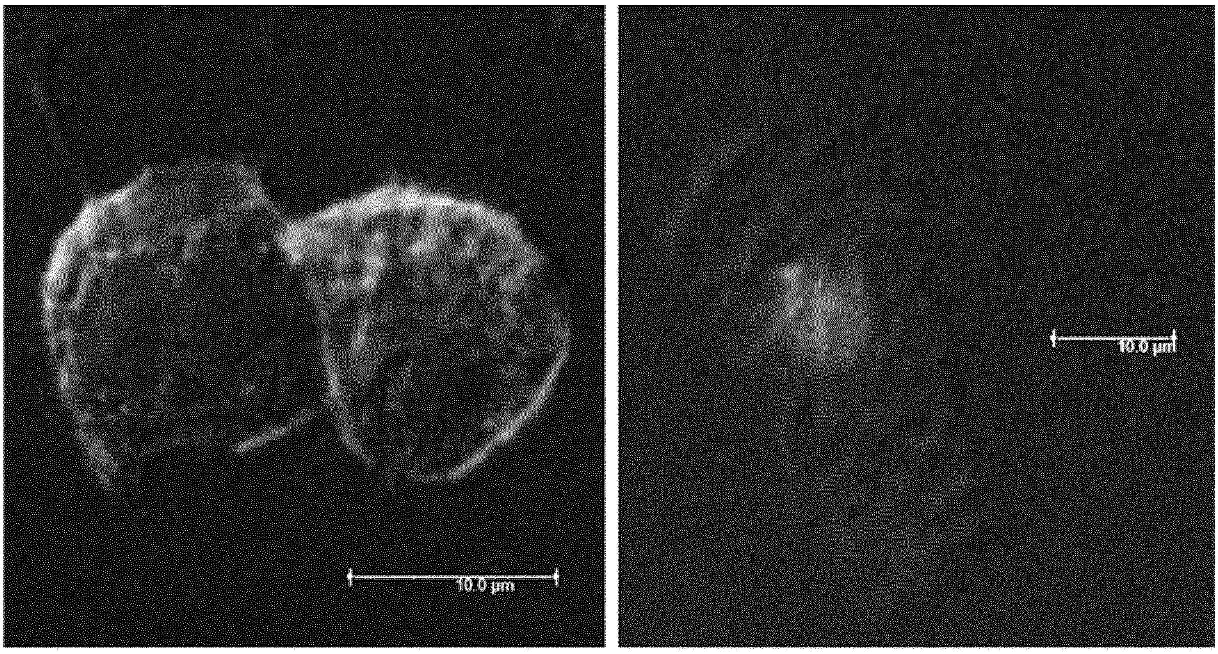


12 h 16 h

**INTERNATIONAL SEARCH REPORT**

International application No. PCT/JP2021/015369

A. CLASSIFICATION OF SUBJECT MATTER
See extra sheet

According to International Patent Classification (IPC) or to both national classification and IPC

B. FIELDS SEARCHED

Minimum documentation searched (classification system followed by classification symbols)
C12N15/12; A61K35/655; A61K38/17; A61K47/42; A61K47/50; A61K47/64; C07K14/435; C07K19/00; C12N1/15; C12N1/19; C12N1/21; C12N5/10; C12N15/63; C12P21/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN); UniProt/GeneSeq; Japio-GPG/FX

C. DOCUMENTS CONSIDERED TO BE RELEVANT

| Category* | Citation of document, with indication, where appropriate, of the relevant passages | Relevant to claim No. |
|---|---|---|
| X | 酒井隆一，新しい海綿毒素 Soitisidine の研究，第 62 回トキシンシンポジウム指名講演，講演番号 O-11, 09 April 2015, pp. 1-7, Internet <URL: http://dokuso-symposium.bio.tokushima-u.ac.jp/program/62th-abstract/62th-o.html#0-11>, [retrieval date 10 June 2021], in particular, page 6, text "0-11", non-official translation (SAKAI, Ryuichi, "Research on a novel sponge toxin, Soitisidine", Lecturer Nomination of the 62nd Japanese Society for Symposium on Toxins, Lecture no. O-11) | 1-25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 June 2021 (17.06.2021) | 29 June 2021 (29.06.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/015369

C (Continuation). DOCUMENTS CONSIDERED TO BE RELEVANT

| Category* | Citation of document, with indication, where appropriate, of the relevant passages | Relevant to claim No. |
|---|---|---|
| X | SAKAI, R. et al., "Soritesidine, a novel proteinous toxin from the Okinawan marine sponge Spongosorites sp.", Mar. Drugs (2019) vol. 17, no. 4, 216, pp. 1-15, Supplementary Materials pp. 1-4, ISSN:1660-3397, in particular, page 1, abstract, page 2, paragraph [0002], page 6, table 3, fig. 5, page 8, fig. 7, page 9, fig. 8, supplementary materials, table S1 | 1-25 |

Form PCT/ISA/210 (continuation of second sheet) (January 2015)

**INTERNATIONAL SEARCH REPORT**

International application No. PCT/JP2021/015369

<Continuation of Box A>
C12N 15/12(2006.01)i; A61K 35/655(2015.01)i; A61K 38/17(2006.01)i; A61K 47/42(2017.01)i; A61K 47/50(2017.01)i; A61K 47/64(2017.01)i; C07K 14/435(2006.01)i; C07K 19/00(2006.01)i; C12N 1/15(2006.01)i; C12N 1/19(2006.01)i; C12N 1/21(2006.01)i; C12N 5/10(2006.01)i; C12N 15/63(2006.01)i; C12P 21/02(2006.01)i
FI: C12N15/12 ZNA; A61K35/655; A61K38/17; A61K47/42; A61K47/50; A61K47/64; C07K14/435; C07K19/00; C12N1/15; C12N1/19; C12N1/21; C12N5/10; C12N15/63 Z; C12P21/02 C

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020069328 A

### Non-patent literature cited in the description


- **GOGINENI ; HAMANN.** *Biochim Biophys Acta Gen Subj.,* 2018, vol. 1862 (1), 81-196
- Mechanism of action of novel proteinous toxin derived from sponge of the genus Spongosorites from Iriomote Island. **YUUSUKE IDA et al.** Abstracts for the annual meeting of the Japanese Society of Fisheries Science. Spring, 2018, vol. 107
- **SAMBROOK ; RUSSELL.** Molecular Cloning: A Laboratory Manual. Laboratory Press, 2012, vol. 4
- **AUSUBEL.** Current Protocols in Molecular Biology. John Wiley & Sons, 1987
- **KARLIN ; ALTSCHUL.** Basic Local Alignment Search Tool. *Proc Natl Acad Sci USA.,* 1990, vol. 87 (6), 2264-8
- *Proc Natl Acad Sci USA.,* 1993, vol. 90 (12), 5873-7
- **JIMENEZ et al.** *Trends Biochem Sci.,* 2015, vol. 40 (11), 648-661
- **VAN ROOIJ ; KAUPPINEN.** *EMBO Mol Med.,* 2014, vol. 6 (7), 851-64
- **CHORN et al.** *RNA.,* 2012, vol. 18 (10), 1796-804
- **BROEDERS et al.** *iScience,* 2019, vol. 23 (1), 100789
- **ARIMORI et al.** *Structure.,* 2017, vol. 25 (10), 1611-1622
- **KUO et al.** *Protein Eng Des Sel.,* 2012, vol. 25 (10), 561-9
- **CHEN et al.** *Adv Drug Deliv Rev.,* 2013, vol. 65 (10), 1357-69
- **ODUMOSU et al.** *Toxins (Basel).,* 2010, vol. 2 (7), 1612-45
- **MICHALSKA ; WOLF.** *Front Microbiol.,* 2015, vol. 6, 963
- **WILLIAMS ; TSAI.** *Curr Opin Cell Biol.,* 2016, vol. 41, 51-6
- **VANOVA et al.** *Materials (Basel),* 2019, vol. 12 (17), E2671
- **SILVA et al.** *Biomolecules,* 2019, vol. 9 (1), E22
- **DERAKHSHANKHAH ; JAFARI.** *Biomed Pharmacother.,* 2018, vol. 108, 1090-1096
- **YAO et al.** *J Control Release.,* 28 October 2016, vol. 240, 267-286
- **YOO et al.** *Cancers (Basel),* 2019, vol. 11 (5), E640
- **SIMEON ; CHEN.** *Protein Cell.,* 2018, vol. 9 (1), 3-14
- **YU et al.** *Annu Rev Anal Chem (Palo Alto Calif).,* 2017, vol. 10 (1), 293-320
- **WUO ; ARORA.** *Curr Opin Chem Biol.,* June 2018, vol. 44, 16-22
- **JAHAN et al.** *J Drug Deliv.,* 2017, vol. 2017, 9090325
- **LENG et al.** *J Drug Deliv.,* 2017, vol. 2017, 6971297
- **ZOLGHADRI et al.** *J Enzyme Inhib Med Chem.,* 2019, vol. 34 (1), 279-309
- **LIEBMANN et al.** *Br J Cancer.,* 1993, vol. 68 (6), 1104-9
- **MIRANDA et al.** *J Cell Biol.,* 1974, vol. 61 (2), 481-500
- **KOBAYASHI et al.** *Chem Pharm Bull (Tokyo).,* 1994, vol. 42 (1), 19-26
- **SAWELEW et al.** *BioRxiv,* 2018, 292219
- **FREW et al.** *Toxicon.,* 2008, vol. 51 (8), 1400-8
- **SAKAI et al.** *J Pharmacol Exp Ther.,* 2001, vol. 296 (2), 650-8
- **YOKOYAMA et al.** *J Biochem.,* 1988, vol. 104 (2), 184-7
- **MOORE ; SCHEUER.** *Science,* 1971, vol. 172 (3982), 495-8
- **NAGAI et al.** *Biochem Biophys Res Commun.,* 2000, vol. 275 (2), 582-8